# EUROPEAN PATENT SPECIFICATION

(11) **EP 4 172 301 B1**
(45) Date of publication and mention of the grant of the patent: **24.07.2024**
(21) Application number: 21737438.8
(22) Date of filing: 29.06.2021
(51) Int. Cl.: C12C 12/00, C12R 1/86

(54) **LOW DIACETYL YEAST**
HEFE MIT NIEDRIGEM DIACETYLGEHALT
LEVURE À FAIBLE TENEUR EN DIACÉTYLE

(30) Priority: 30.06.2020 EP 20183134
(43) Date of publication of application: 03.05.2023
(73) Proprietor: Carlsberg A/S, 1799 Copenhagen V (DK)
(72) Inventor: LENGELER, Klaus, 1799 Copenhagen V (DK); KATZ, Michael, 1799 Copenhagen (DK); FÖRSTER, Jochen, 1799 Copenhagen V (DK); FENNESSY, Ross, 1799 Copenhagen V (DK); GJERMANSEN, Claes, 3000 Helsingør (DK); CHAILYAN, Anna, 2000 Frederiksberg (DK)
(74) Representative: Høiberg P/S
(86) International application number: PCT/EP2021/067882
(87) International publication number: WO 2022/002960

(56) References cited:
- WO-A1-2007/097089
- WO-A1-2015/005378
- JP-A- 2012 217 430
- ZHAO-YUE WANG ET AL: "Construction of self-cloning industrial brewing yeast with high-glutathione and low-diacetyl production", INTERNATIONAL JOURNAL OF FOOD SCIENCE AND TECHNOLOGY., vol. 43, no. 6, 1 June 2008 (2008-06-01), GB, pages 989 - 994, XP055306451, ISSN: 0950-5423, DOI: 10.1111/j.1365-2621.2007.01546.x

## Description

### Technical field

The present invention relates to the field of beer production, and in particular to the field of lager beer production. More specifically, the invention provides low diacetyl yeast, which is particularly useful for fast and efficient fermentation during beer production, in particular in the production of lager beers.

### Background

Lager beers are often characterized by fresh and clean flavour profiles. Diacetyl contributes to the flavour profile of many fermented products. However, its typical buttery flavour is considered as an off-flavour in lager-style beers, and the removal of this compound has a major impact on time and energy expenditure in breweries.

A lager beer is usually prepared by fermentation of wort - a carbohydrate rich liquid - with a lager yeast. Lager yeast in general differs from ale yeast in several ways. Lager yeast generally belong to the species *Saccharomyces pastorianus.* Frequently, lager yeast is also referred to as "bottom-fermenting yeast" because they settle at the bottom during fermentation. The settling, or flocculation, of the yeast can also affect the processing time, since the yeast needs to settle sufficiently to harvest it for the next round of brewing. For lager yeasts that are not very flocculent (settle slowly) this will require cooling and therefore results in additional processing time. Furthermore, lager yeast strains are in general best used at temperatures ranging from 7°C to 18 °C. In contrast to ale yeast, that generally belong to the species *Saccharomyces cerevisiae,* lager yeast is capable of using melibiose as the sole carbon source and typically cannot grow at 37ºC.

During fermentation, diacetyl is formed by non-enzymatic oxidation of acetolactate excreted by the yeast cell, however during the maturation period the diacetyl is taken up by the yeast cell again and metabolized. Part of the fermentation management is undertaken to ensure that the finished beer contains diacetyl below a set threshold. The problem of reducing the diacetyl content in the finished beer is especially prominent with lager beer as fermentation occurs at a lower temperature and this results in a slower re-uptake and metabolism of the diacetyl by the lager yeast.

The lower limit for taste perception of diacetyl in beer is generally considered to be 50 ppb of diacetyl, and the need of reducing the diacetyl concentration to this level in the finished beer conventionally adds a significant amount of extra time needed for maturation of lager beer, before the brewing process is complete. JP2012217430 describes yeast strains with a specific ILV2 variant and several copies of the ILV5 gene. The strains have lowered diacetyl production during fermentation. WO2007/097089 discloses yeast with a disrupted ILV2 gene, also showing lower diacetyl production. Zhao-Yue Wang, Int. J. Food Science Technology, 2008 vol 43, (6), 989-994 shows reduced production of diacetyl by yeast with a disrupted ILV2 gene.

### Summary

As outlined above, fermentation of wort with conventional *Saccharomyces pastorianus* lager yeast strains at temperatures between 7°C and 18 °C, results in diacetyl levels in the wort well above the 50 ppb limit of taste perception. Consequently, several additional days of maturation by the yeast have previously been required to reduce these levels to ensure the diacetyl content of the finished beer is below a set threshold.

Interestingly, the present invention provides new hybrid variants of *Saccharomyces pastorianus* yeast strains that produce low levels of diacetyl and/or quickly consume said diacetyl during fermentation at 18 °C or below, and consequently the beer produced by these strains requires very little time for maturation. In particular, when fermenting wort with the yeast strains of the invention, the total diacetyl level is ≤60ppb, preferably ≤50ppb, at the time the sugar fermentation is complete, i.e. at the time during the fermentation when the sugar level is more or less stable. Generally, a reduction in fermentation time of at least 1 day or at least 2 days is observed when compared to current lager strains.

The present invention also provides methods of producing beverages that require very little time for maturation of the beer after fermentation at 18 °C or below, by using *S*. *pastorianus* yeast strains, and which have a pleasant taste.

In one aspect, the present invention provides a method of producing a fermented aqueous extract, said method comprising the steps of:
i) providing an aqueous extract of malt and/or cereal;
ii) providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said fermented test solution contains at the most 60 ppb diacetyl, preferably at the most 50 ppb diacetyl at the earliest time point when the apparent extract of said fermented test solution has not decreased by more than 0.50° Plato in the preceding 24 hours, wherein said fermentation occurs at a temperature of 18 °C or less, preferably between 12 and 16 °C; and
iii) fermenting the aqueous extract provided in step i) with said yeast strain of step ii), thereby obtaining a fermented aqueous extract.

As used herein, the term "aqueous extract" is used to denote an aqueous extract of malt and/or cereal kernels, such as wort, which is used for making a beverage, e.g. a beer, while the term "test solution" is used to denote a solution with a more strictly defined composition that is used to assess the properties of the microorganism used for fermentation.

In another aspect, the present invention provides a method for producing a beverage, said method comprising the steps of:
i. providing an aqueous extract of malt and/or cereal;
ii. providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said test solution contains at the most 60 ppb diacetyl, preferably at the most 50 ppb diacetyl at the earliest time point when the apparent extract of said test solution has not decreased by more than 0.50° Plato in the preceding 24 hours, wherein said fermentation occurs at a temperature of at the most 18 °C;
iii. fermenting the aqueous extract provided in step i) with said yeast strain, thereby obtaining a fermented aqueous extract; and
iv. processing said fermented aqueous extract into a beverage.

In another aspect, the present invention provides a method for producing a beverage, said method comprising the steps of:
i. providing an aqueous extract of malt and/or cereal;
ii. providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said test solution contains at the most 60 ppb diacetyl, preferably at the most 50 ppb diacetyl at the earliest time point when the apparent extract of said test solution has not decreased by more than 0.50° Plato in the preceding 24 hours, wherein said fermentation occurs at a temperature of at the most 18 °C;
iii. fermenting the aqueous extract provided in step i) with said yeast strain, thereby obtaining a fermented aqueous extract; and
iv. processing said fermented aqueous extract into a beverage, wherein the steps of processing comprise one or more of the following:
   1. Filtration,
   2. Carbonation,
   3. Maturation, or
   4. Bottling

In another aspect, the present invention provides a yeast strain, wherein said yeast strain is capable of producing a fermented test solution after incubation of said yeast strain in a test solution having an apparent extract of at least 10° Plato and wherein said test solution contains at the most 60 ppb diacetyl, preferably at the most 50 ppb diacetyl at the earliest time point where the apparent extract of said test solution has not decreased by more than 0.50° Plato in the preceding 24 hours after incubation of said yeast strain in said extract.

### Description of Drawings

**FIG. 1** shows the diacetyl and °Plato levels of the wort incubated with the hybrid yeast strain ZDA1 and the control yeast strain Hybrid yeast 7 from a 50 L trial at (A) 16 °C and (B) 18 °C. Figure 1 C is the same plot as figure 1B, but with strain ZDA1 alone and a different axis for total diacetyl18 °C. The results are described in Example 1.
**FIG. 2** shows the diacetyl levels of wort incubated with the hybrid yeast strains ZDA1 and the control yeast strain Hybrid yeast 7 from a 10 HL trial at 16° (Figure 2A). The °Plato is additionally shown for the hybrid yeast strain ZDA1 (Figure 2B). The results are described in Example 4.
**FIG. 3** shows the ratio between propanol per isobutanol in nine different commercial beers compared to a beer prepared by the yeast strain ZDA2 (89-84393 ZDA F1) according to the present invention. All beers are close to 5% ABV.
FIG. 4 A-F: show an alignment of ILV2 protein sequences between WS34-78, cer *(Saccharomyces cerevisiae),* eub *(Saccharomyces eubayanus),* Hybrid7 (Hybrid yeast 7), ZDA1 and ZDA2.
FIG. 5 A-C: show an alignment of ILV6 protein sequences between WS34-78, cer *(Saccharomyces cerevisiae),* eub *(Saccharomyces eubayanus),* Hybrid7 (Hybrid yeast 7), ZDA1 and ZDA2.
FIG. 6 A-E: show an alignment of ILV3 protein sequences between WS34-78, cer *(Saccharomyces cerevisiae),* eub *(Saccharomyces eubayanus),* Hybrid7 (Hybrid yeast 7), ZDA1 and ZDA2.
FIG. 7 A-D: show an alignment of ILV5 protein sequences between WS34-78, cer *(Saccharomyces cerevisiae),* eub *(Saccharomyces eubayanus),* Hybrid7 (Hybrid yeast 7), ZDA1 and ZDA2.
FIG. 8 A-E: show an alignment of BAT1 protein sequences between WS34-78, cer (*Saccharomyces cerevisiae),* eub *(Saccharomyces eubayanus),* Hybrid7 (Hybrid yeast 7), ZDA1 and ZDA2.
FIG. 9 A-D: show an alignment of BAT2 protein sequences between WS34-78, cer *(Saccharomyces cerevisiae),* eub *(Saccharomyces eubayanus),* Hybrid7 (Hybrid yeast 7), ZDA1 and ZDA2.
**FIG. 10****:** shows the °Plato and diacetyl levels of wort incubated with the yeast strain ZDA2 and the control yeast strain Hybrid yeast 7 from a commercial scale trial at 16° C. The results are described in Example 8.

### Detailed description

### Definitions

As used herein, "a" can mean one or more, depending on the context in which it is used.

As used herein, the term "approximately" as used herein means ±10%, preferably ±5%, yet more preferably ±2%.

The term "beer" as used herein refers to a beverage prepared by fermentation of wort. Preferably, said fermentation is done by yeast.

The term "green beer" as used herein refers to a solution directly obtained after fermentation of wort, preferably by yeast. Thus "green beer" is obtained before lagering. In other words, green beer is the solution obtained immediately after the sugar fermentation has been completed. Generally speaking, beer is no longer considered "green" when it has reached full maturity of flavor and aroma.

The term "diacetyl" as used herein refers to the chemical compound of the formula:

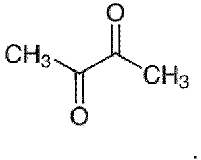

The concentration of diacetyl in a sample may be measured by gas chromatography according to the European Brewing Convention method EBC 9.24.2 that includes an incubation period of the samples at 60 °C for 90 minutes to determine the total diacetyl, that will reflect the total sum of precursor acetolactate and free diacetyl. Throughout the present disclosure, when describing the diacetyl produced by the presently disclosed yeast strains, the term "diacetyl" refers to "total diacetyl" unless otherwise indicated. The diacetyl concentration of a sample thus refers to the total diacetyl concentration, i.e. the total sum of precursor acetolactate and free diacetyl.

The term "cereal" as used herein refers to any plant of the grass family yielding an edible grain, such as wheat, millet, rice, barley, oats, rye, triticale, sorghum, and maize.

The term "grain" as used herein refers to seeds of a cereal comprising the cereal caryopsis, also denoted internal seed. In addition, the grain may comprise the lemma and palea. In most barley varieties, the lemma and palea adhere to the caryopsis and are a part of the grain following threshing. However, naked barley varieties also occur. In these, the caryopsis is free of the lemma and palea and threshes out free as in wheat. The terms "grain" and "kernel" are used interchangeably herein.

By the term "wort" is meant a liquid extract of malt and/or cereal grains and optionally additional adjuncts. Wort is in general obtained by mashing, optionally followed by "sparging", in a process of extracting residual sugars and other compounds from spent grains after mashing with hot water. Sparging is typically conducted in a lauter tun, a mash filter, or another apparatus to allow separation of the extracted water from spent grains. The wort obtained after mashing is generally referred to as "first wort", while the wort obtained after sparging is generally referred to as the "second wort". If not specified, the term wort may be first wort, second wort, or a combination of both. During conventional beer production, wort is boiled together with hops. Wort without hops, may also be referred to as "sweet wort", whereas wort boiled with hops may be referred to as "boiled wort" or simply as wort.

The term "aqueous extract" as used herein refers to any aqueous extract of malt and/or cereal kernels. Thus, non-limiting examples hereof can be wort with a given amount of fermentable sugars.

The term "fermented aqueous extract" as used herein refers to any aqueous extract incubated with a microorganism, such as a yeast strain, where the sugar fermentation has been completed. The sugar fermentation is considered completed at the time point during fermentation, when the sugar level, as measured by °Plato, no longer is significantly reduced. Preferably, the sugar fermentation may be considered completed, when the sugar level has not changed by more than 0.5 °Plato during a period of 24 hours, or when the sugar level has not changed by more than 0.25 °Plato during a period of 12 hours. A fermented aqueous extract may for example be a fermented malt and/or cereal based extract.

The term "test solution" as used herein refers to any aqueous liquids or solutions. The test solution may contain predetermined levels of specific compounds. Thus, non-limiting examples hereof can be wort with a specified sugar content.

The term "fermented test solution" as used herein refers to any test solution incubated with a microorganism, such as a yeast strain, where the sugar fermentation has been completed.

The term "°Plato" as used herein refers to density as measured on the Plato scale. The Plato scale is an empirically derived hydrometer scale to measure density of beer or wort in terms of percentage of extract by weight. The scale expresses the density as grams extract per 100 g wort. Plato can for example be measured with an Alcolyzer or handheld device from Anton Paar.

"Apparent extract" as used herein refers to the density of a given beer or wort measured in °Plato. As the density is primarily determined by the sugar content the apparent extract is an indication of the sugar content of the solution or extract. The apparent extract of a solution can be measured e.g. with a handheld Anton-PAAR serial number DM.

The term "Alcohol by volume (ABV)" as used herein refers to the amount of alcohol (ethanol) in a given volume of an alcoholic beverage (expressed as a volume percent). It is defined as the number of millilitres of pure ethanol present in 100 mL of solution at 20 °C. ABV can be measured with an Alcolyzer.

The term "RDF" or "real degree of fermentation" as used herein refers to the degree to which sugar in wort has been fermented into alcohol in beer, also termed attenuation. The RDF expresses the percentage of extract that was fermented. An RDF between 50 and 60% represent full-bodied beers with over 40% of their original extract left unfermented, whereas an RDF above 80% represent highly attenuated beers with less than 20% of their original extract unfermented. Mouthfeel is largely determined by RDF percentage; the higher the RDF percentage, the lighter and drier the beer.

The term "flocculation" as used herein refers to the process by which fine particles, such as yeast cells, are caused to clump together into a floc. The floc may then float to the top of the liquid (creaming), settle to the bottom of the liquid (sedimentation), or be readily filtered from the liquid. Yeast specialists and brewers often categorize yeast flocculation behaviour as being "high", "medium", or "low" according to the degree of flocculation observed for the yeast strain during the fermentation process. Highly flocculent strains can produce a brighter beer with less suspended yeast, making filtration easier. Flocculation can be increased by lower temperatures, so for low flocculent yeasts an additional cooling step may be needed after the fermentation is completed. High flocculent yeasts therefore have the possibility of reduced processing time compared to low flocculent yeasts, since no cooling is needed to achieve the brighter and easily filtrated beer. Flocculation may for example be determined by counting the number of yeast cells in solution after fermentation, e.g. by counting the number of yeast cells in a sample taken from the upper ¾ part of the container comprising the fermented aqueous extract or test solution.

The term "fermenting" as used herein refers to incubating an aqueous extract or a test solution with a microorganism, such as a yeast strain.

The term "malt" as used herein refers to cereal kernels, which have been malted. The term "green malt" refers to germinated cereal kernels, which have not been subjected to a step of kiln drying. In some embodiments the green malt is milled green malt. The term "kiln dried malt" as used herein refers germinated cereal kernels, which have been dried by kiln drying. In some embodiments the kiln dried malt is milled kiln dried malt. In general, said cereal kernels have been germinated under controlled environmental conditions.

The term "carbon source" as used herein refers to any organic molecule, which can provide energy to yeast and provide carbon for cellular biosynthesis. In particular, said carbon source may be carbohydrates, and more preferably, the carbon source may be monosaccharides, disaccharides trisaccharides, tetrasaccharides and/or short oligosaccharides. The carbon sources which can be fermented by yeast are often termed fermentable sugars, including but not limited to glucose, fructose, maltose, maltotriose and sucrose.

Amino acids may be named herein using the IUPAC one-letter and three-letter codes. If not otherwise indicated the term "amino acid" refers to the standard amino acids.

The term "functional gene" as used herein refers to a gene that, when transcribed and translated, expresses a protein with at least 80%, such as at least 85%, such as at least 90%, such as at least 95%, such as at least 99%, such as 100% sequence identity to the protein encoded by the corresponding gene of the wild type strain wherein the gene is endogenous. The protein expressed by the functional gene must be a functional homologue of the protein encoded by the corresponding gene of the wild type strain wherein the gene is endogenous, i.e. it must have the same type of enzymatic activity and said activity must be at a similar level to the protein encoded by the corresponding gene of the wild type strain wherein the gene is endogenous. In particular, the term "functional gene" does not cover genes encoding truncated protein products with little to no enzymatic activity. Preferably the term "gene" as used herein, refers to a functional gene.

The term "functional copy number" as used herein refers to the total number of functional genes within a yeast strain. "Functional copy number" is used interchangeably with "active copy number" herein.

The term "functional homologue" as used herein denotes a polypeptide sharing at least one biological function with a reference polypeptide. In general said functional homologue also shares a significant sequence identity with the reference polypeptide. Preferably a functional homologue of a reference polypeptide is a polypeptide, which has the same biological function as the reference protein and which shares a high level of sequence identity with the reference polypeptide.

The term "native promoter" as used herein refers to a promoter the nucleotide sequence of which has at least 80%, such as at least 85%, such as at least 90% such as at least 95", such as at least 99%, such as 100% sequence identity to that of the promoter of the wild type strain wherein the gene is endogenous. A gene expressed under its native promoter is thus usually expressed at endogenous levels.

The term "sequence identity" as used herein describes the relatedness between two amino acid sequences or between two nucleotide sequences, i.e. a candidate sequence (e.g. a mutant sequence) and a reference sequence (such as a wild type sequence) based on their pairwise alignment. For purposes of the present invention, the sequence identity between two amino acid sequences is determined using the Needleman-Wunsch algorithm (Needleman and Wunsch, 1970, J. Mo/. Biol. 48: 443-453) as implemented in the Needle program of the EMBOSS package (EMBOSS: The European Molecular Biology Open Software Suite, Rice et al., 2000, Trends Genet. 16: 276-277,), preferably version 5.0.0 or later (available at https://www.ebi.ac.uk/Tools/psa/emboss_needle/). The parameters used are gap open penalty of 10, gap extension penalty of 0.5, and the EBLOSUM62 (EMBOSS version of 30 BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment). The Needleman-Wunsch algorithm is also used to determine whether a given amino acid in a sequence other than the reference sequence corresponds to a given position of the reference sequence. For purposes of the present invention, the sequence identity between two nucleotide sequences is determined using the Needleman-Wunsch algorithm (Needleman and Wunsch, 1970, supra) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the DNAFULL (EMBOSS version of NCBI NUC4.4) substitution matrix. The output of Needle labeled "longest identity" (obtained using the -nobrief option) is used as the percent identity and is calculated as follows: (Identical Deoxyribonucleotides x 100)/(Length of Alignment - Total Number of Gaps in Alignment). Sequence identity is always measured compared to the full-length reference sequence, i.e. truncated proteins with no gaps or mismatches are not considered 100% sequence identical to the reference sequence.

The term "mutations" as used herein include insertions, deletions, substitutions, transversions, and point mutations in the coding and noncoding regions of a gene. Point mutations may concern changes of one base pair, and may result in premature stop codons, frameshift mutations, mutation of a splice site or amino acid substitutions. A gene comprising a mutation may be referred to as a "mutant gene". If said mutant gene encodes a polypeptide with a sequence different to the wild type, said polypeptide may be referred to as a "mutant polypeptide" and/or "mutant protein". A mutant polypeptide may be described as carrying a mutation, when it comprises an amino acid sequence differing from the wild type sequence.

The term "deletions" as used herein may be a deletion of the entire gene, or of only a portion of the gene, or a part of a chromosome.

The term "stop codon" as used herein refers to a nucleotide triplet in the genetic code, which within mRNA results in termination of translation. The term "stop codon" as used herein also refers to a nucleotide triplet within a gene encoding the stop codon in mRNA. The stop codon in DNA typically has one of the following sequences: TAG, TAA or TGA.

The term "growth" as used herein in relation to yeast, refers to the process by which a yeast cells multiply. Thus, when yeast cells are growing, the number of yeast cells increases. The number of yeast cells may be determined by any useful method.

The term "capable of utilizing" as used herein refers to the ability of yeast to use a specific compound as a source of carbon and/or nitrogen for cellular biosynthesis.

### Yeast strain

The present disclosure relates to a yeast strain, such as a *Saccharomyces pastorianus* yeast strain, that surprisingly produces low levels of total diacetyl and/or quickly consumes diacetyl during fermentation at 18 °C or lower, when incubated in an extract of malt and/or cereal having an apparent extract of at least 10° Plato.

Different types of yeast are used for production of beer, the most notable being *Saccharomyces pastorianus* and *Saccharomyces cerevisiae.* Lager beer is typically fermented using yeast of the species *Saccharomyces pastorianus.* Thus, *Saccharomyces pastorianus* according to the invention may for example be any yeast useful for production of lager beer. In particular, the *Saccharomyces pastorianus* may be a bottom fermenting yeast strain. It is preferred that the *Saccharomyces pastorianus* is a hybrid between *S*. *cerevisiae* and *S*. *eubayanus.* It is also preferred that said *Saccharomyces pastorianus* is capable of utilizing maltose and melibiose. Thus, preferably the *Saccharomyces pastorianus* is a yeast strain, which is a hybrid between *S*. *cerevisiae* and *S*. *eubayanus.* It is also preferred that said *Saccharomyces pastorianus* is capable of utilizing maltose and melibiose.

In one aspect, the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said fermented test solution contains at the most 60 ppb diacetyl, preferably at the most 50 ppb diacetyl at the earliest time point where the apparent extract of said test solution has not decreased by more than 0.50° Plato in the preceding 24 hours after incubation of said yeast strain in said extract, wherein said fermentation occurs at a temperature of at the most 18 °C.

Thus, the *Saccharomyces pastorianus* yeast strain according to the invention is preferably capable of producing a fermented test solution, when tested in a method comprising the steps of:
a) Providing a test solution, wherein the test solution is an extract of malt and/or cereals having an apparent extract of at least 10° Plato,
b) incubating said *Saccharomyces pastorianus* yeast strain with said test solution at a temperature of 18°C or less, preferably between 12 and 18°C, more preferably at 16 °C,
c) determining the earliest time point where the apparent extract of said test solution has not decreased by more than 0.50° Plato in the preceding 24 hours during said incubation, and
d) determining the level of diacetyl at said earliest time point,
wherein said test solution contains at the most 60 ppb diacetyl, preferably at the most 50 ppb diacetyl at said earliest time point.

In some embodiments the *Saccharomyces pastorianus* yeast strain is capable of producing a fermented test solution after incubation with said yeast strain in a test solution, wherein the test solution may be any of the test solutions described below, preferably the test solution is an extract of malt and/or cereals having an apparent extract of at least 10° Plato", and wherein the fermented test solution contains a maximum level of diacetyl as described below at the earliest time point where the apparent extract of said test solution has not decreased by more than 0.50° Plato, such as not decreased by more than 0.40° Plato, such as not decreased by more than 0.30° Plato, such as not decreased by more than 0.20° Plato in the preceding 24 hours after incubation with said yeast strain in said test solution wherein said fermentation occurs at a temperature as specified below. In particular, the apparent extract of said test solution has not decreased by more than 0.50° Plato in the preceding 24 hours.

Said maximal level of diacetyl is preferably at the most 60 ppb, such as at the most 55 ppb diacetyl, such as at the most 50 ppb diacetyl, for example at the most 45 ppb diacetyl at the earliest time point where the apparent extract of said test solution has not decreased by more than the aforementioned ° Plato. In particular, said level of diacetyl at said earliest time point may be at the most 45 ppb.

When identifying the properties of the yeast, as described above, the incubation is preferably performed at a temperature of at the most 18 °C, such as at a temperature of in the range of 12°C to 18 °C or in the range of 14 to 16°C. In particular, said incubation may be performed at a temperature of approx. 16 °C.

In some embodiments, the *Saccharomyces pastorianus* yeast strain is capable of producing a fermented test solution after incubation of said yeast strain in a test solution having an apparent extract of at least 10° Plato and wherein said fermented test solution contains at the most 60 ppb diacetyl, preferably at the most 50 ppb diacetyl at the earliest time point where the apparent extract of said test solution has not decreased by more than 0.25° Plato in the preceding 12 hours after incubation of said yeast strain in said extract wherein said fermentation occurs at a temperature of at the most 18 °C.

Thus, the *Saccharomyces pastorianus* yeast strain according to the invention is preferably capable of producing a fermented test solution, when tested in a method comprising the steps of:
a) Providing a test solution, wherein the test solution is an extract of malt and/or cereals having an apparent extract of at least 10° Plato,
b) incubating said *Saccharomyces pastorianus* yeast strain with said test solution at a temperature of at the most 18°,
c) determining the earliest time point where the apparent extract of said test solution has not decreased by more than 0.25° Plato in the preceding 12 hours during said incubation, and
d) determining the level of diacetyl at said earliest time point,
wherein said test solution contains at the most 60 ppb diacetyl, preferably at the most 50 ppb diacetyl at said earliest time point.

In some embodiments the *Saccharomyces pastorianus* yeast strain is capable of producing a fermented test solution after incubation of said yeast strain in a test solution, wherein the test solution may be any of the test solutions described below, and wherein the fermented test solution contains a maximum level of diacetyl as described below at the earliest time point where the apparent extract of said test solution has not decreased by more than 0.25° Plato, such as not decreased by more than 0.20° Plato, such as not decreased by more than 0.15° Plato, such as not decreased by more than 0.10° Plato in the preceding 24 hours after incubation of said yeast strain in said extract wherein said fermentation occurs at a temperature as specified below. In particular, the apparent extract of the said test solution has not decreased by more than 0.25° Plato in the preceding 12 hours.

Said maximal level of diacetyl is preferably at the most 60 ppb diacetyl, such as at the most 55 ppb diacetyl, such as at the most 50 ppb diacetyl, for example at the most 45 ppb diacetyl at the earliest time point where the apparent extract of said test solution has not decreased by more than the aforementioned ° Plato. In particular, said maximal level of diacetyl at said earliest time point may be 50 ppb.

Aforementioned incubation is preferably performed at a temperature of at the most 18 °C, such as at a temperature of in the range of 12°C to 18 °C. In particular, said incubation may be performed at a temperature of approx. 16 °C.

In some embodiments, the *Saccharomyces pastorianus* yeast strain is capable of producing a fermented test solution upon incubation in a test solution, wherein the test solution is an extract of malt and/or cereal having an apparent extract of at least 10° Plato, wherein said test solution contains at the most 120 ppb diacetyl, for example at the most 60 ppb diacetyl at the earliest time point when the apparent extract of said test solution does not decrease by more than 0.50° Plato in the following 24 hours, wherein said fermentation occurs at a temperature of at the most 18 °C.

Thus, the *Saccharomyces pastorianus* yeast strain according to the invention is preferably capable of producing a fermented test solution, when tested in a method comprising the steps of:
a) Providing a test solution, wherein the test solution is an extract of malt and/or cereals having an apparent extract of at least 10° Plato,
b) incubating said *Saccharomyces pastorianus* yeast strain with said test solution at a temperature of at the most 18°,
c) determining the earliest time point where the apparent extract of said test solution does not decrease by more than 0.50° Plato in the following 24 hours during said incubation, and
d) determining the level of diacetyl at said earliest time point,
wherein said test solution contains at the most 120 ppb diacetyl, for example at the most 60 ppb diacetyl at said earliest time point.

In some embodiments the *Saccharomyces pastorianus* yeast strain is capable of producing a fermented test solution after incubation of said yeast strain in a test solution, wherein the test solution may be any of the test solutions described below, and wherein the fermented test solution contains a maximum level of diacetyl as described below at the earliest time point where the apparent extract of said test solution does not decrease by more than 0.50° Plato, such as does not decrease by more than 0.40° Plato, such as does not decrease by more than 0.30° Plato, such as does not decrease by more than 0.20° Plato in the following 24 hours after incubation of said yeast strain in said extract, wherein said fermentation occurs at a temperature as specified below. In particular, the apparent extract of the said test solution does not decrease by more than 0.50° Plato in the following 24 hours.

Said maximal level of diacetyl is preferably at the most 120 ppb diacetyl, such as at the most 115 ppb diacetyl, for example at the most 110 ppb diacetyl, such as at the most 60 ppb diacetyl at the earliest time point where the apparent extract of said test solution has not decreased by more than the aforementioned ° Plato. In particular, said maximal level of diacetyl at said earliest time point may be 110 ppb. In particular, said maximal level of diacetyl at said earliest time point may be 55 ppb.

Aforementioned incubation is preferably performed at a temperature of at the most 18 °C, such as at a temperature of in the range of 12°C to 18 °C. In particular, said incubation may be performed at a temperature of approx. 16 °C.

In some embodiments, the *Saccharomyces pastorianus* yeast strain is capable of producing a fermented test solution upon incubation in a test solution, wherein the test solution is an extract of malt and/or cereal having an apparent extract of at least 10° Plato, wherein said test solution contains at the most 120 ppb diacetyl, such as at the most 65 ppb diacetyl at the earliest time point when the apparent extract of said test solution does not decrease by more than 0.25° Plato in the following 12 hours, wherein said fermentation occurs at a temperature of at the most 18 °C.

Thus, the *Saccharomyces pastorianus* yeast strain according to the invention is preferably capable of producing a fermented test solution, when tested in a method comprising the steps of:
a) Providing a test solution, wherein the test solution is an extract of malt and/or cereals having an apparent extract of at least 10° Plato,
b) incubating said *Saccharomyces pastorianus* yeast strain with said test solution at a temperature of at the most 18°,
c) determining the earliest time point where the apparent extract of said test solution does not decrease by more than 0.25° Plato in the following 12 hours during said incubation, and
d) determining the level of diacetyl at said earliest time point,
wherein said test solution contains at the most 120 ppb diacetyl, such as at the most 65 ppb diacetyl at said earliest time point.

In some embodiments the *Saccharomyces pastorianus* yeast strain is capable of producing a fermented test solution after incubation of said yeast strain in a test solution, wherein the test solution may be any of the test solutions described below, and wherein the fermented test solution contains a maximum level of diacetyl as described below at the earliest time point where the apparent extract of said test solution does not decrease by more than 0.25° Plato, such as does not decrease by more than 0.20° Plato, such as does not decrease by more than 0.15° Plato, such as does not decrease by more than 0.10° Plato in the following 12 hours, wherein said fermentation occurs at a temperature as specified below. In particular, the apparent extract of the said test solution does not decrease by more than 0.25° Plato in the following 12 hours.

Said maximal level of diacetyl is preferably at the most 120 ppb diacetyl, such as at the most 115 ppb diacetyl, such as at the most 110 ppb diacetyl, for example at the most 105 ppb diacetyl at the earliest time point where the apparent extract of said test solution has not decreased by more than the aforementioned ° Plato. In particular, said maximal level of diacetyl at said earliest time point may be 110 ppb, such as at the most 55 ppb diacetyl.

Aforementioned incubation is preferably performed at a temperature of at the most 18 °C, such as at a temperature of in the range of 12°C to 18 °C. In particular, said incubation may be performed at a temperature of approx. 16 °C.

In some embodiments the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said test solution contains at the most 265 ppb diacetyl at any time point within 5 days after initiation of incubation with the yeast, wherein the incubation is performed at a temperature of at the most 18 °C and wherein in the range of 7 to 15 million viable yeast cells/ml are added to said test solution.

In some embodiments the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said fermented test solution contains at the most 50 ppb diacetyl after incubation with said yeast strain for at the most 6 days. In particular, said fermented test solution may contain at the most 50 ppb diacetyl after incubation with said yeast strain for at the most 5 days. In particular, said fermented test solution may contain at the most 50 ppb diacetyl after incubation with said yeast strain for at the most 4 days.

In some embodiments, the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said test solution contains at the most 50 ppb diacetyl after incubation with said test solution for at the most 5 days at a temperature of at the most 16 °C. In particular, said fermented test solution may contain at the most 50 ppb diacetyl after incubation with said yeast strain for at the most 4 days at a temperature of at the most 16 °C.

In some embodiments, the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said yeast strain is capable of generating at least 4.0 mL/L ethanol per °Plato, when said yeast strain is incubated in said test solution.

In some embodiments, the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said yeast strain is capable of generating at least 4.0 mL/L ethanol per °Plato, when said yeast strain is incubated in said test solution for in the range of 4 to 6 days. In particular, said yeast strain may be incubated in said test solution for approximately 4 days.

In a very preferred embodiment the yeast strain is a yeast strain capable of:
- producing a fermented test solution upon incubation in a test solution, wherein the test solution is an extract of malt and/or cereal having an apparent extract of at least 10° Plato, wherein said fermented test solution contains at the most 60 ppb diacetyl at the earliest time point when the apparent extract of said test solution has not decreased by more than 0.50° Plato in the preceding 24 hours;
- producing a fermented test solution upon incubation in a test solution, wherein the test solution is an extract of malt and/or cereal having an apparent extract of at least 10° Plato, wherein said fermented solution contains at the most 60 ppb diacetyl, preferably at the most 50 ppb diacetyl at the earliest time point when the apparent extract of said test solution has not decreased by more than 0.25° Plato in the preceding 12 hours;
- producing a fermented test solution upon incubation in a test solution, wherein the test solution is an extract of malt and/or cereal having an apparent extract of at least 10° Plato, wherein said fermented test solution contains at the most 120 ppb diacetyl, such as at the most 60 ppb diacetyl at the earliest time point when the apparent extract of said test solution does not decrease by more than 0.50° Plato in the following 24 hours; and
- producing a fermented test solution upon incubation in a test solution, wherein the test solution is an extract of malt and/or cereal having an apparent extract of at least 10° Plato, wherein said fermented test solution contains at the most 120 ppb diacetyl, such as at the most 60 ppb diacetyl at the earliest time point when the apparent extract of said test solution does not decrease by more than 0.25° Plato in the following 12 hours;
wherein fermentation occurs as described herein in example 6.

In some embodiments, the yeast strain has high flocculation. Flocculation may for example be determined as cells in suspension after fermentation. "Cells in suspension" is in general determined by counting the yeast cells in a sample taken from the upper ¾, for example from the upper 2/3, such as from the upper half of the container comprising the fermented test solution. If the fermentation is performed in a conical cylindrical tank, said sample is preferably taken above the cone. A low number of cells in solution after fermentation is indicative of high flocculation.

In some embodiments, the *Saccharomyces pastorianus* yeast strain is capable of producing a fermented test solution after incubation of said yeast strain in a test solution, wherein the test solution is an extract of malt and/or cereal having an apparent extract of at least 10° Plato, and wherein said test solution contains at the at the most 10 million cells per millilitre, such as at the most 9.5 million cells per millilitre, such as at the most 9.0 million cells per millilitre, such as at the most 8.5 million cells per millilitre, such as at the most 8.0 million cells per millilitre in solution, at the earliest time point where the apparent extract of said test solution has not decreased by more than 0.50° Plato in the preceding 24 hours.

In some embodiments, the *Saccharomyces pastorianus* yeast strain is capable of growing on media with melibiose as the sole carbon source.

In preferred embodiments, the yeast strain as disclosed herein is a non-GMO organism. Thus, in preferred embodiments, said yeast strain has not undergone a step of genetic engineering.

### Test solution

In some embodiments, the test solution is a wort having an apparent extract of approximately 16° Plato. In some embodiments, the test solution comprises at least 40 g/kg maltose.

The test solution is in general an extract of malt and/or cereal having an apparent extract of at least 10° Plato. Thus the test solution may in particular be wort. The test solution may preferably have an apparent extract in the range of 12 to 18° Plato, and more preferably an apparent extract of approx. 16° Plato. The test solution may further comprise in the range of 0.20 mg/L to 0.40 mg/L zinc and pH may be adjusted to in the range of 4.0 to 6.0.

One example of a test solution comprises glucose in the range of 12-25 g/L, maltose in the range of 60-80 g/L, maltotriose in the range of 15-20 g/L, zinc in the range of
0.20 mg/L to 0.40 mg/L, Free alpha amino nitrogen (FAN) in the range of 110-250 mg/L and Valine/FAN ratio of 0.6 to 0.8.

In some embodiments the test solution is made from pilsner malt, potentially with addition of barley adjunct.

### Genotype

The *Saccharomyces pastorianus* yeast strains according to the invention have the phenotype that they are capable producing a fermented test solution with reduced diacetyl level as described in the section with the heading "yeast strain" above.

In addition to said phenotypic characteristic, the yeast strains according to the invention may preferably have one or more of the genotypes described herein below.

ILV2, ILV6, ILV5, ILV3, BAT1 and BAT2 are involved in synthesizing L-valine from pyruvate. Spontaneous production of diacetyl may occur in this process.

An acetolactate synthase small subunit (ILV6) and an acetolactate synthase catalytic subunit (ILV2) convert pyruvate to alpha-acetolactate.

Ketol-acid reductoisomerase (ILV5) catalyses conversion of alpha-acetolactate to 2,3 hydroxy-isovalerate .

Dihydroxy-acid dehydratase (ILV3) catalyses conversion of 2,3 hydroxy-isovalerate to 2-keto isolaterate.

The branched-chain-amino-acid aminotransferase (BAT1) catalyses conversion of 2-keto isolaterate to L-valine in the mitochondria and the branched-chain-amino-acid aminotransferase (BAT2) catalyses said reaction in the cytosol.

### Chromosome location:

| **Gene** | **Chromosome** | **Start-end** |
|---|---|---|
| ***ILV2*** | 13 | 484084..486147 |
| ***ILV6*** | 3 | 104619..105548 |
| ***ILV5*** | 12 | 838066..839253 |
| ***ILV3*** | 10 | 464451 ..466208 |
| ***BAT1*** | 8 | 517532..518713 |
| ***BAT2*** | 10 | 705744..706874 |

### Protein similarity S. Cerevisiae and S. eubayanus (protein sequences)

| | **Seq. Identity %** | **Length of aa sequence** |
|---|---|---|
| **ILV2** | 93.17 | 688 |
| **ILV6** | 95.81 | 310 |
| **ILV5** | 96.97 | 396 |
| **ILV3** | 96.76 | 586 |
| **BAT1** | 94.42 | 394 |
| **BAT2** | 90.45 | 377 |

It is understood that when a yeast cell "has" an exact number of genes, said cell does not contain more genes than the indicated number. Similarly, if a yeast cell "has" in the range of XX to YY genes, said yeast cell does not contain more than YY genes.

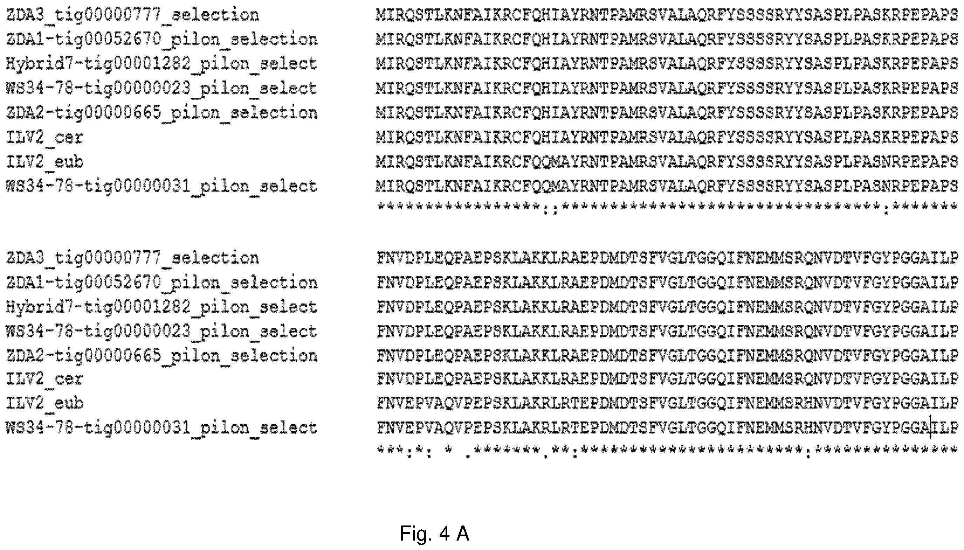

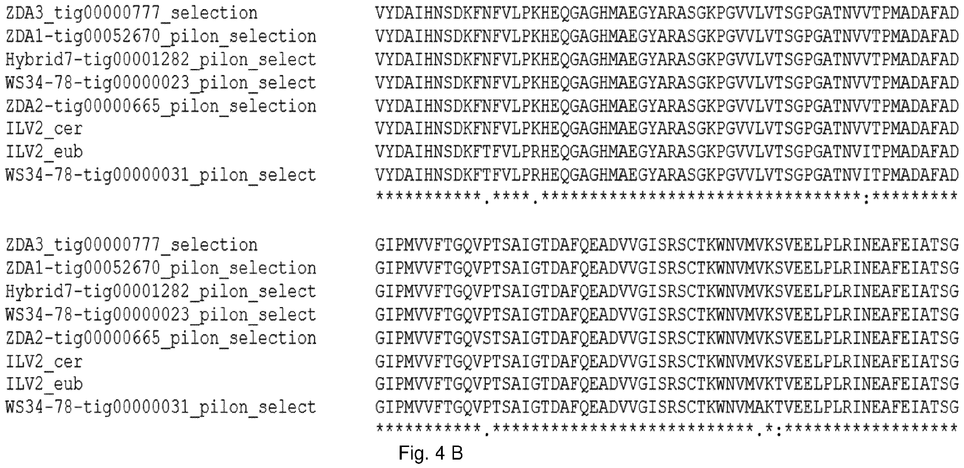

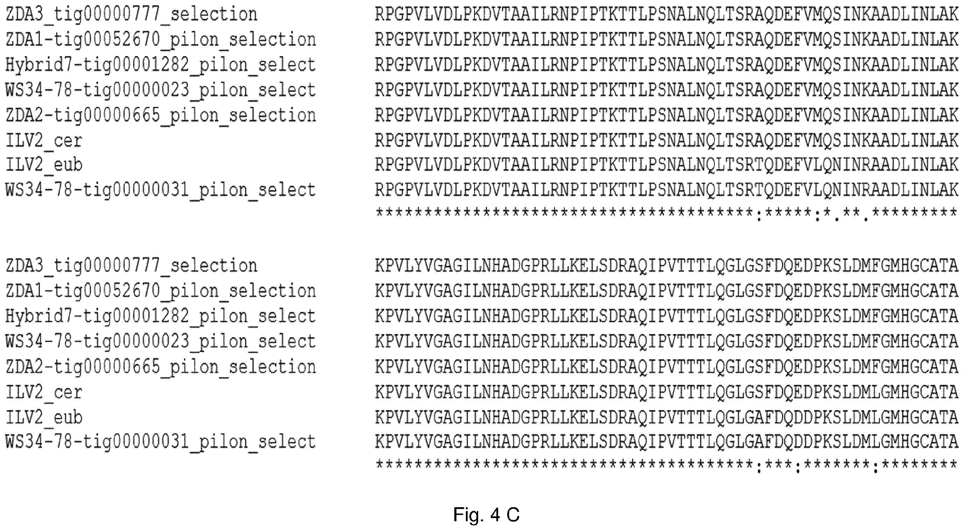

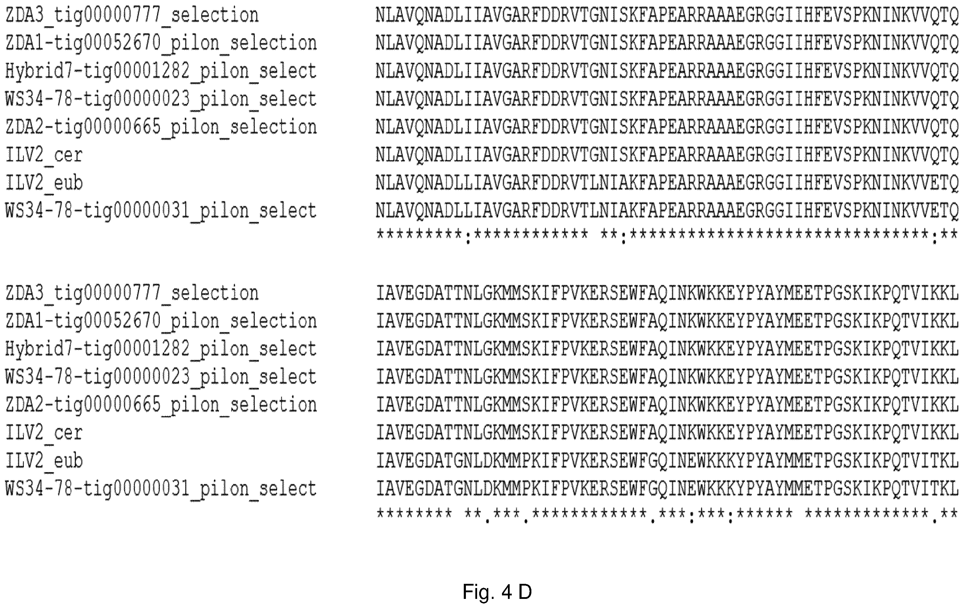

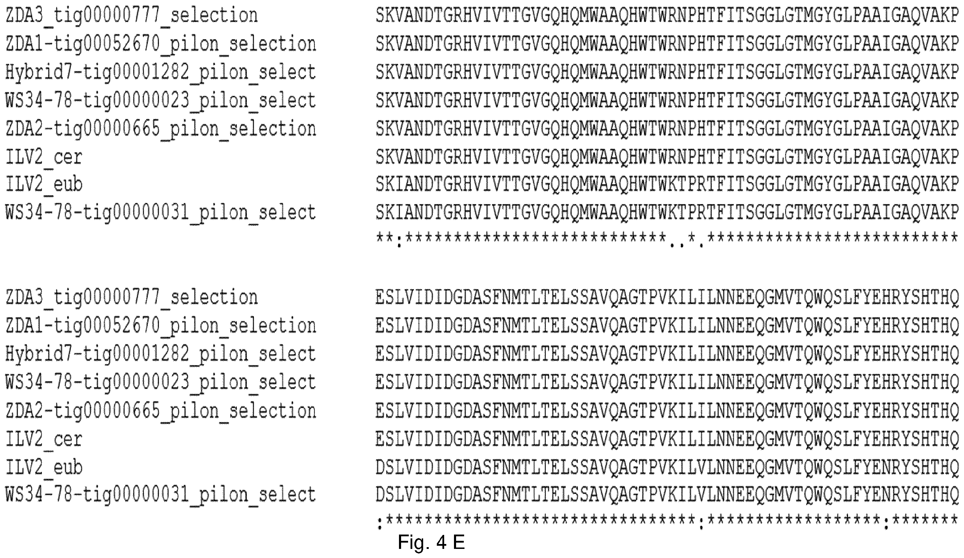

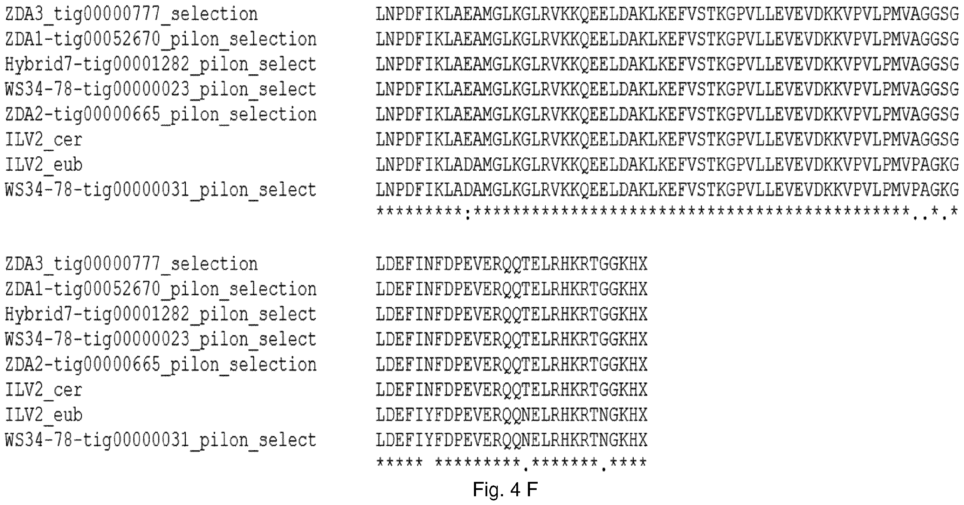

In some embodiments, the *Saccharomyces pastorianus* yeast strain has at the most five genes encoding functional ILV2, for example at the most 4 genes encoding functional ILV2, such as in the range of 1 to 5 genes encoding functional ILV2, such as in the range of 1 to 4 genes encoding functional ILV2, wherein each gene encoding ILV2 encodes ScILV2 of SEQ ID NO: 32 or a functional homologue sharing at least 80%, such as at least 85%, such as at least 90%, such as at least 95%, such as at least 98% sequence identity therewith, or encodes SelLV2 of SEQ ID NO: 38 or a homologue sharing at least 80%, such as at least 85%, such as at least 90%, such as at least 95%, such as at least 98% sequence identity therewith. It is preferred that the yeast strain of the invention has at the most two functional genes encoding ILV2, for example the yeast strain may have in the range of 1 to 2, such as exactly 2 functional genes encoding ILV2. Preferably, said functional genes encodes SclLV2 of SEQ ID NO: 32 or SelLV2 of SEQ ID NO: 38 or a functional homologue of any of the aforementioned sharing at least 80%, such as at least 85%, such as at least 90%, such as at least 95%, such as at least 98% sequence identity therewith. In one embodiment said functional genes encode SclLV2, preferably ScILV of SEQ ID NO:32. In one embodiment said functional genes encode ILV2 of ZDA1, ZDA2 and/or ZDA3, wherein the sequences of ILV2 of ZDA1, ZDA2 and/or ZDA3 are provided in figure 4.

In some embodiments, the yeast strain has at the most two functional genes encoding ILV2, wherein each gene encoding ILV2 encodes SclLV2 of SEQ ID NO: 32 or SelLV2 of SEQ ID NO: 38, or a functional homologue sharing at least 80% sequence identity herewith; at least four functional genes encoding ILV3, such as at least five functional genes encoding ILV3, such as at least six functional genes encoding ILV3, wherein each gene encoding ILV3 encodes ScILV3 of SEQ ID NO: 35 or SelLV3 of SEQ ID NO: 41, or a functional homologue sharing at least 80% sequence identity herewith; and at least three functional genes encoding ILV5, such as at least four functional genes encoding ILV5, such as at least five functional genes encoding ILV5, wherein each gene encoding ILV5 encodes ScILV5 of SEQ ID NO: 34 or SelLV5 of SEQ ID NO: 40, or a functional homologue sharing at least 80% sequence identity herewith.

In some embodiments, the yeast strain has at the most two functional genes encoding ILV2, wherein each gene encoding ILV2 encodes ScILV2 of SEQ ID NO: 32 or SelLV2 of SEQ ID NO: 38 or a functional homologue of any of the aforementioned sharing at least 80%, such as at least 85%, such as at least 90%, such as at least 95%, such as at least 98% sequence identity therewith; and in the range of 5 to 9 functional genes encoding ILV3, such as at in the range of 5 to 7, such as 6 functional genes encoding ILV3, wherein each gene encoding ILV3 encodes ScILV3 of SEQ ID NO: 35 or SelLV3 of SEQ ID NO: 41 or a functional homologue of any of the aforementioned sharing at least 80%, such as at least 85%, such as at least 90%, such as at least 95%, such as at least 98% sequence identity therewith; and in the range of 4 to 8 functional genes encoding ILV5, such as in the range of 4 to 6, such as 5 functional genes encoding ILV5, wherein each gene encoding ILV5 encodes ScILV5 of SEQ ID NO: 34 or SelLV5 of SEQ ID NO: 40 or a functional homologue of any of the aforementioned sharing at least 80%, such as at least 85%, such as at least 90%, such as at least 95%, such as at least 98% sequence identity therewith.

In some embodiments, the *Saccharomyces pastorianus* yeast strain has at the most four genes encoding functional ILV6, for example in the range of two to four genes encoding functional ILV6, wherein each gene encoding ILV6 encode SclLV6 of SEQ ID NO: 33 or a functional homologue sharing at least 80%, such as at least 85%, such as at least 90%, such as at least 95%, such as at least 98% sequence identity therewith, or encode SelLV6 of SEQ ID NO: 39 or a functional homologue sharing at least 80%, such as at least 85%, such as at least 90%, such as at least 95%, such as at least 98% sequence identity therewith.

In some embodiments, the *Saccharomyces pastorianus* yeast strain has at least two genes encoding functional ILV5, such as at least four genes encoding functional ILV5, such as at least five genes encoding functional ILV5, wherein the each gene encoding ILV5 encodes ScILV5 of SEQ ID NO: 34 or a functional homologue sharing at least 80%, such as at least 85%, such as at least 90%, such as at least 95%, such as at least 98 up to 99% sequence identity herewith, or encodes SelLV5 of SEQ ID NO: 40 or a functional homologue sharing at least 80%, such as at least 85%, such as at least 90%, such as at least 95%, such as at least 98% up to 99% sequence identity herewith. In some embodiments, the *Saccharomyces pastorianus* yeast strain has at the most 20 genes, such as at the most 15 genes, such as at the most 10 allelic genes encoding functional ILV5, wherein the gene encoding functional ILV5 may be any of the aforementioned. In some embodiments, the *Saccharomyces pastorianus* yeast strain has in the range of 5 to 20 allelic genes, such as 5 to 15 allelic genes, such as 5 to 10 genes encoding functional ILV5, wherein the gene encoding functional ILV5 may be any of the aforementioned.

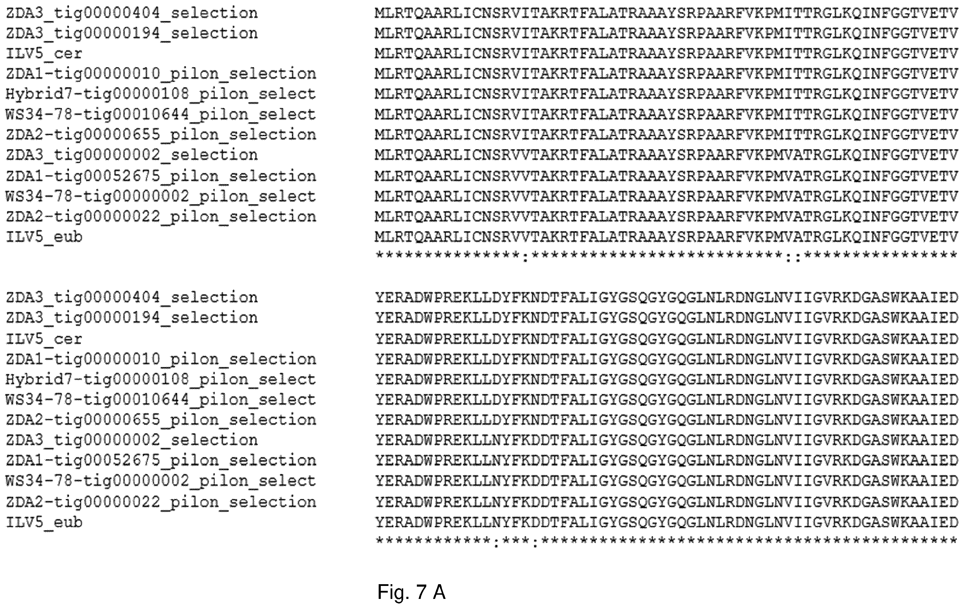

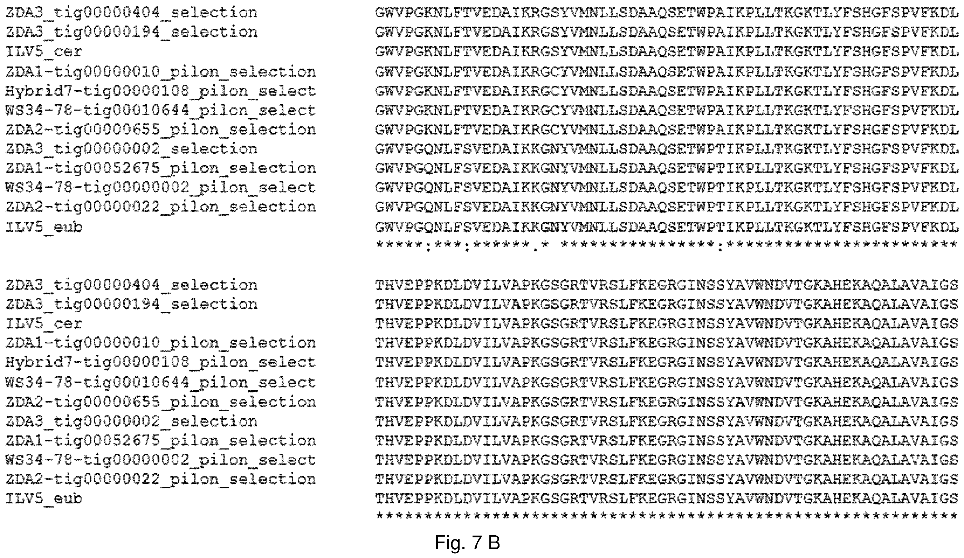

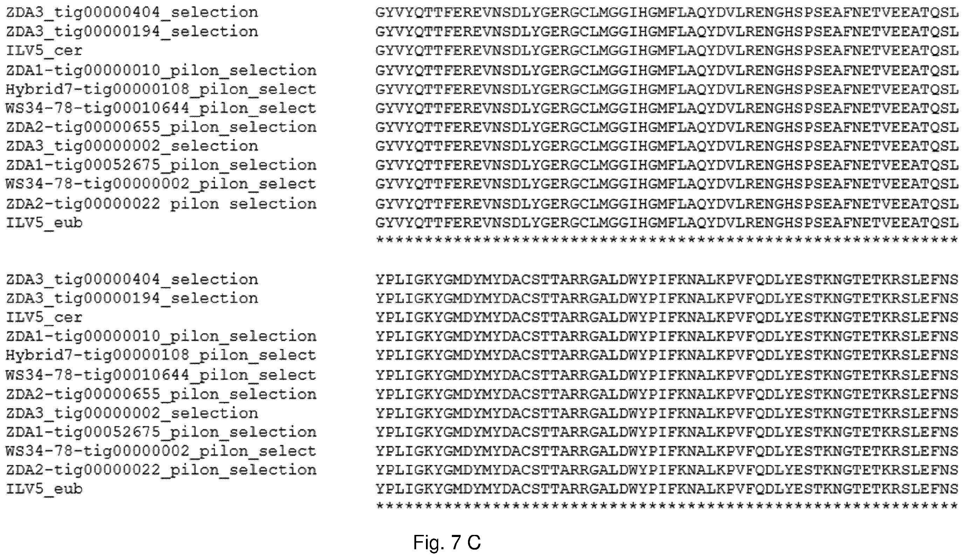

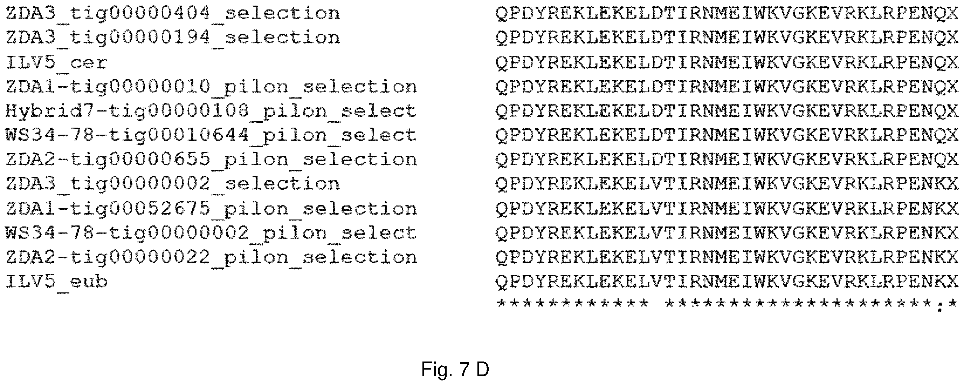

In one embodiment, the yeast strain has in the range of 4 to 8 functional genes encoding ILV5, such as in the range of 4 to 6, such as 5 functional genes encoding ILV5, wherein each gene encoding ILV5 encodes ScILV5 of SEQ ID NO: 34 or SeILV5 of SEQ ID NO: 40 or a functional homologue of any of the aforementioned sharing at least 80%, such as at least 85%, such as at least 90%, such as at least 95%, such as at least 98% sequence identity therewith. In one embodiment, the yeast strain has in the range of 4 to 8 functional genes encoding ILV5, such as in the range of 4 to 6, such as 5 functional genes encoding ILV5, wherein each gene encode ILV5 of ZDA1, ZDA2 and/or ZDA3, wherein the sequences of ILV5 of ZDA1, ZDA2 and/or ZDA3 are given in figure 7.

In some embodiments, the *Saccharomyces pastorianus* yeast strain has at least three genes encoding functional ILV3, such as at least four genes encoding functional ILV3, for example as at least six genes encoding functional ILV3, wherein each gene encoding functional ILV3 encodes ScILV3 of SEQ ID NO: 35 or a functional homologue sharing at least 80%, such as at least 85%, such as at least 90%, such as at least 95%, such as at least 98% up to 99% sequence identity herewith, or encodes SelLV3 of SEQ ID NO: 41 or a homologue sharing at least 80%, such as at least 85%, such as at least 90%, such as at least 95%, such as at least 98% up to 99% sequence identity herewith. In some embodiments, the *Saccharomyces pastorianus* yeast strain has at the most 20 allelic genes, such as at the most 15 allelic genes, such as at the most 10 allelic genes encoding ILV3, wherein each gene encoding functional ILV3 may be any of the aforementioned. In some embodiments, the *Saccharomyces pastorianus* yeast strain has in the range of 5 to 20 allelic genes, such as 5 to 15 allelic genes, such as 5 to 10 genes encoding functional ILV3, wherein the gene encoding functional ILV3 may be any of the aforementioned.

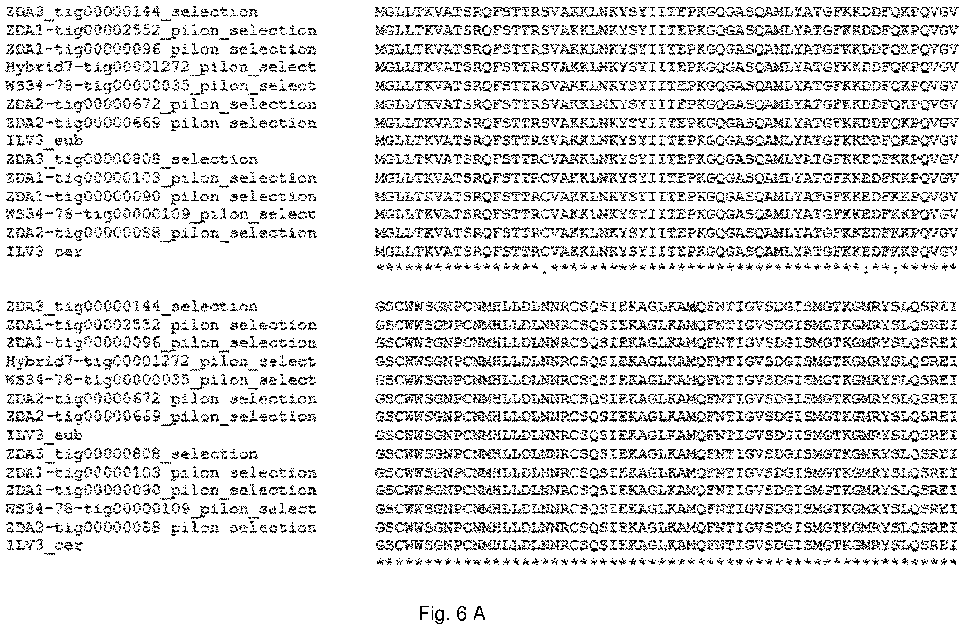

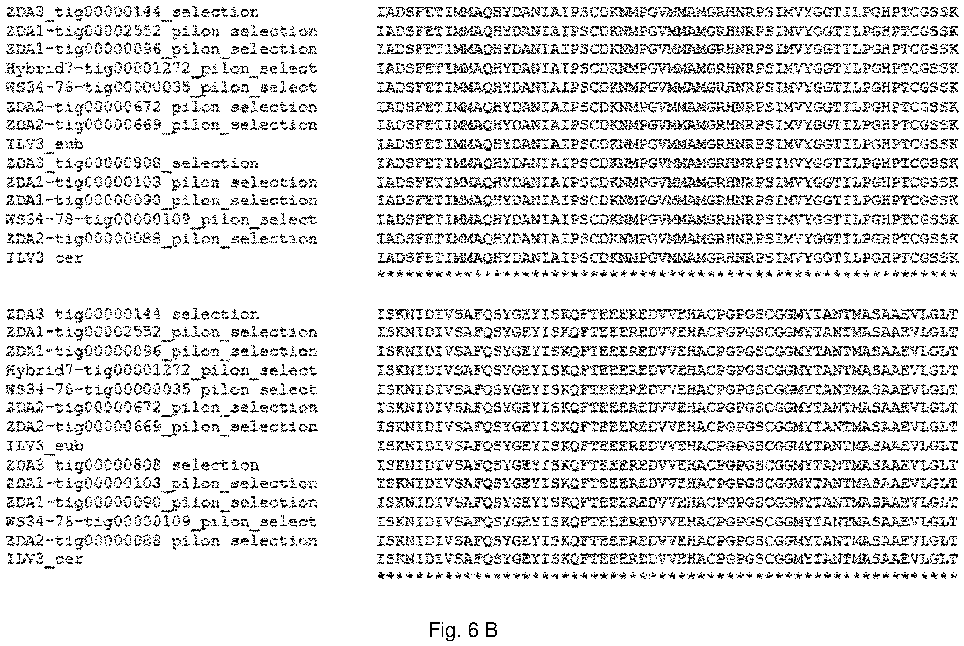

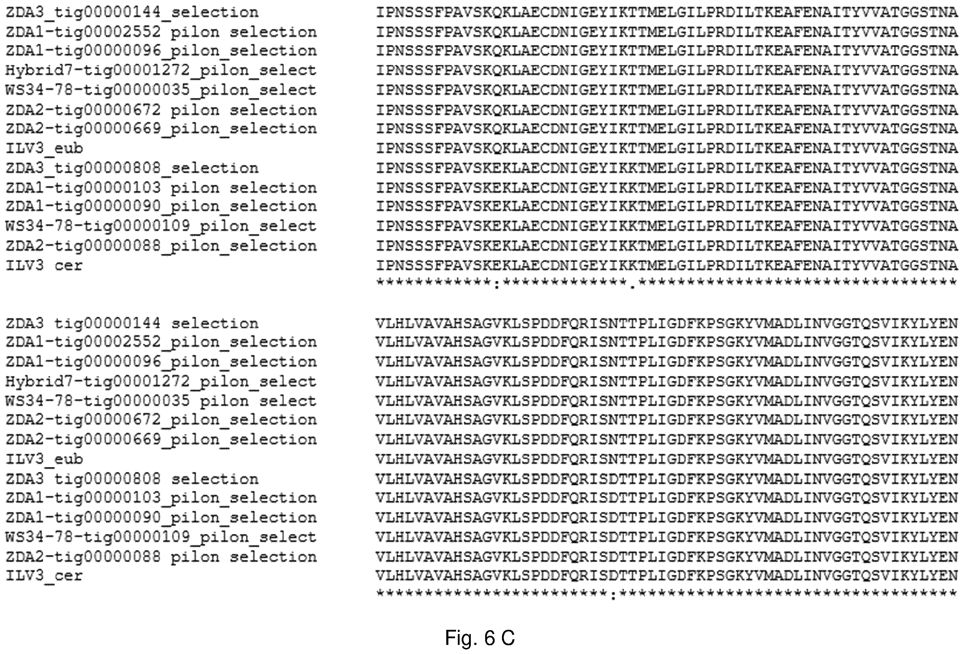

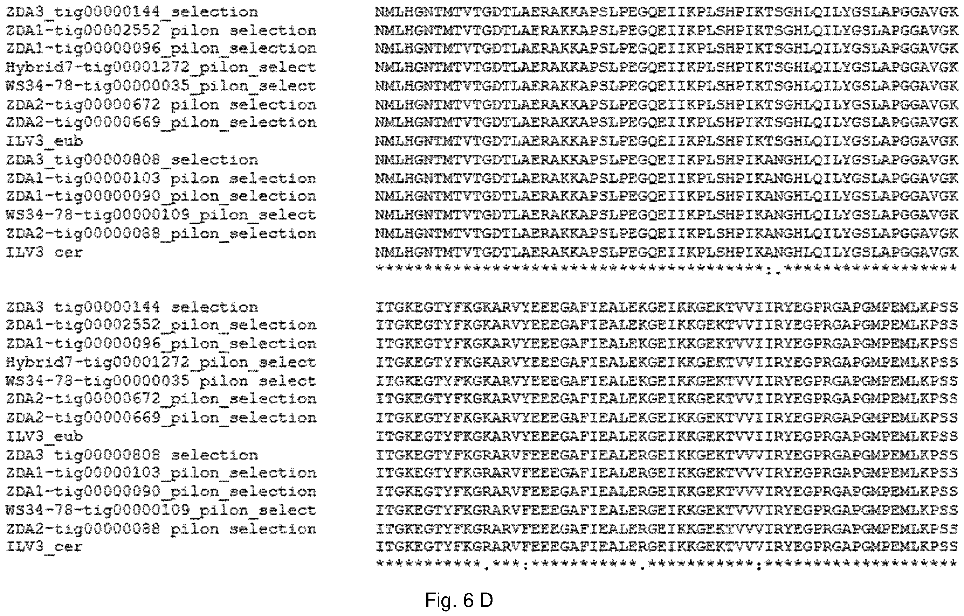

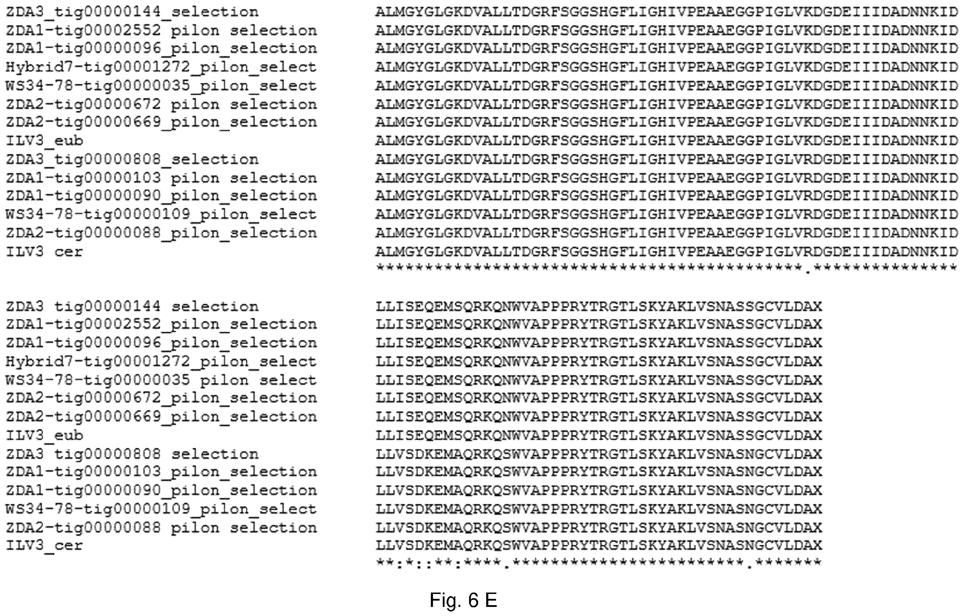

In one embodiment the yeast of the invention has in the range of 5 to 9 functional genes encoding ILV3, such as at in the range of 5 to 7, such as 6 functional genes encoding ILV3, wherein each gene encoding ILV3 encodes ScILV3 of SEQ ID NO: 35 or SeILV3 of SEQ ID NO: 41 or a functional homologue of any of the aforementioned sharing at least 80%, such as at least 85%, such as at least 90%, such as at least 95%, such as at least 98% sequence identity therewith. In one embodiment the yeast of the invention has in the range of 5 to 9 functional genes encoding ILV3, such as at in the range of 5 to 7, such as 6 functional genes encoding ILV3, wherein each gene encode ILV3 of ZDA1, ZDA2 and/or ZDA3, wherein the sequences of ILV3 of ZDA1, ZDA2 and/or ZDA3 are given in figure 6.

In some embodiments, the *Saccharomyces pastorianus* yeast strain has at least three genes encoding functional BAT1, such as at least four genes encoding functional *BAT1,* for example at least five genes encoding functional BAT1, wherein each gene encoding functional *BAT1* encodes ScBAT1 of SEQ ID NO: 36 or a functional homologue sharing at least 80%, such as at least 85%, such as at least 90%, such as at least 95%, such as at least 98% up to 99% sequence identity herewith, or encodes SeBAT1 of SEQ ID NO: 42 or a functional homologue sharing at least 80%, such as at least 85%, such as at least 90%, such as at least 95%, such as at least 98% up to 99% sequence identity herewith. In some embodiments, the *Saccharomyces pastorianus* yeast strain has at the most 20 genes, such as at the most 15 genes, such as at the most 10 genes encoding BAT1, wherein each gene encoding BAT1 may be any of the aforementioned. In some embodiments, the *Saccharomyces pastorianus* yeast strain has in the range of 5 to 20 allelic genes, such as 5 to 15 allelic genes, such as 5 to 10 allelic genes encoding functional BAT1, wherein each allelic gene encoding functional BAT1 may be any of the aforementioned.

In some embodiments, the *Saccharomyces pastorianus* yeast strain has at least four genes encoding functional BAT2, such as at least five genes encoding functional BAT2, for example at least six genes encoding functional BAT2, wherein each gene encoding functional *BAT2* encodes ScBAT2 of SEQ ID NO: 37 or a functional homologue sharing at least 80%, such as at least 85%, such as at least 90%, such as at least 95%, such as at least 98% up to 99% sequence identity herewith, or encodes SeBAT2 of SEQ ID NO: 43 or a functional homologue sharing at least 80%, such as at least 85%, such as at least 90%, such as at least 95%, such as at least 98% up to 99% sequence identity herewith. In some embodiments, the *Saccharomyces pastorianus* yeast strain has at the most 20 genes, such as at the most 15 genes, such as at the most 10 genes encoding functional BAT2, wherein the gene encoding functional BAT2 may be any of the aforementioned. In some embodiments, the *Saccharomyces pastorianus* yeast strain has in the range of 5 to 20 genes, such as 5 to 15 genes, such as 5 to 10 genes encoding functional BAT2, wherein each gene encoding functional BAT2 may be any of the aforementioned.

In some embodiments, the *Saccharomyces pastorianus* yeast strain carries a mutation in or a deletion of one or more *ILV2* genes.

In some embodiments, the *Saccharomyces pastorianus* yeast strain carries a frameshift mutation in one or more *ILV2* genes.

In some embodiments, the *Saccharomyces pastorianus* yeast strain carries a mutation resulting in lower or no expression of one or more *ILV2* genes.

In some embodiments, the *Saccharomyces pastorianus* yeast strain carries a mutation in or a deletion of one or more *ILV6* genes.

In some embodiments, the *Saccharomyces pastorianus* yeast strain carries a frameshift mutation in one or more *ILV6* genes.

In some embodiments, the *Saccharomyces pastorianus* yeast strain carries a mutation resulting in lower or no expression of one or more *ILV6* genes.

In some embodiments of the present invention, the sum of the genes encoding functional ILV2 and functional ILV6 is lower than the sum of the genes encoding functional ILV5 and functional ILV3, wherein said functional ILV2, ILV6, ILV3 and ILV5 may be any of the aforementioned. In some embodiments of the present invention, the sum of the genes encoding functional ILV2 is lower than the sum of the genes encoding functional ILV5 and functional ILV3, wherein said functional ILV2, ILV5 and ILV3 may be any of the aforementioned. In one embodiment, the sum of the genes encoding functional ILV2 and functional ILV6 is lower than the sum of the genes encoding functional ILV5, functional ILV3, functional BAT1 and functional BAT2, wherein said functional ILV2, ILV6, ILV3, ILV5; BAT1 and BAT2 may be any of the aforementioned. In other words, in some embodiments the sum of the genes encoding ILV5 and ILV3 is higher than the sum of the genes encoding ILV2 and ILV6. Thus, in some embodiments, the sum of the genes encoding ILV5 and ILV3 is higher than the sum of the genes encoding ILV2, ILV6, BAT1 and BAT2.

In some embodiments, the gene ratio of *ILV5* and *ILV3* genes vs. *ILV2* genes is at least 1, such as at least 1.5, such as at least 2, such as at least 2.5, such as at least 3, wherein said *ILV2*, *ILV5* and *ILV3* genes encodes any of the aforementioned functional ILV2, ILV5 and ILV3, respectively.

In some embodiments, the gene ratio of *ILV5* and *ILV3* genes vs. *ILV2* and *ILV6* genes is at least 1, such as at least 1.2, such as at least 1.4, such as at least 1.6, such as at least 1.8 or such as at least 2, wherein said *ILV2, ILV6*, *ILV5* and *ILV3* genes encodes any of the aforementioned functional ILV2, ILV6, ILV5 and ILV3, respectively.

In some embodiments, the gene ratio of *ILV5, ILV3, BAT1* and *BAT2* vs. *ILV2* is at least 1, such as at least 1.5, such as at least 2, such as at least 2.5, such as at least 3, such as at least 4, such as at least 5, such as at least 6, wherein said *ILV2, ILV5, ILV3, BAT1* and *BAT2* genes encode any of the aforementioned functional ILV2, ILV5, ILV3, BAT1 and BAT2 respectively.

In some embodiments, the gene ratio of *ILV5, ILV3, BAT1* and *BAT2 vs. ILV2* and *ILV6* is at least 1, such as at least 1.5, such as at least 2, such as at least 2.5, such as at least 3, such as at least 4, wherein said *ILV2, ILV6, ILV5, ILV3, BAT1* and *BAT2* genes encodes any of the aforementioned functional ILV2, ILV6, ILV5, ILV3, BAT1 and BAT2 respectively.

### Malt and/or cereal based fermented aqueous extract and methods of production thereof

The invention provides *Saccharomyces pastorianus* yeast strains described in the section with the heading "yeast strain" above, as well as methods of preparing malt and/or cereal based fermented aqueous extract, using said yeast strains.

It is an aspect of the invention to provide methods of producing a fermented aqueous extract, said method comprising the steps of:
i) providing an aqueous extract of malt and/or cereal;
ii) providing a yeast strain, wherein said yeast strain may be any of the yeast strains described herein above in the section "Yeast strain"; and
iii) fermenting the aqueous extract provided in step i) with said yeast strain of step ii),
thereby obtaining a fermented aqueous extract.

The aqueous extract, may be any aqueous extract of malt and/or cereal kernels. Thus, a non-limiting example hereof is wort. The aqueous extract may for example be prepared by preparing an extract of malt by mashing and optionally sparging as described herein in this section below.

Malt is cereal kernels, such as barley kernels, that have been malted. By the term "malting" is to be understood a process involving steeping and germination of kernels in a process taking place under controlled environmental conditions, optionally followed by a drying step. Said drying step may preferably be kiln drying of the germinated kernels at elevated temperatures. Green malt, which has not been subject to kilning may also be used, in particular malt obtained by the process described in WO 2018/001882.

Malting is important for the synthesis of numerous enzymes that cause kernel modification, processes that principally depolymerize starch and cell walls of the dead endosperm to mobilize the kernel nutrients and activate other depolymerases. In the subsequent drying process, flavour and colour are generated at least partly due to chemical browning reactions.

Steeping may be performed by any conventional method known to the skilled person. One non-limiting example involves steeping at a temperature in the range of 10°C to 25°C with alternating dry and wet conditions. Germination may be performed by any conventional method known to the skilled person. One non-limiting example involves germination at a temperature in the range of 10 to 25°C, optionally with changing temperature in the range of 1 to 4 h. Steeping and germination may also be performed in a combined method, e.g. as described in international patent application WO 2018/001882.

The kiln drying may be performed at conventional temperatures, such as at least 75°C, for example in the range of 80 to 90°C, such as in the range of 80 to 85°C. Thus, the malt may, for example be produced by any of the methods described by Briggs et al. (1981) and by Hough et al. (1982). However, any other suitable method for producing malt may also be used with the present invention, such as methods for production of specialty malts, including, but not limited to, methods of roasting the malt.

Malt may be further processed, for example by milling. Milling can be performed in a dry state, i.e. the malt is milled while dry or in a wet state if green malt is used.

The malt, e.g. the milled malt may be mashed to prepare an aqueous extract of said malt. The starting liquid for preparing the beverage may be an aqueous extract of malt, e.g. an aqueous extract of malt prepared by mashing.

Thus, the method for preparing a malt and/or cereal based fermented aqueous extract according to the invention may comprise a step of producing an aqueous extract, such as wort, by mashing malt and optionally additional adjuncts. Said mashing step may also optionally comprise sparging, and accordingly said mashing step may be a mashing step including a sparging step or a mashing step excluding a sparging step.

In general, the production of the aqueous extract is initiated by the milling of malt and/or kernels. If additional adjuncts are added, these may also be milled depending on their nature. If the adjunct is a cereal, it may for example be milled, whereas syrups, sugars and the like will generally not be milled. Milling will facilitate water access to kernel particles in the mashing phase. During mashing enzymatic depolymerization of substrates initiated during malting may be continued.

In general, the aqueous extract is prepared by combining and incubating milled malt and water, i.e. in a mashing process. During mashing, the malt/liquid composition may be supplemented with additional carbohydrate-rich adjunct compositions, for example milled barley, maize, or rice adjuncts. Unmalted cereal adjuncts usually contain little or no active enzymes, making it important to supplement with malt or exogenous enzymes to provide enzymes necessary for polysaccharide depolymerization etc. During mashing, milled malt and/or milled kernels - and optionally additional adjuncts are incubated with a liquid fraction, such as water. The incubation temperature is in general either kept constant (isothermal mashing), or gradually increased, for example increased in a sequential manner. In either case, soluble substances in the malt/kernel/adjuncts are liberated into said liquid fraction. A subsequent filtration confers separation of the aqueous extract and residual solid particles, the latter also denoted "spent kernel". The aqueous extract thus obtained may also be denoted "first wort". Additional liquid, such as water may be added to the spent kernels during a process also denoted sparging. After sparging and filtration, a "second wort" may be obtained. Further worts may be prepared by repeating the procedure. Non-limiting examples of suitable procedures for preparation of wort is described by Briggs et al. (1981) and Hough et al. (1982).

As mentioned above, the aqueous extract may also be prepared by mashing only unmalted kernels. Unmalted kernels lack or contain only a limited amount of enzymes beneficial for wort production, such as enzymes capable of degrading cell walls or enzymes capable of depolymerising starch into sugars. Thus, in embodiments of the invention where up to 80%, such as 90% or such as 100% of unmalted kernels, such as barley kernels, are used for mashing, it is preferred that one or more suitable, external brewing enzymes are added to the mash Suitable enzymes may be lipases, starch degrading enzymes (e.g. amylases), glucanases [preferably (1-4)- and/or (1-3,1-4)-β-glucanase], and/or xylanases (such as arabinoxylanase), and/or proteases, or enzyme mixtures comprising one or more of the aforementioned enzymes, e.g. Cereflo, Ultraflo, or Ondea Pro (Novozymes).

The aqueous extract may also be prepared by using a mixture of malted and unmalted kernels, in which case one or more suitable enzymes may be added during preparation. Even in embodiments, where malt is used enzymes may also be added. More specifically, kernels can be used together with malt in any combination for mashing - with or without external brewing enzymes - such as, but not limited to, the proportions of kernel: malt = approximately 100 : 0, or approximately 75 : 25, or approximately 50 : 50, or approximately 25 : 75.

The aqueous extract obtained after mashing may also be referred to as "sweet wort". In conventional methods, the sweet wort is boiled with or without hops where after it may be referred to as boiled wort.

The aqueous extract may be heated or boiled before it is subjected to fermentation with the yeast of the invention. In one aspect of the invention, second and further worts may be combined, and thereafter subjected to heating or boiling. The aqueous extract may be heated or boiled for any suitable amount of time, e.g. in the range of 60 min to 120 min.

The outcome of the malt and/or cereal based fermented aqueous extract is highly dependent on the amount and type of fermentable sugars present in the aqueous extract of malt and/or cereal kernels, as well as the characteristics of the yeast strain used during fermentation.

In some embodiments of the present invention, said aqueous extract has an apparent extract of at least 12° Plato, such as at least 15° Plato, such as in the range of 5-15° Plato, such as in the range of 10-20° Plato, such as in the range of 15-25° Plato.

In some embodiments, said aqueous extract is fermented with said yeast strain for at the most 6 days, such as for the most 5 days, such as for the most 4 days, such as for the most 3 days.

An additional advantage of the yeast strains of the present invention may be that they are useful for fermentation of wort with low levels of amino acids. Thus, in some embodiments, said aqueous extract comprises at the most 3500 mg/L, such as at the most 3000 mg/L, for example at the most 2500 mg/L amino acids.

Thus, the aqueous extract, e.g. wort, may be prepared as described above. The malt and/or cereal based fermented aqueous extract may be prepared by fermentation of said aqueous extract with said yeast strain according to the invention.

In some preferred embodiments, said fermented aqueous extract is a green beer.

In general terms, alcoholic fermented aqueous extracts - such as beer - may be manufactured from malted and/or unmalted kernels. Malt, in addition to hops and yeast, contributes to flavor and color of the beverage, such as beer. Furthermore, malt functions as a source of fermentable sugar and enzymes. Non-limited descriptions of examples of suitable methods for malting and brewing can be found, for example, in publications by Briggs et al. (1981) and Hough et al. (1982). Numerous, regularly updated methods for analyses of kernel, malt and beer products are available, for example, but not limited to, American Association of Cereal Chemists (1995), American Society of Brewing Chemists (1992), European Brewery Convention (1998), and Institute of Brewing (1997). It is recognized that many specific procedures are employed for a given brewery, with the most significant variations relating to local consumer preferences. Any such method of producing beer may be used with the present invention.

The first step of producing beer from wort preferably involves heating said wort as described herein above, followed by a subsequent phase of wort cooling and optionally whirlpool rest.

The methods of the invention comprises a step of fermenting an aqueous extract of malt and/or cereal kernels with the yeast strain according to the invention. Said fermentation may be a fermentation of an unfermented aqueous extract or a fermented aqueous extract still containing fermentable sugars for the yeast. Thus, in some embodiments said fermentation may be performed essentially immediately after completion of mashing or after heating of wort.

Fermentation may be performed in fermentation tanks containing yeast according to the invention, i.e. yeast having one or more of the characteristics described herein above.

During the several-day-long fermentation process, flavour substances are developed. If the yeast strain is not capable of converting specific compounds, these will still be present after the fermentation step iii).

In some embodiments, the fermentation step iii) occurs at any of the temperatures specified below, wherein the aqueous extract has been incubated with any of the numbers of yeast cells as described below, wherein the fermented aqueous extract contains a maximum level of diacetyl as described below, and where fermentation is complete after a maximum of 5 days, such as a maximum of 4 days, such as a maximum of 3 days. In particular, fermentation is complete after a maximum of 4 days.

In a specific embodiment the fermentation using a yeast strain of the invention is complete and the diacetyl is below 60 ppb, such as 50 ppb at least 12 hours, such as at least 24 hours earlier than a fermentation conducted with W-34/78 yeast strain under the same conditions.

Aforementioned fermentation is preferably performed at a temperature of at the most 18 °C, such as at a temperature of in the range of 12°C to 18 °C. In particular, said fermentation may be performed at a temperature of approx. 16 °C.

Said maximal level of diacetyl is preferably at the most 60 ppb diacetyl, such as at the most 55 ppb diacetyl, such as at the most 50 ppb diacetyl, for example at the most 45 ppb diacetyl when fermentation is complete. In particular, said level of diacetyl at completion of fermentation is at the most 60 ppb.

Aforementioned fermentation is preferably performed by incubating the aqueous extract with at least 6 million viable yeast cells per millilitre, such as at least 10 million viable yeast cells per millilitre, such as at least 14 million viable yeast cells per millilitre, such as in the range of 7-8 million viable yeast cells per millilitre, for example in the range of 14-16 million viable yeast cells per millilitre. In particular, said fermentation may be performed by incubating the aqueous extract with approx. 15 million yeast cells per millilitre.

In some embodiments, said fermented aqueous extract has an alcohol content of at least 4% ABV, such as at least 5% ABV, such as at least 6% ABV, such as at least 7% ABV.

In some embodiments, said fermented aqueous extract contains at least 25 mg/L propanol, such as at the least 30 mg/L propanol.

In some embodiments, said fermented aqueous extract contains at the most 8 mg/L isobutanol, such as at the most 7 mg/L isobutanol, such as at the most 6 mg/L isobutanol.

In some embodiments, said fermented aqueous extract contains a beneficial propanol:isobutanol ratio. In particular, the propanol:isobutanol ratio may be at least 1.5, such as at least 2.0, such as at least 2.5, such as at least 3.0, such as at least 3.5, such as at least 4.0, such as at least 4.5, such as at least 5.0, such as at least 5.5, such as at least 6.0, such as at least 6.5.

In some embodiments, a fermented aqueous extract obtained with a yeast of the invention has a real degree of fermentation (RDF) that is at least 1.5%, such as 2% lower at the same % ABV than a fermentation conducted with the W-34/78 yeast strain or yeast strain Hybrid yeast 7 under the same conditions.

### Malt and/or cereal based beverage and methods of production thereof

The malt and/or cereal based fermented aqueous extract described herein above may be further processed into a beverage.

It is an aspect of the invention to provide methods of producing a malt and/or cereal based beverage, said method comprising the steps of:
i. Preparing a fermented aqueous extract as described herein above in the section "Malt and/or cereal based fermented aqueous extract and methods of production thereof", and
ii. further processing said fermented aqueous extract into a beverage.

In some embodiment of the present invention, the malt and/or cereal based beverage is diluted with a liquid, such as water.

Optionally, water can be used to dilute the malt and/or cereal based beverage and thereby adjust e.g. the ethanol content. In one embodiment of the present invention the proportions of water:malt and/or cereal based beverage may be in the range of 0.1 to 5 parts water to 1 part malt and/or cereal based beverage.

The further process may for example also include chilling and/or filtering of the malt and/or cereal based beverage. Also additives may be added. Furthermore, CO₂ may be added. Finally, the malt and/or cereal based beverage, such as a beer, may be pasteurized and/or filtered, before it is packaged (e.g. bottled or canned).

In preferred embodiments, the beverage is a beer.

In an aspect of the present invention, the malt and/or cereal based beverage, produced by fermenting the aqueous extract with said yeast strain according to the present invention has a pleasant taste.

The taste of the malt and/or cereal based beverage produced by fermentation with the yeasts according to the invention may be analyzed, for example, by a specialist beer taste panel. Preferably, said panel is trained in tasting and describing beer flavors, with special focus on aldehydes, papery taste, old taste, esters, higher alcohols, fatty acids and sulphury components.

In general, the taste panel will consist of in the range of 3 to 30 members, for example in the range of 5 to 15 members, preferably in the range of 8 to 12 members. The taste panel may evaluate the presence of various flavours, such as papery, oxidized, aged, and bready off-flavours as well as flavours of esters, higher alcohols, sulphur components and body of beer. The overall taste of the beer will generally be rated by the taste panel on several different characteristics on a scale from 1 to 9, where an average rating of over 5 signifies that the beer has an acceptable taste.

The present invention also provides malt and/or cereal based beverages, prepared by the methods described above.

In some embodiments, the beverage contains at least 25 mg/L propanol, such as at least 30 mg/L propanol.

In some embodiments, the beverage contains at the most 8 mg/L isobutanol, such as at the most 6 mg/L isobutanol.

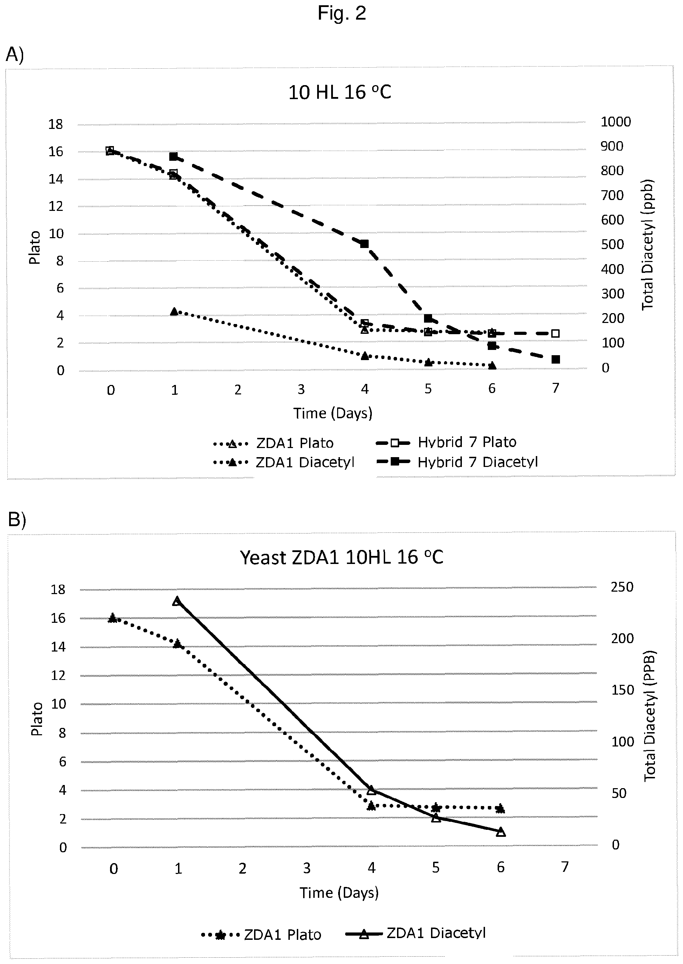

. In general, a fermented aqueous extract or a beverage prepared using the yeast strains of the invention will have a specific ratio of propanol:isobutanol, for example a ratio similar to the ratios shown in figure 2. Thus, if a fermented aqueous extract or a beverage has this specific ratio of propanol:isobutanol, it is an indication that the fermented aqueous extract or a beverage has been produced by fermentation with a yeast strain of the invention. In particular, said propanol:isobutanol ratio may be at least 1.5, such as at least 2.0, such as at least 2.5, such as at least 3.0, such as at least 3.5, such as at least 4.0, such as at least 4.5, such as at least 5.0, such as at least 5.1, such as at least 5.2, such as at least 5.3, such as at least 5.4, such as at least 5.5, such as at least 6.0, such as at least 6.5.

### Items

The invention may further be defined by the following items:
1. A method of producing a fermented aqueous extract, said method comprising the steps of:
   i) providing an aqueous extract of malt and/or cereal;
   ii) providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said fermented test solution contains at the most 60 ppb diacetyl at the earliest time point when the apparent extract of said test solution has not decreased by more than 0.50° Plato in the preceding 24 hours, wherein said fermentation occurs at a temperature of at the most 18 °C; and
   iii) fermenting the aqueous extract provided in step i) with said yeast strain of step ii), thereby obtaining a fermented aqueous extract.
2. A yeast strain encoding within its genome
   a. at the most two functional genes encoding ILV2, wherein each gene encoding ILV2 encodes SclLV2 of SEQ ID NO: 32 or SelLV2 of SEQ ID NO: 38, or a functional homologue sharing at least 80% sequence identity herewith; and
   b. at least five functional genes, such as at least six functional genes encoding ILV3, wherein each gene encoding ILV3 encodes ScILV3 of SEQ ID NO: 35 or SeILV3 of SEQ ID NO: 41, or a functional homologue sharing at least 80% sequence identity herewith.
3. A yeast strain capable of producing a fermented aqueous extract upon incubation in an aqueous extract of malt and/or cereal, wherein said fermented aqueous extract contains a propanol:isobutanol ratio of at least 2.0.
4. The yeast strain according to any one of items 2 to 3, wherein said yeast strain is capable of producing a fermented test solution upon incubation in a test solution, wherein the test solution is an extract of malt and/or cereal having an apparent extract of at least 10° Plato, wherein said fermented test solution contains at the most 60 ppb total diacetyl at the earliest time point when the apparent extract of said test solution has not decreased by more than 0.50° Plato in the preceding 24 hours, wherein said fermentation occurs at a temperature of at the most 18 °C.
5. The method or yeast strain according to item 1 or 4, wherein said time point is the earliest time point when the apparent extract of said test solution has not decreased by more than 0.40° Plato, such as 0.30° Plato, such as 0.20° Plato in the preceding 24 hours.
6. The method or yeast strain according to any one of items 1 and 4-5, wherein said test solution contains at the most 55 ppb diacetyl, such as at the most 50 ppb diacetyl, such as at the most 45 ppb diacetyl at said earliest time point.
7. A method of producing a fermented aqueous extract, said method comprising the steps of:
   i) providing an aqueous extract of malt and/or cereal;
   ii) providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said test solution contains at the most 60 ppb diacetyl, at the earliest time point when the apparent extract of said test solution has not decreased by more than 0.25° Plato in the preceding 12 hours, wherein said fermentation occurs at a temperature of at the most 18 °C; and
   iii) fermenting the aqueous extract provided in step i) with said yeast strain, thereby obtaining a fermented aqueous extract.
8. The yeast strain according to any one of items 2 to 6, wherein said yeast strain is capable of producing a fermented test solution upon incubation in a test solution, wherein the test solution is an extract of malt and/or cereal having an apparent extract of at least 10° Plato, wherein said test solution contains at the most 60 ppb total diacetyl, at the earliest time point when the apparent extract of said test solution has not decreased by more than 0.25° Plato in the preceding 12 hours, wherein said fermentation occurs at a temperature of at the most 18 °C.
9. The method or yeast strain according to any one of items 7 to 8, wherein said time point is the earliest time point when the apparent extract of said test solution has not decreased by more than 0.20° Plato, such as 0.15° Plato, such as 0.10° Plato in the preceding 12 hours.
10. The method or yeast strain according to any one of items 7 to 8, wherein said test solution contains at the most 55 ppb diacetyl, for example at the most 50 ppb diacetyl, such as at the most 45 ppb diacetyl at said earliest time point.
11. A method of producing a fermented aqueous extract, said method comprising the steps of:
   i) providing an aqueous extract of malt and/or cereal;
   ii) providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said test solution contains at the most 120 ppb diacetyl, for example at the most 60 ppb diacetyl at the earliest time point when the apparent extract of said test solution does not decrease by more than 0.50° Plato in the following 24 hours, wherein said fermentation occurs at a temperature of at the most 18 °C; and
   iii) fermenting the aqueous extract provided in step i) with said yeast strain, thereby obtaining a fermented aqueous extract.
12. The yeast strain according to any one of items 2 to 6 and 8 to 10, wherein said yeast strain is capable of producing a fermented test solution upon incubation in a test solution, wherein the test solution is an extract of malt and/or cereal having an apparent extract of at least 10° Plato, wherein said test solution contains at the most 120 ppb total diacetyl, for example at the most 60 ppb diacetyl at the earliest time point when the apparent extract of said test solution does not decrease by more than 0.50° Plato in the following 24 hours, wherein said fermentation occurs at a temperature of at the most 18 °C.
13. The method or yeast strain according to any one of items 11 to 12, wherein said time point is the earliest time point when the apparent extract of said test solution does not decreased by more than 0.40° Plato, such as 0.30° Plato, such as 0.20° Plato in the following 24 hours.
14. A method of producing a fermented aqueous extract, said method comprising the steps of:
   i) providing an aqueous extract of malt and/or cereal;
   ii) providing a yeast strain of the species *Saccharomyces pastorianus,* wherein said yeast strain is capable of producing a fermented test solution upon incubation in a test solution, wherein the test solution is an extract of malt and/or cereal having an apparent extract of at least 10° Plato, wherein said test solution contains at the most 120 ppb diacetyl, for example at the most 65 ppb diacetyl at the earliest time point when the apparent extract of said test solution does not decrease by more than 0.25° Plato in the following 12 hours, wherein said fermentation occurs at a temperature of at the most 18 °C; and
   iii) fermenting the aqueous extract provided in step i) with said yeast strain, thereby obtaining a fermented aqueous extract.
15. The yeast strain according to any one of items 2 to 6, 8 to 10 and 12 to 13, wherein said yeast strain is capable of producing a fermented test solution upon incubation in a test solution, wherein the test solution is an extract of malt and/or cereal having an apparent extract of at least 10° Plato, wherein said test solution contains at the most 120 ppb diacetyl, for example at the most 65 ppb total diacetyl at the earliest time point when the apparent extract of said test solution does not decrease by more than 0.25° Plato in the following 12 hours, wherein said fermentation occurs at a temperature of at the most 18 °C.
16. The method or yeast strain according to any one of items 14 to 15, wherein said time point is the earliest time point when the apparent extract of said test solution does not decrease by more than 0.20° Plato, such as 0.15° Plato, such as 0.10° Plato in the following 12 hours.
17. The method or yeast strain according to any one of items 11 to 15, wherein said test solution contains at the most 115 ppb diacetyl at said earliest time point.
18. The method or yeast strain according to any one of items 1 and 4 to 17,
   wherein said test solution contains at the most 10 million cells per millilitre, such as at the most 9.5 million cells per millilitre, such as at the most 9.0 million cells per millilitre, such as at the most 8.5 million cells per millilitre in solution at said earliest time point.
19. The method or yeast strain according to any one of the preceding items, wherein the test solution of step ii) or the test solution is wort having an apparent extract of approx. 16° Plato.
20. The method or yeast strain according to any one of the preceding items, wherein the test solution of step ii) or the test solution comprises at least 40 g/kg maltose.
21. The method or yeast strain according to any one of the preceding items, wherein said fermentation in step ii) or said fermentation occurs at a temperature in the range of 12 °C to 18 °C.
22. The method or yeast strain according to any one of the preceding items, wherein said fermentation in step ii) or said fermentation occurs at a temperature of at the most 16 °C.
23. The method or yeast strain according to any one of the preceding items, wherein said fermentation in step ii) or said fermentation occurs after inoculation of in the range of 7,000,000 to 20,000,000 viable yeast cells per mL test solution.
24. The method or yeast strain according to any one of the preceding items, wherein said aqueous extract is fermented with said yeast strain for at the most 6 days, such as at the most 5 days, such as at the most 4 days.
25. The method or yeast strain according to any one of the preceding items, wherein said aqueous extract has an apparent extract of at least 12° Plato, such as at least 15° Plato.
26. The method or yeast strain according to any one of the preceding items, wherein said aqueous extract is wort.
27. The method or yeast strain according to any one of the preceding items, wherein said aqueous extract comprises at the most 3500 mg/L, such as at the most 3000 mg/L, such as at the most 2500 mg/L amino acids.
28. The method or yeast strain according to any one of the preceding, items wherein said fermented aqueous extract contains at the most 60 ppb diacetyl, such as at the most 55 ppb diacetyl, such as at the most 50 ppb diacetyl, such as at the most 45 ppb diacetyl, such as at the most 40 ppb diacetyl.
29. The method or yeast strain according to any one of the preceding items, wherein said fermented aqueous extract contains at the most 10 million cells per millilitre, such as at the most 9.5 million cells per millilitre, such as at the most 9.0 million cells per millilitre, such as at the most 8.5 million cells per millilitre in solution at said earliest time point.
30. The method or yeast strain according to any one of the preceding items, wherein said fermented aqueous extract has an alcohol content of at least 4% ABV, such as at least 5% ABV.
31. The method or yeast strain according to any one of the preceding items, wherein said fermented aqueous extract contains a least 25 mg/L propanol, such as at the least 30 mg/L propanol.
32. The method or yeast strain according to any one of the preceding items, wherein said fermented aqueous extract contains at the most 8 mg/L isobutanol, such as at the most 6 mg/L isobutanol.
33. The method or yeast strain according to any one of the preceding items, wherein said fermented aqueous extract contains a propanol:isobutanol ratio of at least 2.0, such as at least 2.5, such as at least 3.0, such as at least 4.0, such as at least 5.0, such as at least 5.5.
34. The method or yeast strain according to any one of the preceding items, wherein said yeast strain is capable of producing a fermented test solution, wherein said test solution contains at the most 265 ppb diacetyl at any time point within 5 days after initiation of incubation, and wherein in the range of 7 to 8 million yeast cells are added to said test solution.
35. The method or yeast strain according to any one of the preceding items, wherein said yeast strain is capable of producing a fermented test solution containing at the most 50 ppb diacetyl after incubation in said test solution for at the most 6 days, such as at the most 5 days, such as the most 4 days.
36. The method or yeast strain according to any one of the preceding items, wherein said yeast strain is capable of producing a fermented test solution containing at the most 50 ppb diacetyl after incubation in said test solution at a temperature of at the most 16 °C for at the most 5 days, such as at the most 4 days.
37. The method or yeast strain according to any one of the preceding items, wherein said yeast strain is capable of producing a first fermented test solution containing at the most 60 ppb diacetyl after incubation in said test solution for a given time, wherein said given time is at least 12 hours, such as at least 24 hours less than the time required for the W-34/78 yeast strain incubated under the same conditions to produce a second fermented test solution containing at the most 60 ppb diacetyl after incubation in said test solution.
38. The method or yeast strain according to any one of the preceding items, wherein said yeast strain is capable of generating at least 4.0, such as at least 4.7 mL/L ethanol per °Plato, when said yeast strain is incubated in said test solution.
39. The method or yeast strain according to any one of the preceding items, wherein said yeast strain is capable of generating at least 4.0, such as at least 4.7 mL/L ethanol per °Plato, when said yeast strain is incubated in said test solution for in the range of 4 to 6 days, such as for approximately 4 days.
40. The method or yeast strain according to any one of the preceding items, wherein said yeast strain is capable of growing on media with melibiose as the sole carbon source.
41. The method or yeast strain according to any one of the preceding items, wherein said yeast strain has at the most five allelic genes encoding ILV2, wherein each gene encoding ILV2 encodes ScILV2 of SEQ ID NO: 32 or SelLV2 of SEQ ID NO: 38, or a homologue sharing at least 80% sequence identity herewith.
42. The method or yeast strain according to any one of the preceding items, wherein said yeast strain has at the most four genes encoding ILV2, wherein each gene encoding ILV2 encodes SclLV2 of SEQ ID NO: 32 or SelLV2 of SEQ ID NO: 38, or a homologue sharing at least 80% sequence identity herewith.
43. The method or yeast strain according to any one of the preceding items, wherein said yeast strain has at the most two genes encoding ILV2, wherein each gene encoding ILV2 encodes SclLV2 of SEQ ID NO: 32 or SelLV2 of SEQ ID NO: 38, or a functional homologue sharing at least 80% sequence identity herewith.
44. The method or yeast strain according to any one of the preceding items, wherein said yeast strain has in the range of 1 to 2, such as exactly 2 functional genes encoding ILV2, wherein each gene encoding ILV2 encodes ScILV2 of SEQ ID NO: 32 or SelLV2 of SEQ ID NO: 38, or a functional homologue sharing at least 80% sequence identity herewith.
45. The method or yeast strain according to any one of the preceding items, wherein said yeast strain has in the range of 1 to 2, such as exactly 2 functional genes encoding ILV2, wherein each gene encoding ILV2 encodes ScILV2 of SEQ ID NO:32 or a functional homologue sharing at least 80% sequence identity herewith.
46. The method or yeast strain according to any one of the preceding items, wherein said yeast strain has at the most four genes encoding ILV6, wherein each gene encoding ILV6 encodes *Sc*ILV6 of SEQ ID NO: 33 or *Se*ILV6 of SEQ ID NO: 39, or a homologue sharing at least 80% sequence identity herewith.
47. The method or yeast strain according to any one of the preceding items, wherein said yeast strain has at the most four functional genes encoding ILV6, wherein each gene encoding ILV6 encodes *Sc*ILV6 of SEQ ID NO: 33 or *Se*ILV6 of SEQ ID NO: 39, or a homologue sharing at least 80% sequence identity herewith.
48. The method or yeast strain according to any one of the preceding items, wherein said yeast strain has at least four genes encoding ILV5, such as at least five genes encoding ILV5, wherein each gene encoding ILV5 encodes ScILV5 of SEQ ID NO: 34 or SelLV5 of SEQ ID NO: 40, or a homologue sharing at least 80% sequence identity herewith.
49. The method or yeast strain according to any one of the preceding items, wherein said yeast strain encodes within its genome at least four functional genes encoding ILV5, such as at least five functional genes encoding ILV5, wherein each gene encoding ILV5 encodes ScILV5 of SEQ ID NO: 34 or SeILV5 of SEQ ID NO: 40, or a functional homologue sharing at least 80% sequence identity herewith.
50. The method or yeast strain according to any one of the preceding items, wherein said yeast strain has at the most functional 15 genes encoding ILV5, such as between 4 and 10 functional genes encoding ILV5, such as between 4 and 5 functional genes encoding ILV5.
51. The method or yeast strain according to any one of the preceding items, wherein said yeast strain has in the range of and in the range of 4 to 8 functional genes encoding ILV5, such as in the range of 4 to 6, such as 5 functional genes encoding ILV5, wherein each gene encoding ILV5 encodes ScILV5 of SEQ ID NO: 34 or SelLV5 of SEQ ID NO: 40 or a functional homologue of any of the aforementioned sharing at least 80% sequence identity therewith.
52. The method or yeast strain according to any one of the preceding items, wherein said yeast strain has at least three genes, such as at least four genes encoding ILV3, wherein each gene encoding ILV3 encodes ScILV3 of SEQ ID NO: 35 or SeILV3 of SEQ ID NO: 41, or a homologue sharing at least 80% sequence identity herewith.
53. The method or yeast strain according to any one of the preceding items, wherein said yeast strain has at the most 15 functional genes encoding ILV3, such as between 5 and 10 functional genes encoding ILV3, such as between 5 and 6 functional gens encoding ILV3.
54. The method or yeast strain according to any one of the preceding items, wherein said yeast strain has in the range of 5 to 9 functional genes encoding ILV3, such as at in the range of 5 to 7, such as 6 functional genes encoding ILV3, wherein each gene encoding ILV3 encodes ScILV3 of SEQ ID NO: 35 or SeILV3 of SEQ ID NO: 41 or a functional homologue of any of the aforementioned sharing at least 80% sequence identity therewith.
55. The method or yeast strain according to any one of the preceding items, wherein said yeast strain has at least three genes, such as at least four genes encoding BAT1, wherein each gene encoding BAT1 encodes ScBAT1 of SEQ ID NO: 36 or SeBAT1 of SEQ ID NO: 42, or a homologue sharing at least 80% sequence identity herewith.
56. The method or yeast strain according to any one of the preceding items, wherein said yeast strain has at least three functional genes, such as at least four functional genes encoding BAT1, wherein each gene encoding BAT1 encodes ScBAT1 of SEQ ID NO: 36 or SeBAT1 of SEQ ID NO: 42, or a homologue sharing at least 80% sequence identity herewith.
57. The method or yeast strain according to any one of the preceding items, wherein said yeast strain has at least four genes, such as at least five genes encoding BAT2, wherein each gene encoding BAT2 encodes ScBAT2 of SEQ ID NO: 37 or SeBAT2 of SEQ ID NO: 43, or a homologue sharing at least 80% sequence identity herewith.
58. The method or yeast strain according to any one of the preceding items, wherein said yeast strain has at least four functional genes, such as at least five functional genes encoding BAT2, wherein each gene encoding BAT2 encodes ScBAT2 of SEQ ID NO: 37 or SeBAT2 of SEQ ID NO: 43, or a homologue sharing at least 80% sequence identity herewith.
59. The method or yeast strain according to any one of the preceding items, wherein the sum of the genes in said yeast strain encoding ILV2 and ILV6 is lower than the sum of the genes encoding ILV5 and ILV3.
60. The method or yeast strain according to any one of the preceding items, wherein the sum of the genes in said yeast strain encoding ILV2 is lower than the sum of the genes encoding ILV5 and ILV3.
61. The method or yeast strain according to any one of the preceding items, wherein the gene ratio in said yeast strain of *ILV5* and *ILV3* vs. *ILV2* is at least 1, such as at least 1.5, such as at least 2, such as at least 2.5 or such as at least 3.
62. The method or yeast strain according to any one of the preceding items, wherein the functional gene ratio in said yeast strain of *ILV5* and *ILV3* vs. *ILV2* is at least 1, such as at least 1.5, such as at least 2, such as at least 2.5, such as at least 3.
63. The method or yeast strain according to any one of the preceding items, wherein the gene ratio in said yeast strain of *ILV5* and *ILV3* vs. *ILV2* and *ILV6* is at least 1, such as at least 1.2, such as at least 1.4, such as at least 1.6, such as at least 1.8 or such as at least 2.
64. The method or yeast strain according to any one of the preceding items, wherein the functional gene ratio in said yeast strain of *ILV5* and *ILV3* vs. *ILV2* and *ILV6* is at least 1, such as at least 1.2, such as at least 1.4, such as at least 1.6, such as at least 1.8 or such as at least 2.
65. The method or yeast strain according to any one of the preceding items, wherein the genes encoding ILV2, ILV3 and/or ILV5 are expressed from their native promoters.
66. The method or yeast strain according to any one of the preceding items, wherein the genes encoding ILV2, ILV3, ILV5, ILV6, BAT1 and/or BAT2 are expressed from their native promoters.
67. The method or yeast strain according to any one of the preceding items, wherein said yeast strain carries a mutation in or a deletion of one or more *ILV2* genes.
68. The method or yeast strain according to any one of the preceding items, wherein said yeast strain carries a frameshift mutation in one or more *ILV2* genes.
69. The method or yeast strain according to any one of the preceding items, wherein said yeast strain carries a mutation resulting in lower or no expression of one or more *ILV2* genes.
70. The method or yeast strain according to any one of the preceding items, wherein said yeast strain carries a mutation in or a deletion of one or more *ILV6* genes.
71. The method or yeast strain according to any one of the preceding items, wherein said yeast strain carries a frameshift mutation in one or more *ILV6* genes.
72. The method or yeast strain according to any one of the preceding items, wherein said yeast strain carries a mutation resulting in lower or no expression of one or more *ILV6* genes.
73. The method or yeast strain according to any one of the preceding items, wherein said yeast strain does not comprise any heterologous DNA.
74. The method or yeast strain according to any one of the preceding items, wherein said yeast strain has not undergone a step of genetic engineering.
75. A fermented aqueous extract prepared by the method according to any one of the preceding items.
76. A method for producing a beverage, said method comprising the steps of:
   i. preparing a fermented aqueous extract according to any one of the preceding items, and
   ii. processing said fermented aqueous extract into a beverage.
77. The method according to item 76, wherein the steps of processing comprise one or more of the following:
   i. Filtration,
   ii. Carbonation,
   iii. Maturation, or
   iv. Bottling
78. A beverage prepared by the method according to any one of items 76 to 77.
79. The beverage according to item 78, wherein said beverage contains at least 25 mg/L propanol, such as at least 30 mg/L propanol.
80. The beverage according to any one of items 78 to 79, wherein said beverage contains at the most 8 mg/L isobutanol, such as at the most 6 mg/L isobutanol.
81. The beverage according to any one of items 78 to 80, wherein said beverage is a beer.
82. The fermented aqueous extract according to item 75 or the beverage according to any one of items 78-81, wherein said extract or said beverage has a propanol:isobutanol ratio of at least 3.0, such as at least 4.0, such as at least 5.0, such as at least 5.5.
83. A yeast strain, wherein said yeast strain is capable of producing a fermented test solution after incubation of said yeast strain in a test solution having an apparent extract of at least 10° Plato and wherein said test solution contains at the most 60 ppb diacetyl, for example at the most 50 ppb diacetyl at the earliest time point where the apparent extract of said test solution has not decreased by more than 0.50° Plato in the preceding 24 hours after incubation of said yeast strain in said extract.
84. A *Saccharomyces pastorianus* yeast strain according to item 83, wherein the yeast strain is as defined in any one of items 1 to 72.

### Examples

### Materials and methods

### Wort preparation

Generally, the wort used in the experimental examples below was made by mashing 70% pilsner malt and 30% barley adjunct in 2.7 L of water/kg malt.

The mashing regime was as shown in table 1 below:

**Table 1. Mashing regime for wort preparation.**

| | Temp (°C) at start | Temp (°C) at end | Time (min) | Total time (min) |
|---|---|---|---|---|
| Mashing in | 52.0 | 52.0 | 5 | 5 |
| *Hold* | 52.0 | | 15 | 20 |
| Temp. increase | 52.0 | 65.0 | 13 | 33 |
| *Hold* | 65.0 | | 50 | 83 |
| Temp. increase | 65.0 | 72.0 | 7 | 90 |
| *Hold* | 72.0 | | 20 | 100 |
| Temp. increase | 72.0 | 78.0 | 6 | 106 |
| *Hold* | 78.0 | | 10 | 116 |

Variations in this mashing regime can still produce a wort suitable for fermentation.

Following mashing, the wort was boiled for 60 min. To ensure that the wort is suitable for fermentation it should preferably have contents within the following ranges:

| | |
|---|---|
| • Glucose | 12-25 g/L |
| • Maltose | 60-80 g/L |
| • Maltotriose | 15-20 g/L |
| • Zinc | 0.16 to 0.18 mg/L |
| • Free alpha amino nitrogen (FAN) | 110-250 mg/L |
| • Valine/FAN | 0.6 to 0.8 |

As a guide, an average content of a wort made from 70% pilsner malt and 30% barley adjunct is given in table 2 below. Worts which are outside some of the parameters below are still expected to produce good beer.

**Table 2. Example composition of a wort made from 70% pilsner malt and 30% barley adjunct.**

| **Sugars** | **g/100 ml wort** | **STD** | **Minerals** | **mg/L** | **STD** |
|---|---|---|---|---|---|
| Fructose | 0.29 | 0.06 | Ca Calcium | 46.15 | 9.28 |
| Glucose | 1.93 | 0.70 | Mg Magnesium | 115.73 | 15.31 |
| Sucrose | 0.35 | 0.09 | Na Sodium | 21.03 | 10.63 |
| Maltose | 7.84 | 0.52 | K Potassium | 815.82 | 170.68 |
| Maltotriose | 1.69 | 0.55 | Zn Zinc | 0.17 | 0.10 |
| Sum Fermentable Sugars | 12.10 | 0.95 | Cu Copper | 0.07 | 0.02 |
| Sugar (Mono Disaccharides) | 10.59 | 0.45 | Fe Iron | 0.12 | 0.08 |
| Inverted sugars BUR Law FR | 2.30 | 0.40 | Al Aluminium | 0.01 | 0.01 |
| | | | Mn Manganese | 0.11 | 0.08 |
| | | | P Phosphorus | 511.96 | 61.85 |
| | | | SI Silica | 18.43 | 9.03 |

| **Amino acids** | **mg/L** | **STD** | | | |
|---|---|---|---|---|---|
| Aspartic acid (Asp) | 89.53 | 16.66 | **FAN*** mg/L | 207.16 | 52.16 |
| Glutamic acid (Glu) | 91.43 | 15.62 | | | |
| Serine (Ser) | 92.10 | 28.01 | **VAL/FAN** | 0.61 | 0.03 |
| Histidine (His) | 59.09 | 15.86 | | | |
| Glycine (Gly) | 44.98 | 12.42 | | | |
| Threonine (Thr) | 81.00 | 25.22 | | | |
| Arginine (Arg) | 161.68 | 59.35 | | | |
| Alanine (Ala) | 120.60 | 27.66 | | | |
| Tyrosine (Tyr) | 133.16 | 39.81 | | | |
| Methionine (Met) | 47.92 | 17.61 | | | |
| Valine (Val) | 141.48 | 34.78 | | | |
| Phenylalanine (Phe) | 147.45 | 30.83 | | | |
| Isoleucine (Iso) | 81.80 | 21.26 | | | |
| Leucine (Leu) | 181.02 | 31.62 | | | |
| Lysine (Lys) | 115.49 | 36.49 | | | |
| Sum Amino acids Class 1 | 410.67 | 90.89 | | | |
| Sum Amino acids Class 2 | 681.55 | 148.43 | | | |
| Sum Amino acids Class 3 | 532.18 | 113.77 | | | |

| | | | | | |
|---|---|---|---|---|---|
| * FAN= Free alpha amino nitrogen | | | | | |

To aid flocculation of the yeast, additional zinc may be added to the wort prior to fermentation as described in some of the examples below.

### Droplet PCR

Genomic DNA was prepared from diploid yeast strains and haploid spore clones using the MasterPure Genomic DNA purification kit from Epicentre. Concentrations of each final DNA preparation was adjusted to 50 ng/microliter DNA using Milli Q water. DNA was quantified using a NanoDrop 2000 Spectrophotometers (Thermo Scientific).

Using the Biorad droplet PCR (QX200) and probes designed to anneal to the *Saccharomyces eubayanus* and *Saccharomyces cerevisiae ILV2* genes. These probes were used to target genomic DNA in haploid spore clones and control diploid yeast strains. Using this method the inventors were able to determine the ratio between the two variant *ILV2* genes in a quantitative manner. In order to distinguish *S. eubayanus* and *S*. *cerevisiaie ILV2* alleles a species-specific digital PCR reaction based on a TaqMan assay was designed. For this purpose the target specific primers were designed in homologous regions of the *ILV2* gene for *S. eubayanus* and *S. cerevisiae,* meaning that the same forward and reverse primer could amplify both *ILV2* species copies simultaneously. The species-specific probes were designed to be identical apart from a single nucleotide, allowing a discrimination between the *ILV2* alleles of *S. eubayanus* and *S. cerevisiae* type The species specific probe for *S. cerevisiae* has a T at nucleotide position 1515 in the *S. cerevisiae ILV2* wild type sequence, whereas the species-specific probe for S. eubayanus has a C at nucleotide position 1515 in the S. eubayanus *ILV2* wild type sequence. The following primers and probes were developed to distinguish between the S. eubayanus *ILV2* allele and S. cerevisiae *ILV2* allele:
Target specific *ILV2* primers that will amplify both *S. cerevisiae* and *S. eubayanus ILV2* simultaneously:
Target specific forward primer (5'- GCCAACGACACAGGAAGAC - 3') (SEQ ID NO: 1)
Target specific reverse primer (5'- GTACCTAAACCACCTGATGT - 3') (SEQ ID NO: 2)

Species specific probes that will allow discrimination between *S. cerevisiae* and *S. eubayanus ILV2* copies:
*S*. *cerevisiae ILV2* allele specific probe (5'- TGGGCTGCTCAACAC - 3') (SEQ ID: 3) - labelled with 5' FAM and a 3' BHQ1
*S. eubayanus ILV2* allele specific probe (5'- ACGTACGGAATGTGGATT - 3') (SEQ ID NO: 4) - labelled with 5' HEX and a 3' BHQ1

Using a Droplet Digital PCR QX200 system (Bio-Rad Laboratories), ddPCR was performed according to the instructions provided by the manufacturer. For analytical purposes, 5µl of purified gDNA (5ng/µl DNA concentration) of yeast strains was added to a 17-µl PCR mixture containing 11 µl 2x ddPCR Supermix for probes (No. dUTP; Bio-Rad), 900 nM target-specific PCR forward primer, 900 nM target-specific PCR reverse primer, 250 nM *S. cerevisiae ILV2* allele specific probe and 250 nM *S. eubayanus ILV2* allele specific probe. The reaction mixture was loaded onto the AutoDG Droplet Generator (Bio-Rad Laboratories) and droplet generation carried out according to the manufacturer's manual. The droplet emulsion was thermally cycled using standard PCR conditions: denaturation at 95 °C for 10 min, 40 cycles of PCR at 94 °C for 30 sec and 55 °C for 1 min, and a final extension at 98 °C for 10 min before storage of the microtiter plate at 8 °C. PCR amplification in droplets was confirmed using the QX200 Droplet Reader (Bio-Rad Laboratories) and the data was analysed using the software QuantaSoft (version v1.7, Bio-Rad Laboratories).

### Detection of total ILV2 gene copy number

In the first round, the ratios between *S. eubayanus* and *S*. *cerevisiae ILV2* were analysed directly on purified genomic DNA from each single spore clone. Some spore clones despite originating from the same parental yeast had different *ILV2* genetic make-up. Some spore clones appear to have only 100% S. cerevisiae *ILV2* genes the spore clone. Since most parental lager yeasts are tri- or tetraploid (Wahlter et al, 2014) it can be assumed that the 100 % *S. cerevisiae ILV2* spore clones may have somewhere between 1-3 copies. For the spore clones with both types of *ILV2* such as 50/50 one would assume 1 copy of each type in spore clones or 2 + 2 in parental yeasts. Strains with ratios close to 67/33 would mean 1 copy of *S. cerevisiae* and 2 copies of *S*. *eubayanus ILV2.*

To allow a more accurate estimation of copy numbers in spore clones with close to 100 % *S. cerevisiae ILV2* the trial was repeated by adding or spiking in pure external *S. eubayanus* DNA to the samples to get a the S. cerevisiae/S. eubayanus distribution after spike in. Ratios *ScILV2*/*SeILV2* that would shift strongly towards S. eubayanus after spike in would indicate low copies of *S. cerevisiae ILV2* and if the ratio would shift only little towards S. eubayanus DNA and still be dominated by high S. cerevisaie *ILV2* would indicate that the original spore clone has many *S*. *cerevisiae ILV2* copies.

Spiking was done such that each sample of 50 ng/microliter DNA was blended with S. eubayanus DNA of the same concentration 50 ng/microliter in a 50/50 ratio (10 microliter sample + 10 mictroliter S. eubayanus DNA).

Droplet QPCR estimation method quantitatively reflects total *ILV2* copies present but cannot reveal if there are some inactivated *ILV2* alleles due to the presence of particular SNPs or frame shift mutations. Such mutations can first be detected by a subsequent genomic DNA sequencing by Sanger sequencing or Next generation sequencing.

### Screening for flocculation

To asses flocculation abilities of yeast spore clones, they were first grown in regular wort at room temperature for several days. For each strain 2 ml of culture was transferred to a 2 ml reaction tube, vortexed thoroughly, and cells were left to settle for 10 min. To determine calcium dependent flocculation, cells from 2 ml of the previously mentioned cultures were harvested, washed twice with 50 mM EDTA solution and subsequently resuspended in 2 ml 50 mM Tris, 50 mM succinic acid and 100 mM KOH without calcium (Stratford 1996). The pH was adjusted to pH 4.0, which resembles the final pH values of the wort cultures. Cell suspensions were again thoroughly vortexed, and cells were left to settle for 5 min. Results were documented photographically.

### Measurement of total diacetyl of spore clones in wort fermentations using cylinders with magnetic stirring

Spore clones and control yeasts were pregrown for three days in 2 ml liquid YPD on a Stuart SB2 rotary unit (www.stuart-equipment.com). Non-baffled shake flasks of 25 ml pasteurized standard Pilsner wort of 16 Plato were inoculated with the two ml precultures and grown at room temperature on a shaking table with intermediate shaking for 5 days to reach dense stationary phase cultures.

Beer fermentations were conducted in glass cylinders as described previously (Guiterrez et al., 2018). Fermentations were performed in 250 mL tall glass measuring cylinders (331 × 39 × 39 mm; Duran) containing 200 mL of Pilsner wort and sealed with an inverted glass beaker (Duran) on the top of the cylinder to enable escape of carbon dioxide and easy sampling access for analyses. Fermentations were performed with continuous stirring at 150 rpm using a magnetic bar on a stirring Variomag stirring platform at 16 °C inside a refrigerator (incubator cabinet of type TS 606-G/4-i). Yeast cell counts were estimated with a cellometer (https://www.nexcelom.com/applications/cellometer) and inoculated (pitched) at a rate of 15 million cells per ml wort and fermentation performance was monitored by measuring the weight loss resulting from metabolic CO₂ release. 10 ml samples were collected during the fermentation at day 2, 3 and 4 for diacetyl analyses. Samples were centrifuged at 1900 g for 10 min and supernatants were stored in a freezer at -20 °C until further use. Fermentations were considered completed when no further weight loss of the fermentation cylinders could be measured. We measured total diacetyl by gas chromatography according to the European Brewing Convention method EBC 9.24.2 that includes an incubation period of the samples at 60 °C for 90 minutes to determine the total diacetyl, that will reflect the total sum of precursor acetolactate and free diacetyl.

### Yeast strains

**Hybrid yeast 7** *Saccharomyces pastorianus,* is a lager production strain and has a high flocculation allowing collection of the yeast at the end of fermentation without additional cooling. Hybrid yeast 7 is used as reference to the yeasts of the current invention.
**ZDA1** is a yeast of the invention.
**ZDA2** is a yeast of the invention
**ZDA3** is a yeast of the invention

**W-34/78** *Saccharomyces pastorianus,* is a commercially available lager yeast strain from Weihenstephan Hefebank (info@hefebank-weihenstephan.de), Germany. This yeast is known to reduce diacetyl quite efficiently and it shows high flocculation, but still significantly less than the Hybrid yeast 7 strain when fermented in the wort described above. It is used as reference to the yeast of the current invention. W-34/78 is also referred to herein as WS34/78.

### Example 1 - 50 L trial at 16 and 18 °C with yeast strain ZDA1 and comparative strain Hybrid yeast 7

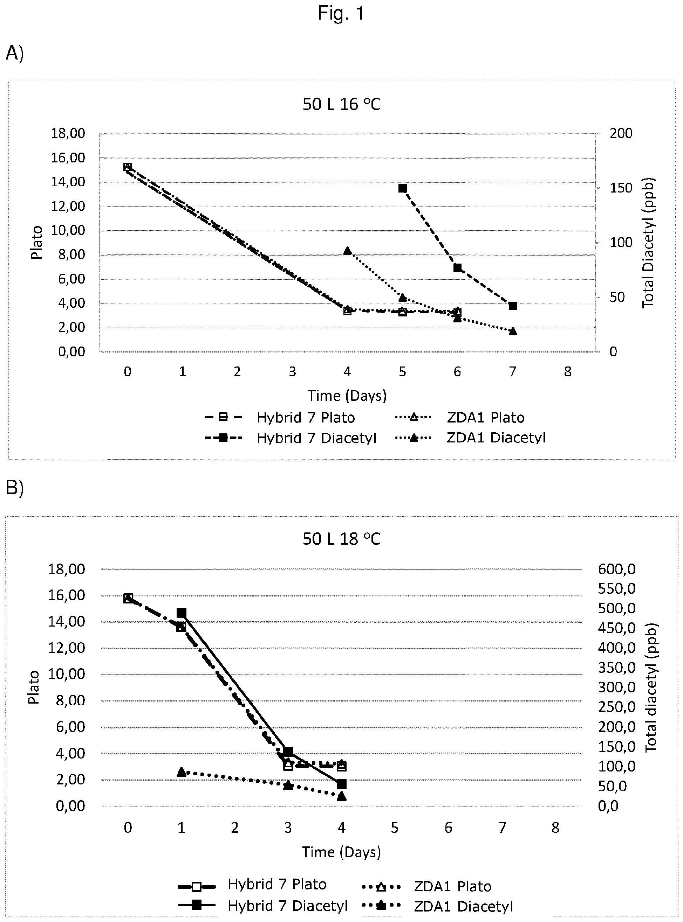

To test the difference in diacetyl levels during fermentation of a new hybrid yeast strain against a control yeast strain, 50 L beer was prepared from a wort as described in the materials and methods section with a °Plato of between 15 and 16. Prior to fermentation, the wort was adjusted to a pH of 4.9 and zinc was added to a final concentration 0.30 mg/L. The wort was inoculated with 12-15 million viable yeast cells per mL of wort. Fermentation was performed at 16 °C or 18 °C. Generally, lagers are fermented between 12 °C and 16 °C. If the yeast tolerates 18 °C this can serve to speed up the fermentation process. Samples were obtained at day 0 and at various time points during fermentation as indicated in the tables below. The results are shown in Table 3 and 4 below and plotted in Figure 1 A to C.

**Table 3. 50 L fermentation at 18 °C.**

| **Yeast** | **Day** | **Temp** °C | **°Plato** | **Diacetyl** ppb | **pH** | **Alcohol** Vol% |
|---|---|---|---|---|---|---|
| ZDA1 | 0 | | 15.79 | | 4.90 | |
| | 1 | 18.3 | 13.61 | 87 | 4.33 | |
| | 3 | | 3.33 | 54 | | |
| | 4 | 18.0 | 3.23 | 26 | 4.16 | 6.95 |
| Hybrid yeast 7 | 0 | | 15.78 | | 4.90 | |
| | 1 | 17.9 | 13.6 | 489 | 4.45 | |
| | 3 | | 3.06 | 137 | | |
| | 4 | 17.7 | 3.01 | 56 | 4.05 | 7.02 |

**Table 4. 50 L fermentation at 16 °C.**

| **Yeast** | **Day** | **Temp** °C | **°Plato** | **Diacetyl** ppb | **pH** | **Alcohol** Vol% |
|---|---|---|---|---|---|---|
| ZDA1 | 0 | | 15.27 | | 4.89 | |
| | 4 | 15.8 | 3.52 | 93 | 4.11 | |
| | 5 | 15.7 | 3.38 | 50 | 4.15 | |
| | 6 | 15.7 | 3.38 | 31 | 4.15 | |
| | 7 | 15.7 | | 19 | 4.15 | 6.55 |
| Hybrid yeast 7 | 0 | | 15.27 | | 4.89 | |
| | 4 | 16.2 | 3.39 | | 4.04 | |
| | 5 | 16.3 | 3.28 | | 4.09 | |
| | 6 | 16.3 | 3.26 | 77 | 4.09 | |
| | 7 | 16.3 | | 42 | 4.09 | 6.59 |

A beer is said to be in spec. when the diacetyl level is below 50 ppb, and the Plato is stable (all fermentable sugar has been utilized). It is an advantage to get the beer in spec. at the shortest time possible, while producing at least a similar amount of alcohol as compared to the control yeast.

### Diacetyl levels at 18 °C

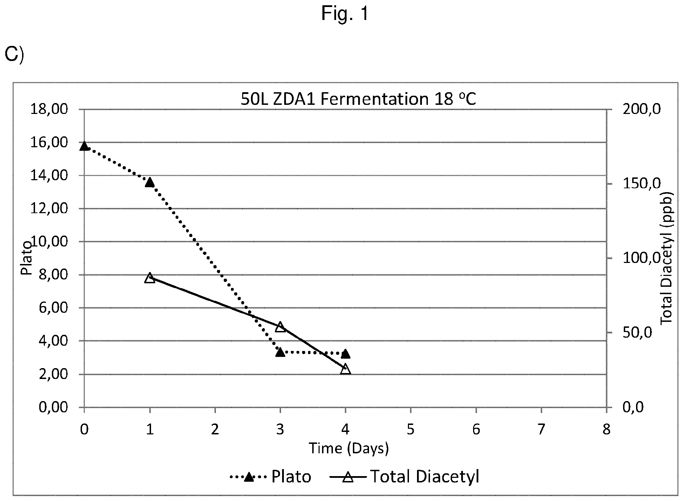

As can be seen from table 3 and Figures 1B and 1C, yeast strain ZDA1 was able to produce a fermented wort (green beer) within three days where the diacetyl level was close to 50 ppb and at the same time all fermentable sugar present in the wort was utilized (which is evident by the drop in °Plato being less than 0.50 over 24h) when fermented at 18 °C. In contrast, the control yeast strain Hybrid yeast 7 started with a very high diacetyl level (Figure 1B) and needed 4 days to reach a similar level of diacetyl as strain ZDA1 had on day 3.

The earliest point in time where the apparent extract did not decrease by more than 0.50° Plato in the preceding 24 hours was considered to be day 4 for both strains. At day 4 the green beer obtained with Hybrid yeast 7 contained approx. 56 ppb diacetyl, whereas the green beer obtained with ZDA1 contained only 26 ppb.

When the fermentation was performed at 16 °C (table 4 and figure 1A) the earliest point in time where the apparent extract did not decrease by more than 0.50° Plato in the preceding 24 hours was on day 5 for both strains. At that time the ZDA1 strain had reached the desired 50 ppm diacetyl level, whereas the control yeast Hybrid yeast 7 had to ferment an additional two days to reach a diacetyl level below 50 ppm.

### Propanol, isobutanol and SO₂ levels after fermentation at 16 °C or 18 °C 16 °C18 °C

In addition to the diacetyl and Plato, the propanol and isobutanol levels were measured in final beer made from the 50 L trial beer. The final beer was adjusted to an alcohol content of 5.0% by volume, CO₂ to 5.1 g/L and bottled in 33 cl green bottles. Without being bound by theory, propanol and isobutanol isobutanolis believed to be indicators of a restricted pathway flux within the branched chain amino acid pathway, in particular low *ILV2* activity of the yeast, and is therefore a good way to assess whether a yeast strain has the potential to be a low diacetyl producer. A high propanol:isobutanol ratio is an indication of a low diacetyl producing yeast. The results are shown in table 5 below:

**Table 5. Propanol and isobutanol content in the fermented wort (green beer)**

| **Yeast** | **Temp** °C | **Propanol** | **Isobutanol** | **Propanol: isobutanolratio** | **SO₂** |
|---|---|---|---|---|---|
| ZDA1 | 16 | 35.09 | 4.93 | 7.73 | 7 |
| | 18 | 42.18 | 6.39 | 6.60 | 6 |
| Hybrid yeast 7 | 16 | 15.53 | 17.50 | 0.89 | 4 |
| | 18 | 19.3 | 19.57 | 0.97 | 2 |

As can be seen from table 5 both at 16 °C and at 18 °C the green beer obtained from fermentation with hybrid yeast stain ZDA1 showed higher levels of propanol compared to the green beer obtained with the control yeast strain Hybrid yeast 7. The wort incubated with hybrid yeast stain ZDA1 also had lower levels of isobutanol compared to Hybrid yeast 7, resulting in an at least 6.8 fold difference in the propanol:isobutanol ratio.

It was also observed that the SO₂ levels in the green beer obtained with yeast strain ZDA1 was higher than in the green beer from the control strain. This can provide an advantage in terms of preservation of the final beer.

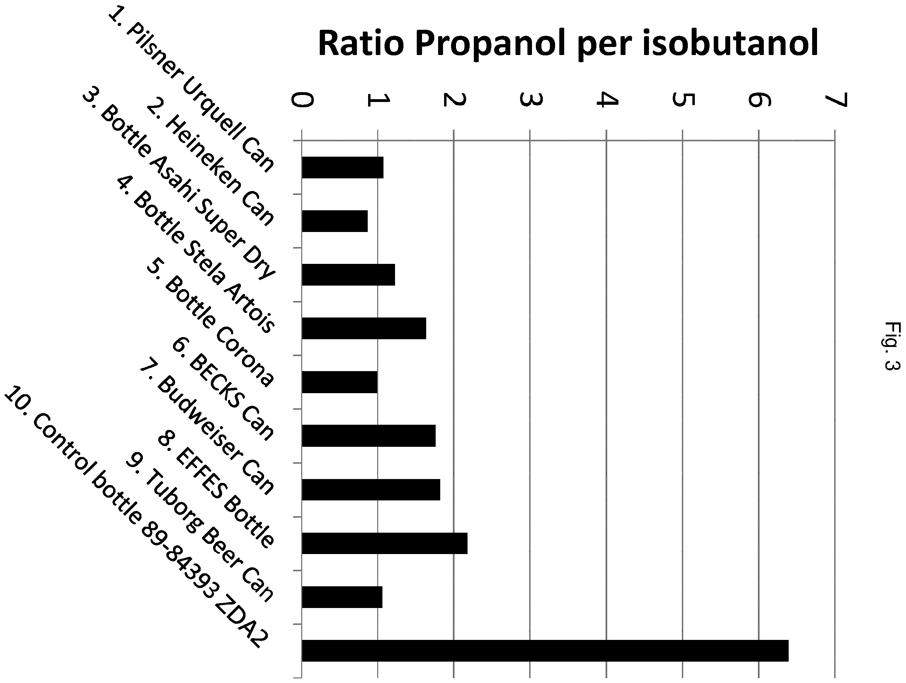

Figure 3 shows the propanol per isobutanol ratio of commercial beers compared to a "control bottle 89-84393 ZDA2" beer produced using the ZDA2 yeast strain according to the invention. The "control bottle 89-84393 ZDA2" is produced at 50L trial and debrewed to 5%. All the commercial beers have lower propanol to isobutanol ratio.

### Example 2 - 50 L trial at 16 °C with yeast strains ZDA2 and ZDA3

Two further yeast hybrid strains with favourable diacetyl profiles were identified and compared with strain ZDA1 of example 1. 50 L beer was prepared from a wort as described in the materials and methods section with a °Plato of 16. Prior to fermentation the wort was adjusted to a pH of 4.9 and zinc was added to a final concentration of 0.30 mg/L. The wort was inoculated with 14 million viable yeast cells per ml of wort. The yeast for inoculation was obtained from a 50 L first fermentation, where the yeast cells were harvested at the end of fermentation and used to re-pitch a second 50 L fermentation trial. This resembles the way large scale beer production is done. Fermentation was performed at 16 °C. Samples were obtained at day 0 and during fermentation as indicated in the table below. The results are shown in table 6 below.

**Table 6. 50 L fermentation at 16°C.**

| **Yeast** | **Day** | **Temp** °C | **°Plato** | **Diacetyl** ppb | **pH** | **Alcohol** Vol% |
|---|---|---|---|---|---|---|
| ZDA1 | 0 | | 16.05 | | 4.9 | |
| | 1 | 16.2 | 13.7 | 90 | 4.45 | |
| | 3 | | | 63 | | |
| | 4 | 16.4 | 2.99 | 48 | 4.22 | |
| | 5 | 16.3 | 2.94 | | 4.24 | 7.29 |
| ZDA2 | 0 | | 16.05 | | 4.9 | |
| | 1 | 16.2 | 14.13 | 48 | 4.47 | |
| | 3 | | | 55 | | |
| | 4 | 16.5 | 3.09 | 42 | 4.21 | |
| | 5 | 16.4 | 3.01 | | 4.24 | 7.28 |
| ZDA3 | 0 | | 16.05 | | 4.9 | |
| | 1 | 15.9 | 13.73 | 68 | 4.41 | |
| | 3 | | | 45 | | |
| | 4 | 16.2 | 3.04 | 35 | 4.28 | |
| | 5 | 15.9 | 3 | | 4.29 | 7.26 |

As shown in table 6, all three candidates (ZDA1, ZDA2 and ZDA3) showed diacetyl levels below 50 ppb at day 4, which corresponded to the time where the apparent extract had reached a plateau. Furthermore, ZDA3 had diacetyl levels below 50 ppb already at day 3.

### Example 3 - Taste results

The fermented wort obtained in example 2 was further processed to final beers by adjusting the alcohol content to 5% by volume, CO₂ to 5.1 g/L and bottled in standard Danish green 33 cl bottles. The beers were evaluated by a specialist beer taste panel, consisting of 10 tasters. Each taste panel member was extensively trained, in particular each member was skilled in evaluating the taste properties of esters, higher alcohols, sulfur components and the body of beer. Each taste panel member evaluated different flavor notes. In particular, the overall taste was evaluated on a scale from 1 to 9 and provided as the "Main result" in Table 7 below.

**Table 7. Taste results of the beer from the 50 L trial of example 2.**

| **Sample name** | **First fermentation** |
|---|---|
| | **Main result** |
| ZDA1 | 6.1 Satisfactory |
| ZDA2 | 5.7 Satisfactory |
| ZDA3 | 5.8 Satisfactory |

As shown in table 7, the samples of beers produced by all strains of yeast were rated at least 5.7 (Satisfactory).

### Example 4 - 10 HL beer trial at 16 °C with yeast strain ZDA1 and comparative strain Hybrid yeast 7

The purpose of this example was to investigate whether the hybrid yeast strain ZDA1 exhibited the same advantageous diacetyl profile as observed at 50 L scale in example 1. 10 HL beer was prepared from a wort as described in the materials and methods section with a °Plato of approx. 16. Prior to fermentation the wort was adjusted to a pH of 4.95 and zinc was added to a final concentration of 0.8 mg/L. The wort was inoculated with at least 15 million viable yeast cells per mL wort of the hybrid yeast strains ZDA1 or the control lager yeast strain Hybrid yeast 7, followed by fermentation at approx. 16 °C. The yeast for inoculation was obtained from a first brew as described in example 2. Samples were obtained at day 0 and at the days indicated in the results table 8 below. The results are shown in table 8 and illustrated in Figure 2A and B.

**Table 8. 10 HL fermentation at 16°C.**

| **Yeast** | **Day** | **Temp** °C | **°Plato** | **Diacetyl** ppb | **pH** | **Alcohol** Vol% |
|---|---|---|---|---|---|---|
| ZDA1 | 0 | 15.7 | 16.05 | | 4.73 | |
| | 1 | 15.9 | 14.24 | 239 | 4.49 | 0.92 |
| | 4 | 15.7 | 2.87 | 55 | 4.11 | 7.15 |
| | 5 | 15.9 | 2.73 | 28 | 4.19 | 7.28 |
| | 6 | 15.9 | 2.65 | 14 | 4.13 | 7.43 |
| Hybrid yeast 7 | 0 | 15.8 | 16.1 | | 4.72 | |
| | 1 | 15.8 | 14.41 | 868 | 4.54 | 0.87 |
| | 4 | 16.0 | 3.34 | 509 | 4.07 | 6.84 |
| | 5 | 15.7 | 2.67 | 205 | 4.06 | 7.32 |
| | 6 | 15.8 | 2.56 | 91 | 4.05 | 7.48 |
| | 7 | 15.0 | 2.53 | 36 | 4.11 | 7.48 |

Yeast strain ZDA1 produces a quite low initial level of diacetyl and the green beer obtained with hybrid yeast strains ZDA1 reached close to the taste threshold of diacetyl (50 ppb) on day 4 and on day 5 it was down to 28 ppb. The green beer obtained with the control yeast strain Hybrid yeast 7, however first reached this threshold at day 7 (Figure 2A). Figure 2B shows that the total diacetyl level in green beer obtained with the yeast hybrid ZDA1 is close to 50 ppb approximately at the same time as the fermentable sugars are close to complete fermentation, i.e. at day 4.

### Example 5 - Taste results

The fermented wort obtained in example 4 was further processed to final beer by adjusting the alcohol content to 4.6% by volume, CO₂ to 5.4 g/L and bottled in 33 cl amber bottles. The beers were evaluated by a specialist beer taste panel bot in Denmark and in France, consisting of 10 tasters. Each taste panel member was extensively trained, in particular each member was skilled in evaluating the taste properties of esters, higher alcohols, sulfur components and the body of beer. Each taste panel member evaluated different flavor notes. In particular, the overall taste was evaluated on a scale from 1 to 9 and provided as the "Main result" in Table 9 below.

**Table 9. Taste results of the beer from the 10 HL trial of example 4.**

| **Sample name** | **Main result** |
|---|---|
| ZDA1 | 6.6 Satisfactory |
| Hybrid yeast 7 | 6.4 Satisfactory |

As shown in table 9, the samples of beers produced by yeast strain ZDA1 was rated 6.6 (Satisfactory), which is a bit above the beer produced with the control strain Hybrid yeast 7.

### Example 6 - Trial at 16 °C including three control lager yeast strains

This experiment was conducted to compare the three novel yeast strains from example 2 with additional commercially available yeast strains as well as the control used in examples 1, 2 and 4. 40 L beer was prepared from a wort as described in the materials and methods section with the small change that the first hold at 52 °C was 30 min. For fermentation, the °Plato was between 15 and 16. Prior to fermentation, the wort was adjusted to a pH of 4.9 and zinc was added to a final concentration 0.30 mg/L. The wort was inoculated with 7.5 million viable yeast cells per ml of the hybrid yeast strains ZDA1, ZDA2 and ZDA3, as well as with a control lager yeast strain Hybrid yeast 7, and the control lager yeast strains W-34/78 which is commercially available from Weihenstephan, Germany. Wort was fermented at approx. 16 °C. Samples were obtained at day 1 and the days indicated in the results table below. The results are shown in table 10.

**Table 10. 40 L fermentation at 16°C.**

| **Yeast** | **Day** | **Temp** °C | **% Plato** | **Diac etyl** ppb | **pH** | **Cells in liquid** (million / ml) | **Alcoh ol** Vol% | **Propan ol** | **Iso-propan ol** |
|---|---|---|---|---|---|---|---|---|---|
| Hybrid yeast 7 | 1 | 15.7 | 15.54 | 88 | 4.71 | | | | |
| | 2 | 16.5 | 12.78 | 431 | 4.45 | | | | |
| | 3 | | | 869 | | | | | |
| | 4 | | | 520 | | | | | |
| | 5 | 16.4 | 2.49 | 265 | 4.02 | | | | |
| | 6 | 16.5 | 2.45 | 115 | 4.05 | 0.8 | | | |
| | 7 | | 2.37 | 57 | 4.09 | | | | |
| | 8 | | | 44 | | | 7.35 | 11.02 | 9.57 |
| | | | | | | | | | |
| | | | | | | | | | |
| | | | | | | | | | |
| | | | | | | | | | |
| | | | | | | | | | |
| | | | | | | | | | |
| | | | | | | | | | |
| | | | | | | | | | |
| W-34/78 | 1 | 14.5 | 15.4 | 176 | 4.64 | | | | |
| | 2 | 16.1 | 12.54 | 470 | 4.33 | | | | |
| | 3 | | | 485 | | | | | |
| | 4 | | | 286 | | | | | |
| | 5 | 16.3 | 2.97 | 150 | 4.1 | | | | |
| | 6 | 16.2 | 2.68 | 62 | 4.15 | | | | |
| | 7 | 15.4 | 2.63 | 41 | 4.19 | 17 | | | |
| | 8 | | | 33 | | | 7.2 | 11.08 | 9.85 |
| ZDA1 | 1 | 15.6 | 15.26 | 89 | 4.69 | | | | |
| | 2 | 16.3 | 12.24 | 166 | 4.38 | | | | |
| | 3 | | | 133 | | | | | |
| | 4 | | | 74 | | | | | |
| | 5 | 16.2 | 2.9 | 42 | 4.15 | | | | |
| | 6 | 16.1 | 2.73 | 24 | 4.2 | 2.5 | | | |
| | 7 | 16.2 | 2.69 | 16 | 4.25 | | | | |
| | 8 | 15.6 | 15.26 | 89 | 4.69 | | 7.18 | 27.04 | 4.00 |
| ZDA2 | 1 | 14.7 | 15.47 | 20 | 4.64 | | | | |
| | 2 | 16.1 | 12.7 | | 4.4 | | | | |
| | 3 | | | 245 | | | | | |
| | 4 | | | 102 | | | | | |
| | 5 | 16.3 | 2.68 | 49 | 4.05 | | | | |
| | 6 | 16.2 | 2.55 | 25 | 4.09 | 1.2 | | | |
| | 7 | 16.2 | 2.5 | 15 | 4.13 | | 7.3 | 22.54 | 3.61 |
| | | | | | | | | | |
| ZDA3 | 1 | 14.6 | 15.5 | 39 | 4.63 | | | | |
| | 2 | 16.5 | 12.96 | 92 | 4.42 | | | | |
| | 3 | | | 116 | | | | | |
| | 4 | | | 63 | | | | | |
| | 5 | 16.4 | 3.78 | 32 | 4.08 | | | | |
| | 6 | 16.5 | 3.28 | 18 | 4.12 | 2.8 | | | |
| | 7 | 16.3 | 3.21 | 12 | 4.15 | | 6.9 | 24.20 | 3.56 |

In this trial all three low diacetyl strains (ZDA1, ZDA2 and ZDA3) showed diacetyl levels below 50 ppb at day 5, which corresponded to the time where the apparent extract had reached a plateau for all yeast strains tested, while none of the control strains showed diacetyl levels below 110 ppb at the same day. The earliest point in time where the apparent extract did not decrease by more than 0.50° Plato in the preceding 24 hours is considered to be day 6 for all of these trials. At day 6, all the low diacetyl strains (ZDA1, ZDA2 and ZDA3) had a level of diacetyl below 45 ppb (24 ppb, 25 ppb and 18 ppb, respectively), whereas the control strains (Hybrid yeast 7, W-34/70 and W-34/78) had higher levels of diacetyl (115 ppb, 62 ppb and 62 ppb, respectively).

These results are quite remarkable considering that the Weihenstephan yeast is less flocculent than the Hybrid yeast 7 strain and the strains of the invention. The cell count in the liquid (above the yeast pellet) at the end of fermentation is a good measure of flocculation. These data are shown in table 10 above as Cells in Liquid (the lower the value the more flocculent the yeast is). High flocculation is an advantage when the yeast is to be harvested from the production tank to inoculate the next wort for beer production. If the flocculation of the yeast is low, this process will require cooling of the green beer for up to 24 hours. High flocculation, on the other hand, can result in less contact between yeast and wort during fermentation, which is likely to reduce the speed at which the yeast can consume diacetyl and prolong fermentation time.

The examples described herein therefore provide yeast strains which are capable of reducing diacetyl to less than 50 ppb approximately at the same time where the fermentable sugars have been consumed (or even before that) and at the same time does not require any additional cooling of the green beer to harvest the yeast for the next beer production due to their high flocculation at 16°C.

Furthermore it can be seen that the low diacetyl yeast strains easily can be identified via the propanol/isobutanol ratio since they all are above 6, whereas the control stains all are below 1.4.

### Example 7 - Genotyping of yeast strains

### Assembly of the genomes

For four (Hybrid Yeast 7, ZDA1, ZDA2 and W 34-78) out of five genomes a tandem analysis approach of using the NGS data from Illumina and Pacbio platforms has been deployed. Quality control, filtering and assembly have been carried out following an in-house designed pipeline. All the genomes were de novo assembled using Canu 1.9 software which uses data generated by Pacbio platform. Subsequently the reads generated by Illumina platform were mapped (Pilon 1.22) against the assembly produced by Canu 1.9 in order to improve the consensus accuracy of the draft assemblies. For the genome ZDA3, due to the absence of Illumina data, only Canu 1.9 has been used without further assembly improvement.

The resulting assembled genomes were used in the BLAST queries.

### Blast and alignment of the hits

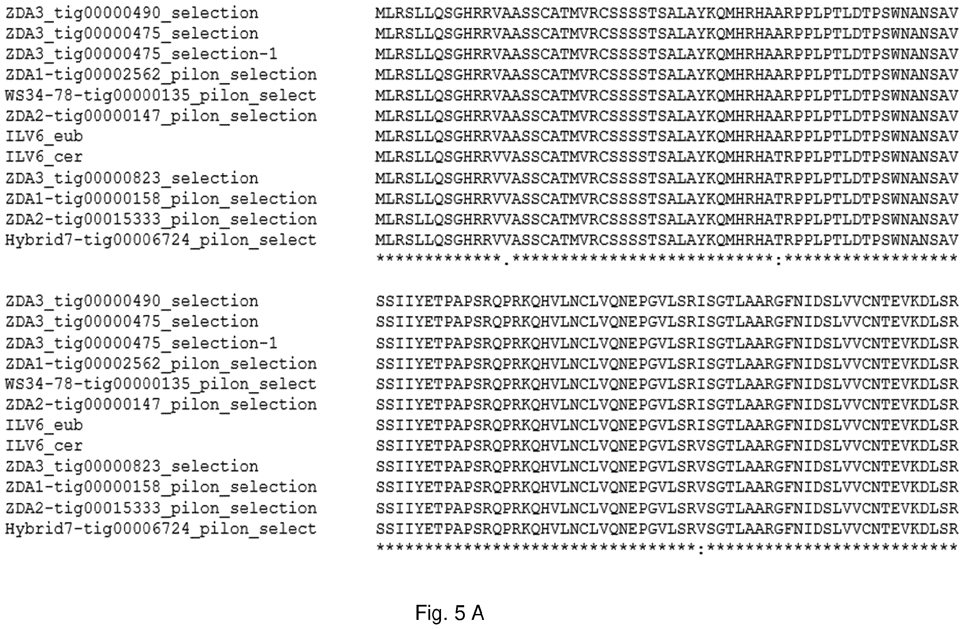

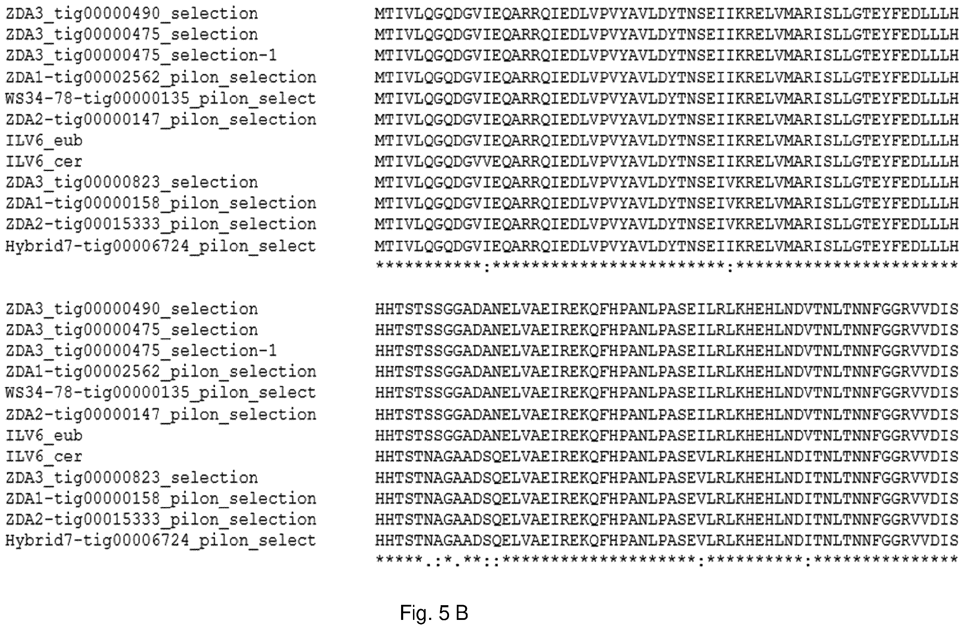

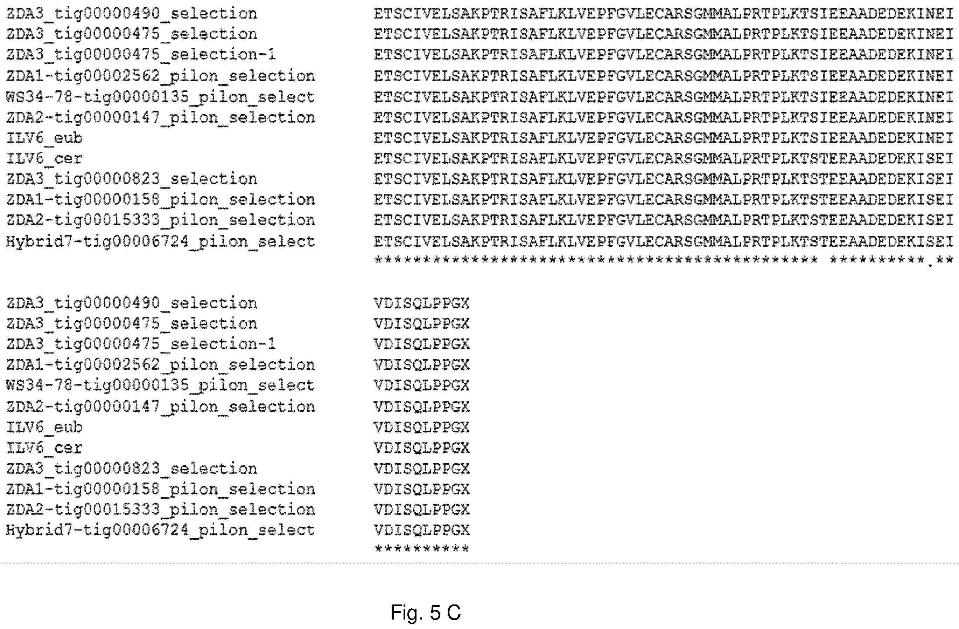

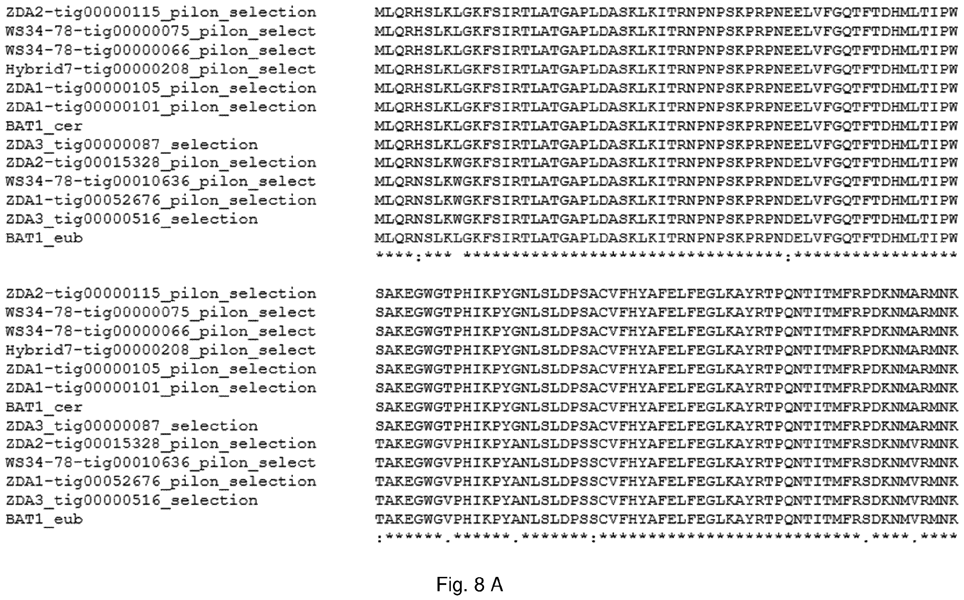

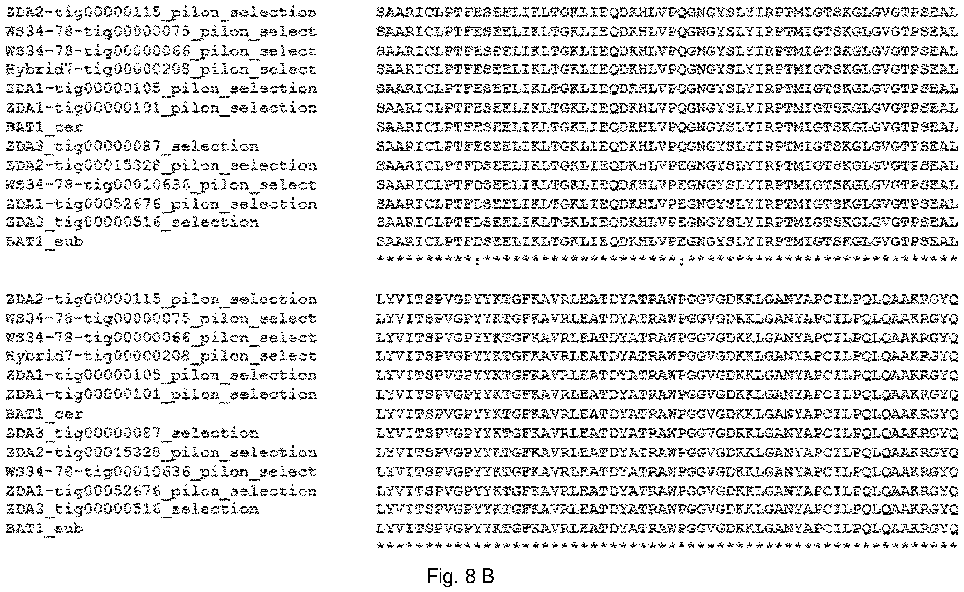

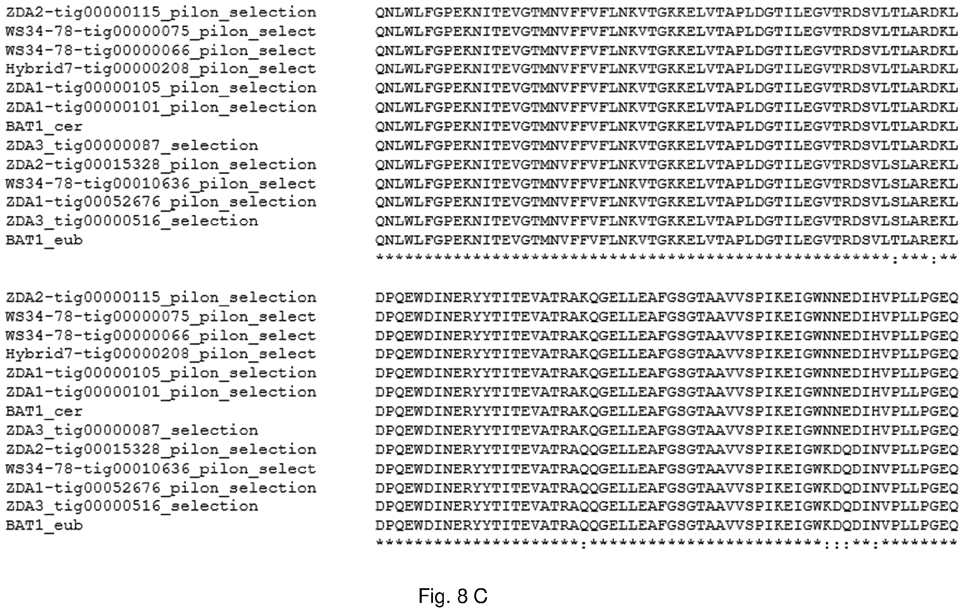

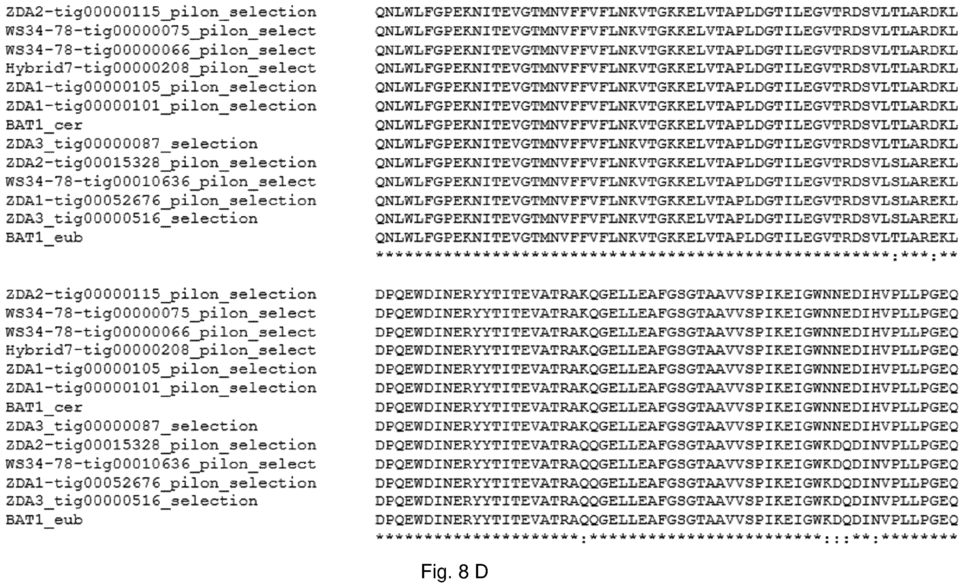

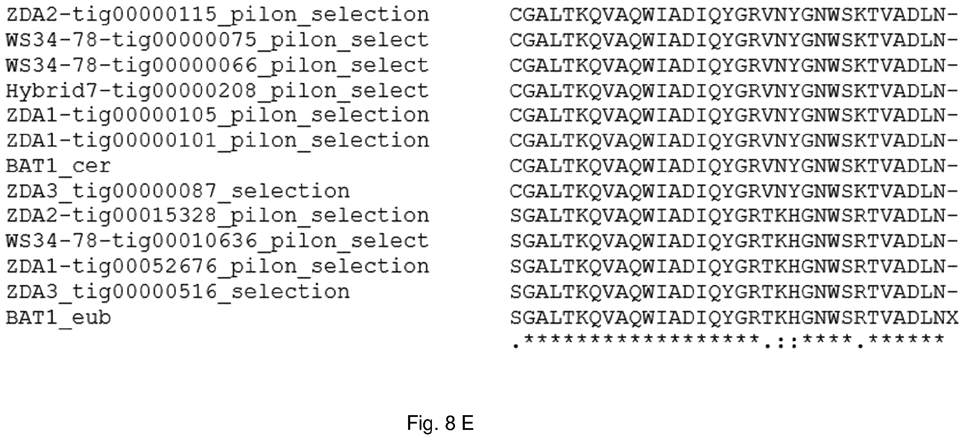

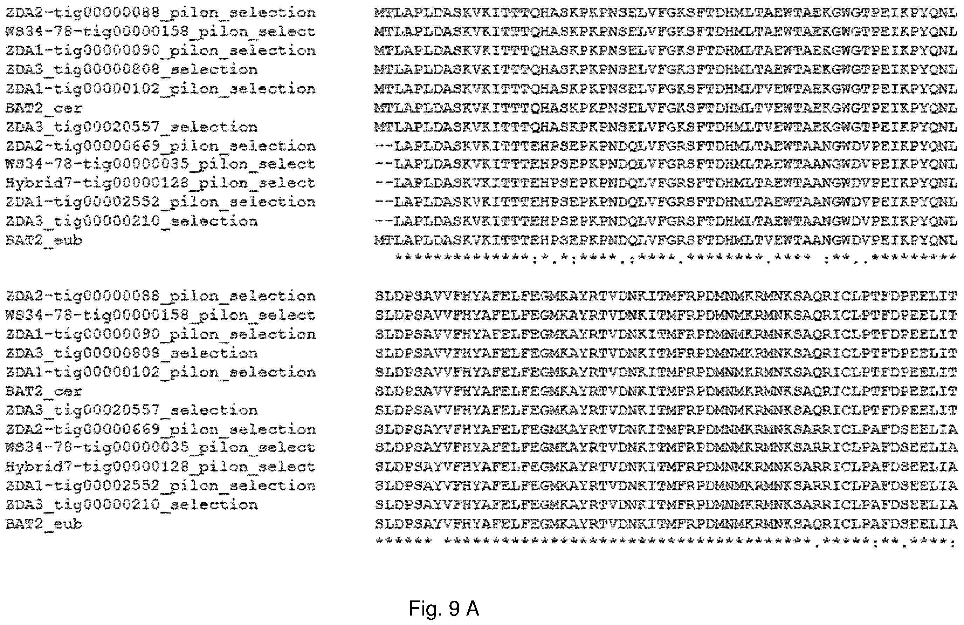

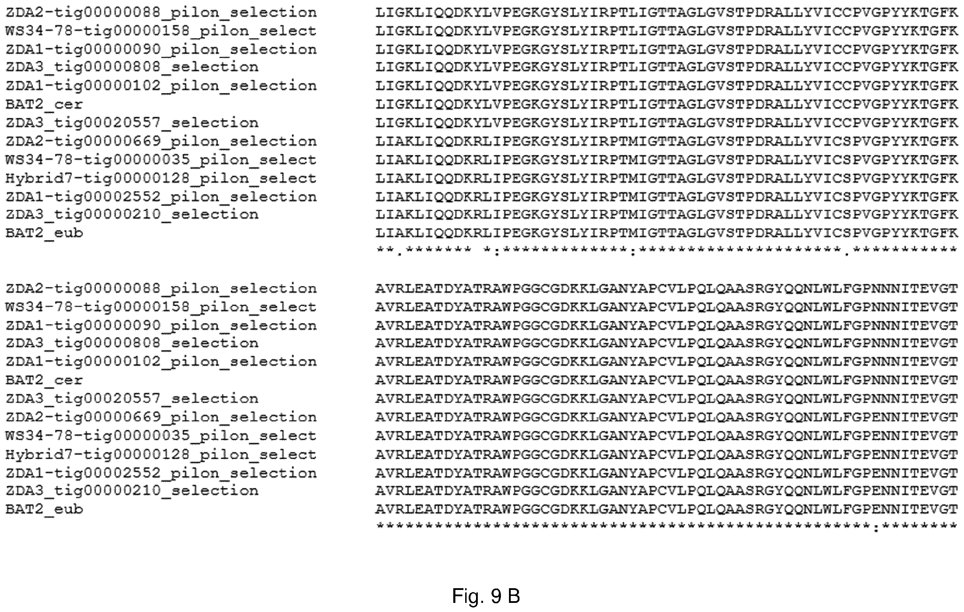

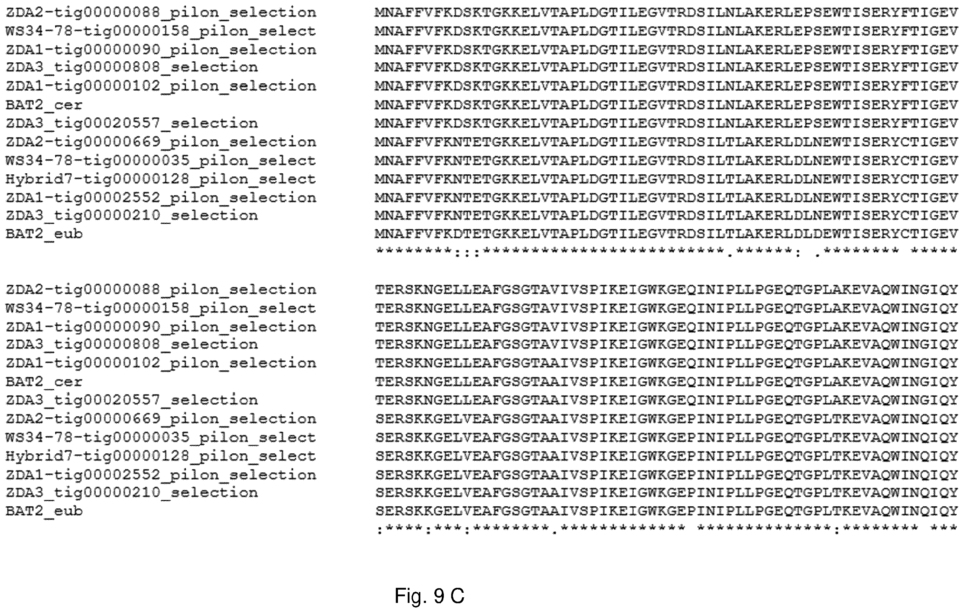

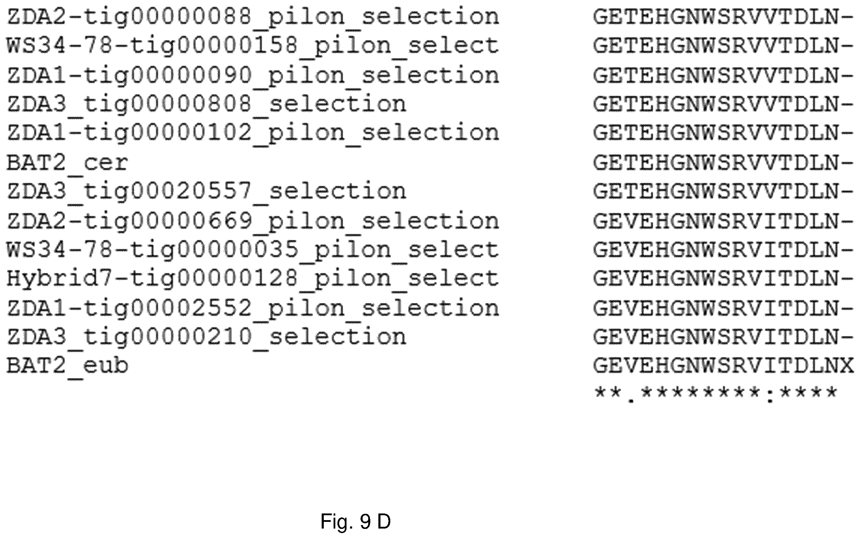

For each of the genes (*ILV2, ILV3, ILV5, ILV6, BAT1, BAT2*) the corresponding amino acid sequence of the S. *cerevisiae* gene was used as a query sequence against each of the 5 genomes using TBLAStN algorithm. Finally, the hits were filtered by retaining ones having at least 65-80% sequence identity or covering at least 40% of the length of the query sequence. Amino acid sequence alignment of ILV2, ILV3, ILV5, ILV6, BAT1, BAT2 between WS34-78, cer (*Saccharomyces cerevisiae*)*,* eub (*Saccharomyces eubayanus*), Hybrid7 (Hybrid yeast 7), ZDA1 and ZDA2 are shown in Figures 4-9

The BLAST analysis was not repeated for the *S*. *Eubayanus* corresponding genes, since on the last step of filtering the hits were allowed to have as low as 65% sequence identity, thus in this setup all the hits from both *S*. *cerevisiae* and *S*. *eubayanus* would be captured because of the high similarity in between them. MUSCLE (3.8) multiple sequence alignment tool was used to align all the hits identified through BLAST analysis.

### Ploidy identification analysis

The ploidy identification analysis has been carried out by deploying a modified version of the spplDer algorithm. Short reads of Illumina platform of four out of five genomes were mapped to a combination reference genome of S. *cerevisiae* and S. *eubayanus* and reads with a mapping quality (MQ) >3 were retained and sorted into the combination reference genome order; then, coverage across the combination reference genome was computed. A custom script then calculated the mean coverage for each species, and the combination reference genome broken into windows.

### Technical shortcomings of BLAST analysis

Using BLAST to study the effect of genomic differences on the phenotype of strains suffers from various shortcomings, the most important being:
i) the inability to assess duplications at the gene and/or chromosome level
ii) the sensitivity to errors or ambiguities in the assemblies

All genome assembly methods have problems in creating meaningful assemblies in the presence of polyploidy. This results in an outcome where multiple regions from different chromosome copies are collapsed into a single scaffold. In principle, the situation is less severe when gene duplication events occur, since those might be in identified using specific assembly methods and proper experimental setups. It is however still quite likely that gene duplications, especially if no or limited genomic differences are present in the different copies, will fail to be properly detected at the assembly stage. As for the second point, BLAST would be in principle be able to identify more detailed information on different variants, such as functional SNPs and non-functional ORFs. However, errors or ambiguity in the assembly might produce a large number of false calls - e.g. spurious SNPs and ambiguous gene architectures.

We have therefore decided to perform, in parallel to the simple BLAST identification of variants, an estimate of the observed ploidy of each genomic region in the sequencing data, resulting by chromosome ploidy, and gene duplication or deletion.

Overall the generation of haplotype-resolved de novo assemblies remains a major challenge nowadays. The NGS techniques (Pacbio and Illumina) produce huge amount of data, but it is still technically difficult distinguishing between errors and true sequence variants. On the top of it, one needs to assign the true variants to the different genome copies when the sample is polyploid. The assemblies in these analyses were carried out using haplotype-unaware algorithms, thus the final resulting assembly represented the collapsed haplotype genome.

### Results

The results are shown in **Table 11,** below.

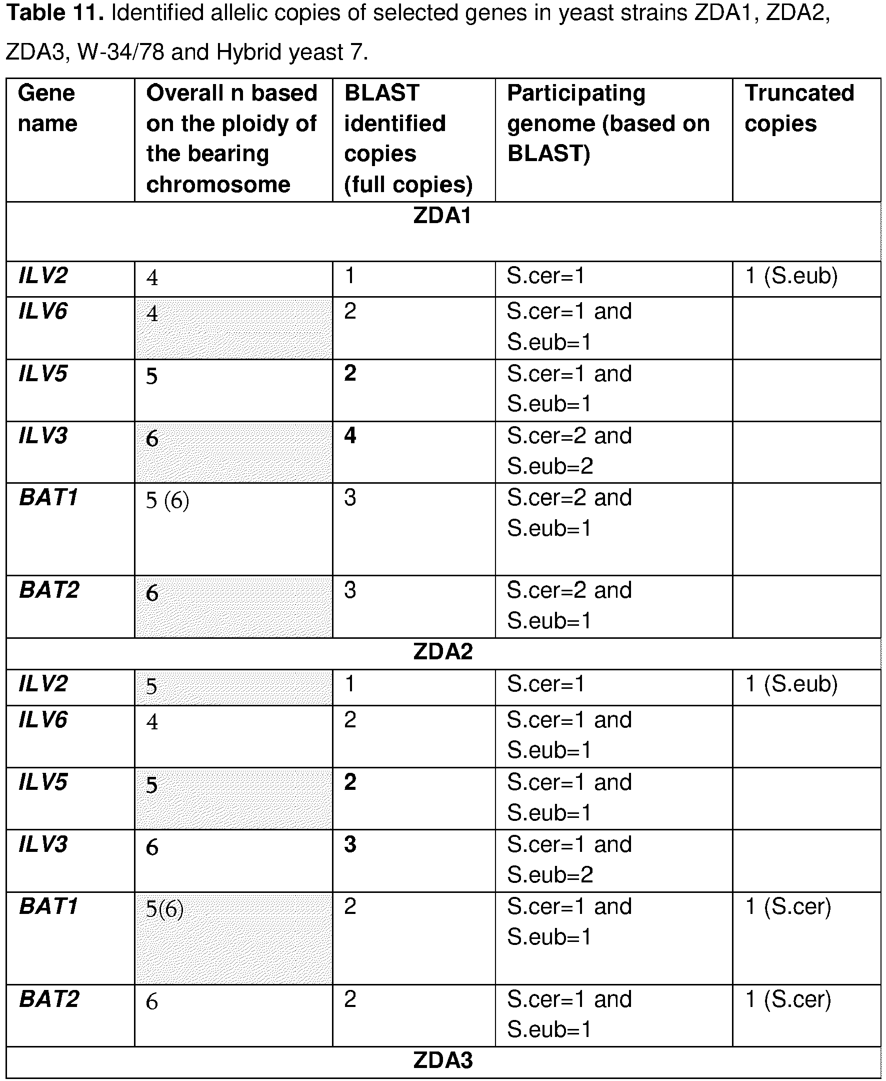

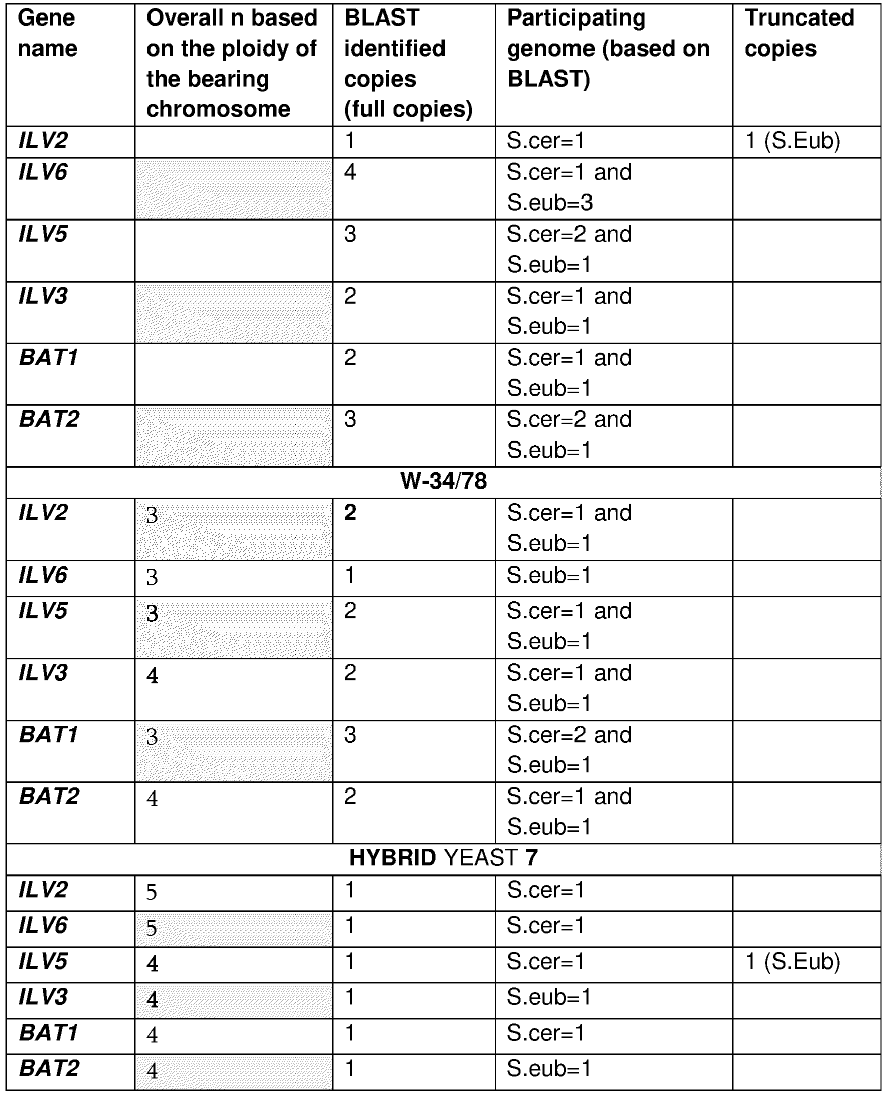

The number in the brackets is the mode ploidy of the chromosome. The number without the brackets is the ploidy of the gene location on that chromosome.

### Example 8 - Commercial scale brewing trial with yeast strain ZDA2 and comparative strain Hybrid yeast 7

The purpose of this example was to investigate whether the hybrid yeast strain ZDA2 exhibited the same advantageous diacetyl profile as observed in the previous examples when applied in commercial scale brewing (approximately 1800 HL) where yeast is harvested and re-pitched.

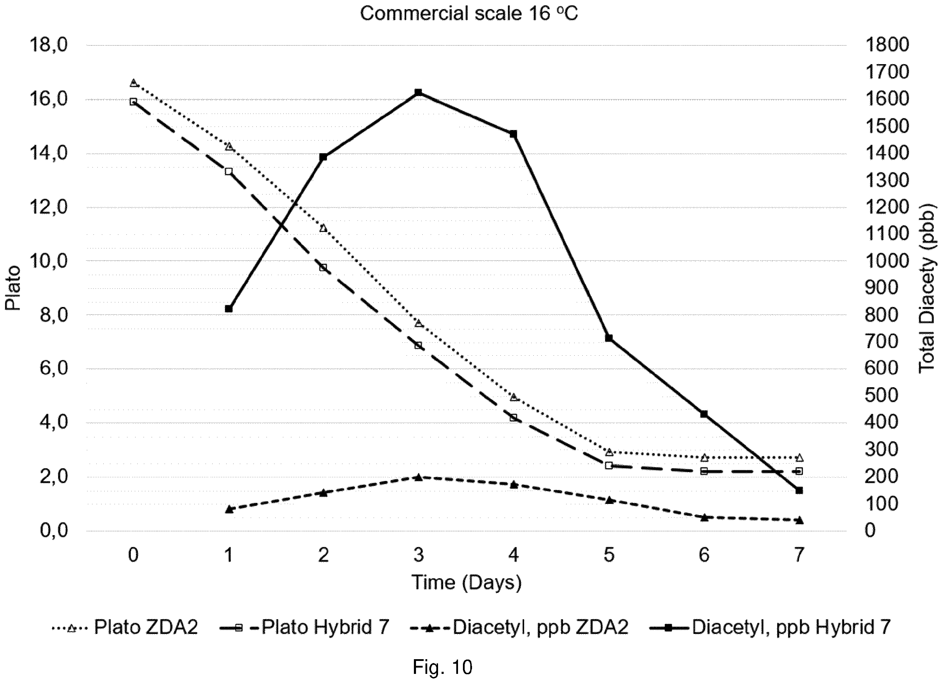

1800 HL beer was prepared from a wort comprising malt and adjunct obtained by conventional infusion mashing and with a °Plato of approx. 16. Prior to fermentation the wort was adjusted to a pH of 4.9- 5.0. The wort was re-pitched with generation 8 of both the ZDA2 and control lager yeast strain Hybrid yeast 7, and fermented at approx. 16 °C. Samples were obtained at day 0 and at the days indicated in the results table 12 below and illustrated in Figure 10.

**Table 12. 1800 HL fermentation at 16°C.**

| **Yeast** | **Day** | **Temp** °C | **°Plato** | **Diacetyl** ppb | **pH** | **Alcohol** Vol% | **RDF %** |
|---|---|---|---|---|---|---|---|
| ZDA2 | 0 | 15.0 | 16.60 | | 5.00 | | |
| | 1 | 15.0 | 14.25 | 80.0 | 4.60 | | |
| | 2 | 16.0 | 11.23 | 142.0 | 4.42 | | |
| | 3 | 16.0 | 7.70 | 200.0 | 4.29 | | |
| | 4 | 16.0 | 4.95 | 172.0 | 4.24 | | |
| | 5 | 16.0 | 2.91 | 116.0 | 4.27 | | |
| | 6 | 16.0 | 2.72 | 51.0 | 4.35 | | |
| | 7 | 16.0 | 2.70 | 38.0 | 4.36 | 7.64 | 69.9 |
| Hybrid yeast 7 | 0 | 15.0 | 15.90 | | 4.90 | | |
| | 1 | 15.0 | 13.30 | 822.0 | 4.54 | | |
| | 2 | 16.0 | 9.73 | 1387.0 | 4.37 | | |
| | 3 | 16.0 | 6.85 | 1622.0 | 4.27 | | |
| | 4 | 16.0 | 4.17 | 1469.0 | 4.23 | | |
| | 5 | 16.0 | 2.39 | 713.0 | 4.25 | | |
| | 6 | 16.0 | 2.18 | 429.0 | 4.29 | | |
| | 7 | 16.0 | 2.18 | 148.0 | 4.28 | 7.52 | 71.9 |

Yeast strain ZDA2 produces a quite low level of diacetyl throughout the fermentation and the green beer obtained with hybrid yeast strain ZDA2 reached the taste threshold of diacetyl (50 ppb) already on day 6. At that time the plato had not decreased more than 0.5 the last 24 hours. The green beer obtained with the control yeast strain Hybrid yeast 7, had not reached the taste threshold for diacetyl at day 7, two days after the plato did not decrease more than 0.5 over 24 hours (Figure 10). Consequently, the specifications for the green beer in terms of diacetyl and plato was reached at least 2 days earlier when using the ZDA2 yeast compared to the reference yeast Hybrid yeast 7.

Interestingly, the ZDA2 yeast reached a real degree of fermentation (RDF) which was 2% lower than the Hybrid yeast 7, while the alcohol percentage is almost similar in the two green beers. Consequently, the ZDA yeast is capable of producing the same amount of alcohol with a lower real degree of fermentation, which will result in a beer with a better mouthfeel. This trend of a 1.5 - 2.5% lower RDF at the same alcohol level for the ZDA2 yeast was observed across all the generations of fermentations preceding the 8^{th} generation shown in this example (see table 13).

**Table 13 - RDF and ABV across 8 generations of fermentations.**

| **Yeast generation** | | **1** | **2** | **3** | **4** | **5** | **6** | **7** | **8** |
|---|---|---|---|---|---|---|---|---|---|
| **ZDA2** | RDF | 68.2 | 69.5 | 69 | 69.5 | 68.9 | 69 | 70.4 | 69.9 |
| | ABV | 7.2 | 7.5 | 7.31 | 7.38 | 7.43 | 7.43 | 7.58 | 7.64 |
| **Hybrid yeast 7** | RDF | 70.1 | 70.6 | 71.1 | 71.1 | 71.3 | 71.3 | 72.1 | 71.9 |
| | ABV | 7.4 | 7.28 | 7.36 | 7.48 | 7.47 | 7.58 | 7.6 | 7.52 |

### Example 9 - Hybrid Nanopore - Illumina Sequencing and Bioinformatics Evaluations of brewing strains to calculate copy number of relevant genes

Strains were sequenced using the Illumina platform as described previously.

For Nanopore sequencing, ultra high molecular weight DNA was prepared using the methodology outlined by Denis et al.

To prepare the HMW gDNA for sequencing the samples were processed using the Zymo Genomic Clean and Concentrator columns and afterwards checked for quality and quantity using the deNovix dsDNA Broad Range fluorometric Assay.

All samples were multiplexed and libraries were generated by using SQK-LSK109 chemistry and Native Barcode Extension packs EXP-NBD104 and EXP-NBD114 from Oxford Nanopore Technologies. All necessary clean up steps were carried out using Clean NA magnetic beads for NGS. Genome Sequencing took place on MinlOn FlowCells FLO-MIN106D over 48-72h.

Raw sequencing Data were basecalled using bonito (https://github.com/nanoporetech/bonito) and demultiplexed using guppy (https://nanoporetech.com/nanopore-sequencing-data-analysis). Hybrid-Genome assemblies were carried out using Marsurca (https://github.com/alekseyzimin/masurca) in combination with the Nanopore and Illumina fastq files and the assembled genomes were annotated with prokka. (https://github.com/tseemann/prokka). The sequencing coverage and copy number of the genes of interest were calculated with qualimap (http://qualimap.conesalab.org/) and visualized using the Integrative Genomics Viewer (https://software.broadinstitute.org/software/igv/).

Read mapping and SNP detection was performed in CLC Genomics work Bench (version 11). Briefly, trimmed reads were mapped to an *"in silico"* yeast hybrid genome which comprised a concatenation of the *S*. *eubayanus* (Accession: GCA_0012986.25.1) and *S*. *cerevisiae* (GCA_000146045.2) genomes. Using the basic variant detection tool, single-nucleotide polymorphisms (SNP's) and insertions/deletions (indels) were detected. Here, the functional copy number of ILV2 genes could be estimated based upon diagnostic SNP's which existed on single alleles within the ZDA yeast.

In the *S*. *cerevisiae* ILV2 genes there is a C' frameshift which inactivates the resulting protein (Leu575fs) due to a T nucleotide insertion within this codon. The % of Illumina reads mapping at this frequency was roughly 33% suggesting that 1 out 3 ILV2sc copies had been inactivated. Furthermore it was observed that in the *eubayanus* ILV2 alleles, a T nucleotide deletion resulted in a frameshift that results in an inactivating truncation (Leu304fs). Knowing the copy number (CN) of ILV2 *cerevisiae* genes allowed calculation of copy number of other genes based on read mapping as described previously. Estimation of the WS34/78 CN was also based on read mapping based estimation where the ILV2 copy number was based on prior evidence that a highly similar strain (WS34/70) is a tetraploid yeast with equal *S. cerevisiae* and *S. eubayanus* genome content (Walther et al.2014). Furthermore, in example 10, when removing ILV2 *eubayanus* alleles, it was observed that WS34/78 indeed had two copies of ILV2 euybanus alleles (a total of 4 ILV2 alleles, including cerevisiae alleles). These results are summarized in **Table 14.**

**Table 14. Calculated Functional Copy Numbers of Genes of Interest. Cere refers to Cerevisiae alleles and Eub refers to Euybanus alleles.**

| | Functional copy number - ZDA1 | Functional copy number - ZDA2 | Functional copy number - WS3478 |
|---|---|---|---|
| ILV2_Cere | 2 | 2 | 2 |
| ILV2_Eub | 0 | 0 | 2 |
| ILV3_Cere | 4 | 4 | 2 |
| ILV3_Eub | 2 | 2 | 2 |
| ILV5_Cere | 3 | 3 | 1 |
| ILV5_Eub | 2 | 2 | 2 |
| ILV6_Cere | 2 | 2 | 1 |
| IIV6_Eub | 2 | 2 | 3 |
| BAT1_Cere | 3 | 3 | 4 |
| BAT1_Eub | 2 | 2 | 2 |
| BAT2_Cere | 4 | 4 | 2 |
| BAT2_Eub | 2 | 2 | 3 |

The reference WS3478 strain has 4 functional copies of the ILV2 gene, while ZDA1 and ZDA2 each only have 2 functional copies of this gene. In addition, WS3478 has 4 functional copies of ILV3 and 3 functional copies of ILV5, while this number is 6 and 5, respectively, for the ZDA1 and ZDA2 strains.

### Example 10 - Gene Copy Number Adaptations in the Model Brewer's Yeast W-34/78

The overall goal of this experiment was to adjust copy numbers of relevant genes in a model yeast strain (*Saccharomyces pastorianus ssp. carlsbergensis* strain (WS34/78) to mirror the genotype of ZDA1 and ZDA2. WS34/78 is commercially available from Weihenstephan, Germany and is also referred to as W-34/78 herein. To do so, genes in strain WS34/78 had to be either inactivated or additionally expressed according to **Table 15.**

**Table 15. Necessary alterations in the genotype of strain WS34/78 to mirror the relevant genotype of low diacetyl strains of interest**

| **Gene** | **Allele** | **Alteration** |
|---|---|---|
| ILV2 | *S. eubayanus* | -2 Copies |
| ILV3 | *S. cerevisiae* | +2 Copies |
| ILV5 | *S. cerevisiae* | +2 Copies |

Inactivation was done with the help of resistance conferring antibiotic-cassettes that targeted the coding sequences of the genes of interest (i.e. ILV2). Here, the coding DNA sequence (cds) was looped out via homologous recombination in a way that only 99 bp of the N-terminal region, and 107 bp of the C-terminal region of the gene remained. Overexpression of genes (i.e. ILV3 and ILV5) was done in a way that left the authentic genomic context intact, i.e. a cassette that comprised roughly 1 kbp upstream of the cds, the cds, and 0,5 kbp downstream of the cds was cloned. Thus the cassette comprises the native promotor and terminator sequences, and expression of the additional gene copies is presumably under native control. Furthermore, a yeast single-copy plasmid was used so that the copy number was under control.

### Transformation of WS34/78 cells

Cells of *Saccharomyces pastorianus ssp. carlsbergensis* strain WS34/78 were made competent for DNA uptake following the method described by Gietz and Schiestel (DOI: 10.1038/nprot.2007.17). Routinely, cells were transformed with 1 µg of plasmid DNA or PCR-amplicon. The duration of the heat-shock treatment was reduced to 15 min while the temperature was kept at 42°C.

### Generation of expression cassettes

Based on DNA-sequence information of strain WS34/78 from Example 9, expression cassettes were generated by PCR-amplification of the native WS34/78 DNA-sequences comprising roughly 1000 bp upstream of the cds and 500 bp downstream of the cds, thus comprising the native promotor and terminator sequences. The exact length of the amplicons can be deduced from the respective primer sequences given in the examples. The cds-flanking regions of 1000 bp, or 500 bp, should allow the authentic / native control of the genes of interest. The cloned PCR-amplicons (in plasmid / integrated in chromosome) were controlled by Sanger-DNA sequencing with isolated plasmid DNA as template for sequencing reactions.

### Chromosomal integration of expression cassettes

In a first step, the expression cassette was assembled by fusing the gene expression module with an antibiotic resistance module that allows for positive selection of the integration event. The cassette was fused to PCR-amplicons that encode DNA-stretches for homologous recombination with the chromosome of WS34/78. The sites for integration was the PAD1 loci of WS34/78, as this gene is non-functional in this yeast, and the replacement of this gene thus has no effect on the flavor profile. The flanking regions used for homologous recombination had a length of roughly 500 bp. Yeast clones that grew in presence of the antibiotic were analyzed by PCR (i.e. colony PCR), to verify the insertion of the cassette. Positive clones were further analyzed for mutations in the expression module. Amplicons that cover the expression module were controlled by Sanger-DNA sequencing.

### Cloning of ILV-genes in plasmid pYESVIII

Plasmid pYESVIII (BRAIN;) is an *E.coli* - Yeast shuttle vector that is replicated at high copy number in *E. coli* and maintained as a single copy plasmid in yeast cells due to the CEN6 ori of replication. Expression cassettes were routinely inserted in the multiple cloning site using Gibson-assembly.

If two copies of the same allele had to be inserted, preferably one copy of the gene of interest (GOI) had the authentic DNA-sequence, while the cds of the second copy was altered in a way that left the native protein sequence unaltered. This was achieved by chemical gene synthesis employing algorithms to optimize gene expression in yeast cells (codon usage optimization). This was done to reduce the chances of recombination events upon plasmid transformation into the yeast cells.

For the expression of ILV3 genes from a plasmid, a tandem construct will be assembled (bi-cistronic expression). Here, a single promotor governs the expression of the two ILV3 coding sequences (one native, one synthetic cds). The two coding sequences will be joined by a 2A-peptide linker that enables the ribosomal skipping, so that both cds are translated under control of the native ILV3 promotor. The 2A-peptide technology alters the C-terminus of the first protein by adding a 3 amino acid tag (NPG), while the N-terminus of the subsequent protein starts with a proline (Liu, Z. et al., 2017; DOI: 10.1038/s41598-017-02460-2).

*E. coli* was transformed with the *in vitro* assembled plasmids, plasmids were isolated from transformants, controlled by restriction digest and DNA-sequencing, followed by transformation of competent WS34/78 cells.

**Table 16. Strains and plasmids used/constructed in the project. Subscript sc denotes Saccharomyces cerevisiae genes and eu denotes Saccharomyces eubayanus genes in the interspecies hybrid brewing yeast WS34/78.**

| **Strain** | **ILV Genotype** | **Manipulation / Adjustment** | **Description** |
|---|---|---|---|
| #1 | Native | None (WT Strain) | *Saccharomyces pastorianus ssp. carlsbergensis* strain WS34/78 |
| #2 | ΔΔilv2eu | WS34/78 [2x ILV2eu]:: Zeo R | Both ILV2 alleles *(S. eubayanus*) inactivated by insertion of ShBle-cassette |
| #3 | ΔΔilv2eu; +1 ILV3eu | Strain #2; PAD1 ::ILV3eu | ILV3 *(S. eubayanus*) expression cassette insertion in PAD1 locus |
| #4 | Native | Plasmid #0 | Strain #1 transformed with plasmid pYESVIII W/O insert |
| #5 | ΔΔilv2eu; +1 ILV5sc | Strain #2, Plasmid #1 | Strain #2 harbouring pYESVIII encoding 1 copy of the ILV5sc allele |
| #7 | ΔΔilv2eu; +1 ILV5sc; +1 ILV3eu | Strain #3, Plasmid #1 | Strain #3 harbouring pYESVIII encoding 1 copy of the ILV5sc allele |

| **Plasmids** | | | |
|---|---|---|---|
| #0 | G418R | pYESVIII | Base plasmid; cen6-ori; KanMX-resistance marker |
| #1 | +1 ILV5sc | pYESVIII, 1x ILV5sc | ILV5sc allele cloned in plasmid #0 |

### Examples / mutant variants of S. pastorianus WS34/78

### Recombinant strains

### Generation of strain #2

### Templates for PCR:

Plasmid pYES5-Cen6-Sh.ble (sc) 2.0 (BRAIN), encoding the Sh.ble cassette gDNA *S. pastorianus* WS34/78, strain #1 (Table. 16)

### Primers used:

CPA43: SEQ ID NO: 44
CPA44: SEQ ID NO: 45
CPA49: SEQ ID NO: 46
CPA50: SEQ ID NO: 47
CPA51: SEQ ID NO: 48
CPA52: SEQ ID NO: 49

The sh.ble-cassette (fragment 1) was amplified from plasmid pYES5-Cen6-Sh.ble (*Saccharomyces cerevisiae* (sc)) 2.0 (BRAIN Biotech AG) using primers CPA43/44. Homology arms were generated by amplification of the ILV2 (*Saccharomyces eubayanus* (se)) flanking regions from gDNA strain #1 with the allele-specific primers CPA49/51 (5'-flanking region; fragment 2) and CPA50/52 (3'-flanking region; fragment 3). The HR template was generated with the aid of overlap extension PCR using primers CPA49/50 and fragments 1 + 2 + 3 as template. Allele-specific knockout of ILV2 (se) via homologous recombination was assisted by BRAIN's proprietary NUCLEASE technology. This resulted in a number of strains which had single or double integrations at ILV2 (se) loci and was visible on an agarose gel. A strain with a double deletion of ILV2 (se) was chosen for downstream analysis.

Sequence of the ILV2 (se) knockout cassette: SEQ ID NO: 50

### Generation of strain #3

### Templates for PCR:

gDNA *S*. *pastorianus* WS34/78, strain #1 (Table. 16)
NTC-DNA cassette (Nourseothricin); BRAIN Biotech AG

### Primers used:

CPA70: SEQ ID NO: 51
CPA71: SEQ ID NO: 52
CPA91: SEQ ID NO: 53
CPA96: SEQ ID NO: 54
CPA111: SEQ ID NO: 55
CPA112: SEQ ID NO: 56
CPA113: SEQ ID NO: 57
CPA114: SEQ ID NO: 58
CPA128: SEQ ID NO: 59
CPA129: SEQ ID NO: 60

ILV3 (se) expression cassette (product1) was amplified from gDNA strain #1 with allele-specific primers CPA70/71. Product1 was then further amplified with primers CPA70/129, resulting in product2. Amplification of NTC-expression cassette (products) was performed with primers CPA91/96 and NTC-DNA-String as template. Product4 was generated by amplification of products with primers CPA91/128. Products and product4 were assembled via NEBuilder^{®} HiFi DNA Assembly kit and amplified with primers CPA70/91 to create fragment1. Homology arms were generated by amplification of the PAD1 flanking regions from gDNA strain #1 with primers CPA111/112 (5'-flanking region; fragment 2) and CPA113/114 (3'-flanking region; fragment 3). The final ILV3-NTC integration cassette was generated by assembly of fragment 1 + 2 + 3, followed by amplification with primers CPA111/114. In order to generate strain #3, strain#2 was transformed with the final ILV3-NTC integration cassette.
Sequence of the ILV3 (se) - NTC integration cassette: SEQ ID NO: 61

### Recombinant plasmids

### Construction of plasmid #1

### Vector preparation

Plasmid #0 (pYESVIII, BRAIN Biotech AG) was linearized using Pmel present in the multiple cloning site. Prior to purification the hydrolyzed vector was dephosphorylated.

### Generation of Vector insert

### Template for PCR:

gDNA *S. pastorianus* WS34/78, strain #1 (Table. 16)

### Primers used:

CPA74: SEQ ID NO: 62
CPA75: SEQ ID NO: 63
CPA86: SEQ ID NO: 64
CPA87: SEQ ID NO: 65

The native ILV5-expression cassette (sc) was amplified from WS34/78 gDNA using allele-specific primers CPA74/75. Vector insert was then generated by amplification of the resulting PCR product with primers CPA86/87.

The ILV5-expression vector (plasmid #1) was assembled via NEBuilder^{®} HiFi DNA Assembly and *E. coli* DH10β were transformed with the reaction product.

After plasmid preparation the expression cassette was analyzed by Sanger-DNA sequencing.
Sequence of the ILV5 (sc) expression cassette: SEQ ID NO: 66

### Example 11 - Further Gene Copy Number Adaptations in the Model Brewer's Yeast WS34/78

The purpose of this experiment was similar to Example 10, i.e. to generate further genetically modified WS34/78 yeast strains to adjust copy numbers of relevant genes to mirror the genotype of ZDA1 and ZDA2.

### PCR of native Saccharomyces cerevisiae genes

The S. *cerevisiae* genes *ILV3, BAT1, BAT2,* and *ILV6,* alongside their native promoter and terminator regions were amplified from Weihenstephan 34/78 (W34/78) lager yeast using the primers SEQ ID no 67 to SEQ ID no 74 and Phusion^{®} High-Fidelity DNA Polymerase (New England BioLabs). Regions to target native promoter and terminator were selected approximately 1000 base pairs upstream of the start codon (promoter) and 400 base pairs downs stream of the stop codon (terminator) according to previous literature (Mumberg et al, 1995). Genomic DNA used as template for the PCR reactions was extracted from W34/78 yeast using the MasterPure Yeast DNA Purification Kit fact No MPY80200 from Lucigen.

Primers used to amplify upstream and downstream of the desired *S. cerevisiae* genes:
ScILV3_F: SEQ ID NO: 67
ScILV3_R: SEQ ID NO: 68
ScBAT1_F: SEQ ID NO: 69
ScBAT1_R: SEQ ID NO: 70
ScBAT2_F: SEQ ID NO: 71
ScBAT2_R: SEQ ID NO: 72
ScILV6_F: SEQ ID NO: 73
ScILV6_R: SEQ ID NO: 74

### Cloning of PCR fragments into plasmid vectors by Gibson Assembly and Transformation into E. coli cells

The pure PCR fragments were cloned into a centromeric single copy vector containing the kanMX G418 resistance marker. In some instances, the genes were combined to create plasmids containing more than one gene. pSH67 from Euroscarf (http://www.euroscarf.de/plasmid details.php?accno=P30673) was linearized with Pvull-HF (New England BioLabs) at 37 ºC for 60 minutes to remove the Cre-expressing construct. The linearized plasmid was then ligated with T7 DNA ligase (New England BioLabs) at 25 ºC overnight. The ligated plasmid was transformed into NEB 5α *Escherichia coli* competent cells (New England BioLabs) and plated on Lysogeny Broth (LB) agar plates containing ampicillin (100 µg/mL). This plasmid was then used as a backbone for the insertion of the genes of interest.

The Pvull-HF digested vector and PCR-amplified inserts were assembled using NEBuilder^{®} HiFi DNA Assembly (New England BioLabs). A 3:1 ratio of insert to vector was used and the reaction was incubated at 50 ºC for 60 minutes. The reaction was then transformed into NEB 5α *E. coli* competent cells (New England BioLabs) and plated on LB agar plates containing ampicillin (100 µg/mL). Clones were grown on LB liquid medium with ampicillin (100 µg/mL) and the plasmid was extracted using the Monarch Plasmid MiniPrep Kit (New England BioLabs).

The single copy plasmids created in this study were:
1. pSH67-*ScILV3*
*2.* pSH67*-ScILV3-ScBAT1*
*3.* pSH67*-ScILV3-ScBAT2*
4. pSH67-*ScILV6*
5. pSH67-*ScBAT2*

### Yeast transformation with vectors and constructed yeasts

Weihenstephan 34/78 and Strain #2 from example 10 (Weihenstephan 34/78 with two deleted S. eubayanus ILV2 gene copies also called WS34/78 [2x ILV2eu]:: Zeo R) yeasts were transformed with the plasmids via electroporation following the protocol outlined by Benatuil et al. 2010. Some minor modifications were made: the yeast was grown at 25 ºC and recovered for 2-3 hours after electroporation, and the cells were plated on YPD agar plates containing geneticin G418 (200 µg/mL).

The yeasts generated in this study are shown in Table 17, below.

**Table 17. Yeasts generated by transformation of single copy plasmids**

| **Yeast strain name** | **Yeast background** | **Plasmid inserted** |
|---|---|---|
| FGMG100 | W34/78 | pSH67-*ScILV3-ScBAT2* |
| FGMO101 | W34/78 | pSH67-*ScILV6* |
| FGMO102 | W34/78 | pSH67-*ScBAT2* |
| FGMO103 | W34/78 | pSH67-*ScILV3* |
| FGMO104 | W34/78 | pSH67-no-insert (Empty) |
| FGMO105 | W34/78 | pSH67*-ScILV3-ScBAT1* |
| FGMG0095 | WS34/78 [2x ILV2eu]:: Zeo R | pSH67*-ScILV6* |
| FGMO0096 | WS34/78 [2x ILV2eu]:: Zeo R | pSH67-*ScILV3* |
| FGMG0097 | WS34/78 [2x ILV2eu]:: Zeo R | pSH67-*ScILV3-ScBAT2* |
| FGMO0098 | WS34/78 [2x ILV2eu]:: Zeo R | pSH67-*ScBAT2* |
| FGMG0099 | WS34/78 [2x ILV2eu]:: Zeo R | pSH67*-ScILV3-ScBAT1* |

### Example 12 - Evaluation of Fermentation Properties of Modified WS34/78 Strains

Strains were fermented in cylindrical vessels as previously described. Cells were grown overnight in 10ml of liquid YPD containing 25 µg/ml of gentamicin (G418) before being transferred to 20ml of the previously described pilsner wort containing 100 µg/ml of G418. Cell pitching was performed as previously described in pilsner wort containing 100 µg/ml of G418. Plato, Diacetyl and volatile measurements were made on Days 3, 4 and 5 of the fermentations. On day 5, carbohydrate profiles and ester profile samples were taken.

The genotypes of yeast strain tested are shown in Table 18, below.

**Table 18. Genetically modified yeast created in examples 10 and 11 tested in this example.**

| **Strain Number** | **Resistance feature** | **Genotype** | **Source** |
|---|---|---|---|
| FGMO 0092 | G418 Resistant | WS34/78 Plasmid 0 (pYESVIII; no insert) control | WS34/78 |
| FGMO 0093 | G418 + Zeocin Resistant | 2x ILV2eu KO:Zeocin insert, 1x ILV5cer pYESVIII copy | Strain #2 from Example 10 |
| FGMO 0094 | G418 + Zeocin + Nourseothricin Resistant | 2x ILV2eu KO:Zeocin insert, 1x ILV5cer pYESVIII copy, 1x ILV3eu insert: KO PDC1 | Strain #2 from Example 10 |
| FGMO 0095 | G418 + Zeocin Resistant | ΔΔ Seu ILV2 Sc ILV6-G418 | Strain #2 from Example 10 |
| FGMO 0096 | G418 + Zeocin Resistant | ΔΔ Seu ILV2 Sc ILV3-G418 | Strain #2 from Example 10 |
| FGMO 0100 | G418 Resistant | WS34/78 Sc ILV3 - Sc BAT 2 - G418 | WS34/78 |
| FGMO 0101 | G418 Resistant | WS34/78 Sc ILV 6 - G418 | WS34/78 |
| FGMO 0102 | G418 Resistant | WS34/78 Sc BAT 2 - G418 | WS34/78 |
| FGMO 0103 | G418 Resistant | WS34/78 Sc ILV 3 - G418 | WS34/78 |
| FGMO 0104 | G418 Resistant | WS34/78 empty vector G418 | WS34/78 |
| FGMO 0105 | G418 Resistant | WS34/78 Sc ILV 3 - fBAT 1 - G418 | WS34/78 |

The results of the fermentation with each yeast strain are summarized in Tables 19 and 20, below.

**Table 19. Results of fermentation with genetically modified yeast created in examples 10 and 11 (total diacetyl).**

| **Strain Number** | **Total diacetyl** | | | **Plato** | | |
|---|---|---|---|---|---|---|
| | **Day 3** | **Day 4** | **Day 5** | **Day 3** | **Day 4** | **Day 5** |
| FGMG0092 | 319 | 216 | 170 | 10 | 7,6 | 5,5 |
| FGMG0093 | 209 | 140 | 94 | 8,9 | 6,4 | 4,8 |
| FGMG0094 | 184 | 123 | 87 | 9,2 | 6,4 | 4,9 |
| FGMG0095 | 315 | 191 | 153 | 8,9 | 7,1 | 6,2 |
| FGMO0096 | 201 | 121 | 97 | 8,6 | 5,5 | 4,4 |
| FGMG0100 | 329 | 187 | 142 | 7,9 | 5,1 | 3,4 |
| FGMG0101 | 293 | 167 | 153 | 7,6 | 4,4 | 3,3 |
| FGMG0102 | 370 | 244 | 269 | 8 | 5,1 | 4,4 |
| FGMG0103 | 396 | 234 | 197 | 7,8 | 4,4 | 4 |
| FGMG0104 | 265 | 180 | 167 | 7,4 | 4 | 3,4 |
| FGMO0105 | 279 | 203 | 154 | 8,1 | 4,7 | 3,5 |

**Table 20. Results of fermentation with genetically modified yeast created in examples 10 and 11 (Propanol/lsobutanol ratio). n.m. = not measured.**

| **Strain Number** | **Propanol/Isobutanol ratio** | | **Plato** | | |
|---|---|---|---|---|---|
| | **Day 4** | **Day 5** | **Day 3** | **Day 4** | **Day 5** |
| FGMO0092 | 1,9 | 1,6 | 10 | 7,6 | 5,5 |
| FGMO0093 | 3,04 | 3,3 | 8,9 | 6,4 | 4,8 |
| FGMO0094 | 2,92 | n.m. | 9,2 | 6,4 | 4,9 |
| FGMO0095 | 2,97 | 2,77 | 8,9 | 7,1 | 6,2 |
| FGMO0096 | 2,71 | 2,93 | 8,6 | 5,5 | 4,4 |
| FGMO0100 | 1,55 | 1,44 | 7,9 | 5,1 | 3,4 |
| FGMO0101 | 1,85 | 1,87 | 7,6 | 4,4 | 3,3 |
| FGMO0102 | 1,32 | 1,37 | 8 | 5,1 | 4,4 |
| FGMO0103 | 1,76 | 1,68 | 7,8 | 4,4 | 4 |
| FGMO0104 | 1,69 | 1,64 | 7,4 | 4 | 3,4 |
| FGMO0105 | 1,64 | 1,64 | 8,1 | 4,7 | 3,5 |

It should be noted, that the genetically engineered strains of this example were fermented in the presence of antibiotics, and the results above might therefore be slightly different when the strains are fermented industrially without the presence of antibiotics. In particular, the strains will likely have faster growth rate, and fermentation will thus proceed faster. Similarly, reduction of initially produced diacetyl is also expected to proceed faster.

The strain with the lowest amount of total diacetyl in the test solution on day 5 was the strain with two copies of the ILV2 gene deleted, an extra copy of ILV3 genomically integrated and an extra copy of ILV5 expressed from a single-copy plasmid (FGMO0094).

### Sequence listing

| | |
|---|---|
| SEQ ID NO:1 | *ILV2* target specific forward primer *S. cerevisiae* and *S. eubayanus* |
| SEQ ID NO:2 | *ILV2* target specific reverse primer *S. cerevisiae* and *S. eubayanus* |
| SEQ ID NO:3 | *S. cerevisiae ILV2* allele specific probe |
| SEQ ID NO:4 | *S. eubayanus ILV2* allele specific probe |
| SEQ ID NO:5 | ZDA1 ILV2 |
| SEQ ID NO:6 | ZDA1 ILV6 - tig00000158 |
| SEQ ID NO:7 | ZDA1 ILV6 - tig00002562 |
| SEQ ID NO:8 | ZDA1 ILV5 -tig00000010 |
| SEQ ID NO:9 | ZDA1 ILV5 -tig00052675 |
| SEQ ID NO:10 | ZDA1 ILV3 -tig00000103 |
| SEQ ID NO:11 | ZDA1 ILV3 -tig00002552 |
| SEQ ID NO:12 | ZDA1 ILV3 -tig00000090 |
| SEQ ID NO:13 | ZDA1 ILV3 -tig00000096 |
| SEQ ID NO:14 | ZDA1 BAT1 -tig00000105 |
| SEQ ID NO:15 | ZDA1 BAT1 -tig00000101 |
| SEQ ID NO:16 | ZDA1 BAT1 -tig00052676 |
| SEQ ID NO:17 | ZDA1 BAT2 -tig00000102 |
| SEQ ID NO:18 | ZDA1 BAT2 -tig00000090 |
| SEQ ID NO:19 | ZDA1 BAT2 -tig00002552 |
| SEQ ID NO:20 | ZDA2 ILV2 |
| SEQ ID NO:21 | ZDA2 ILV6 -tig00015333 |
| SEQ ID NO:22 | ZDA2 ILV6 -tig00000147 |
| SEQ ID NO:23 | ZDA2 ILV5 -tig00000655 |
| SEQ ID NO:24 | ZDA2 ILV5 -tig00000022 |
| SEQ ID NO:25 | ZDA2 ILV3 -tig00000088 |
| SEQ ID NO:26 | ZDA2 ILV3 -tig00000672 |
| SEQ ID NO:27 | ZDA2 ILV3 -tig00000669 |
| SEQ ID NO:28 | ZDA2 BAT1 -tig00000115 |
| SEQ ID NO:29 | ZDA2 BAT1 -tig00015328 |
| SEQ ID NO:30 | ZDA2 BAT2 -tig00000088 |
| SEQ ID NO:31 | ZDA2 BAT2 -tig00000669 |
| SEQ ID NO:32 | Reference ILV2 *S. cerevisiae* |
| SEQ ID NO:33 | Reference ILV6 *S. cerevisiae* |
| SEQ ID NO:34 | Reference ILV5 *S. cerevisiae* |
| SEQ ID NO:35 | Reference ILV3 *S. cerevisiae* |
| SEQ ID NO:36 | Reference BAT1 *S. cerevisiae* |
| SEQ ID NO:37 | Reference BAT2 *S. cerevisiae* |
| SEQ ID NO:38 | Reference ILV2 *S. eubayanus* |
| SEQ ID NO:39 | Reference ILV6 *S. eubayanus* |
| SEQ ID NO:40 | Reference ILV5 *S. eubayanus* |
| SEQ ID NO:41 | Reference ILV3 *S. eubayanus* |
| SEQ ID NO:42 | Reference BAT1 *S. eubayanus* |
| SEQ ID NO:43 | Reference BAT2 *S. eubayanus* |
| SEQ ID NO:44 | CPA43 |
| SEQ ID NO:45 | CPA44 |
| SEQ ID NO:46 | CPA49 |
| SEQ ID NO:47 | CPA50 |
| SEQ ID NO:48 | CPA51 |
| SEQ ID NO:49 | CPA52 |
| SEQ ID NO:50 | ILV2 (se) knockout cassette |
| SEQ ID NO:51 | CPA70 |
| SEQ ID NO:52 | CPA71 |
| SEQ ID NO:53 | CPA91 |
| SEQ ID NO:54 | CPA96 |
| SEQ ID NO:55 | CPA111 |
| SEQ ID NO:56 | CPA112 |
| SEQ ID NO:57 | CPA113 |
| SEQ ID NO:58 | CPA114 |
| SEQ ID NO:59 | CPA128 |
| SEQ ID NO:60 | CPA129 |
| SEQ ID NO:61 | ILV3 (se) NTC integration cassette |
| SEQ ID NO:62 | CPA74 |
| SEQ ID NO:63 | CPA75 |
| SEQ ID NO:64 | CPA86 |
| SEQ ID NO:65 | CPA87 |
| SEQ ID NO:66 | ILV5 (sc) expression cassette |
| SEQ ID NO:67 | ScILV3_F |
| SEQ ID NO:68 | SclLV3_R |
| SEQ ID NO:69 | ScBAT1_F |
| SEQ ID NO:70 | ScBAT1_R |
| SEQ ID NO:71 | ScBAT2_F |
| SEQ ID NO:72 | ScBAT2_R |
| SEQ ID NO:73 | ScILV6_F |
| SEQ ID NO:74 | ScILV6_R |

### References

Lorenzo Benatuil, Jennifer M. Perez, Jonathan Belk, Chung-Ming Hsieh, An improved yeast transformation method for the generation of very large human antibody libraries, Protein Engineering, Design and Selection, Volume 23, Issue 4, April 2010, Pages 155-159, https://doi.org/10.1093/protein/gzq002
Briggs, D. E. et al. Malting and Brewing science. 1981.
Denis E, Sanchez S, Mairey B et al. Extracting high molecular weight genomic DNA from Saccharomyces cerevisiae, Protocol Exchange DOI 10.1038/protex.2018.076
Hough, J. S. et al. Malting and Brewing science: Hopped Wort and Beer, Volume 2. 1982.
Gutierrez A, Boekhout T, Gojkovic Z, Katz M. Evaluation of non-Saccharomyces yeasts in the fermentation of wine, beer and cider for the development of new beverages J Inst Brew 2018 DOI 10.1002/jib.512
Mumberg D, Müller R, Funk M
Yeast vectors for the controlled expression of heterologous proteins in different genetic backgrounds Gene 1995:156:119-22.
doi: 10.1016/0378-1119(95)00037-7
Walther A, Hesselbart A, Wendland J. Genome Sequence of Saccharomyces Carlsbergensis, the World's First Pure Culture Lager Yeast G3 (Bethesda) 2014:4:783-93.

## Claims

1. A yeast strain encoding within its genome
a. at the most two functional genes encoding ILV2, wherein each gene encoding ILV2 encodes ScILV2 of SEQ ID NO: 32 or SelLV2 of SEQ ID NO: 38, or a functional homologue sharing at least 80% sequence identity herewith; and
b. at least five functional genes, such as at least six functional genes encoding ILV3, wherein each gene encoding ILV3 encodes ScILV3 of SEQ ID NO: 35 or SelLV3 of SEQ ID NO: 41, or a functional homologue sharing at least 80% sequence identity herewith.

2. The yeast strain according to claim 1, wherein said yeast strain encodes within its genome at least four functional genes encoding ILV5, such as at least five functional genes encoding ILV5, wherein each gene encoding ILV5 encodes ScILV5 of SEQ ID NO: 34 or SelLV5 of SEQ ID NO: 40, or a functional homologue sharing at least 80% sequence identity herewith.

3. The yeast strain according to any one of the preceding claims, wherein the genes encoding ILV2, ILV3 and/or ILV5 are expressed from their native promoters, optionally wherein the genes encoding ILV6, BAT1 and/or BAT2 are expressed from their native promoters.

4. The yeast strain according to any one of the preceding claims, wherein said yeast strain does not comprise any heterologous DNA and/or said yeast strain has not undergone a step of genetic engineering.

5. The yeast strain according to any one of the preceding claims, wherein said yeast strain has at the most 15 functional genes encoding ILV3, such as between 5 and 10 functional genes encoding ILV3, such as between 5 and 6 functional genes encoding ILV3, and/or
wherein said yeast strain has at the most functional 15 genes encoding ILV5, such as between 4 and 10 functional genes encoding ILV5, such as between 4 and 5 functional genes encoding ILV5 and/or wherein said yeast strain has at the most four functional genes encoding ILV6, wherein each gene encoding ILV6 encodes SclLV6 of SEQ ID NO: 33 or SelLV6 of SEQ ID NO: 39, or a homologue sharing at least 80% sequence identity herewith, and/or
wherein said yeast strain has at least three functional genes, such as at least four functional genes encoding BAT1, wherein each gene encoding BAT1 encodes ScBAT1 of SEQ ID NO: 36 or SeBAT1 of SEQ ID NO: 42, or a homologue sharing at least 80% sequence identity herewith, and/or
wherein said yeast strain has at least four functional genes, such as at least five functional genes encoding BAT2, wherein each gene encoding BAT2 encodes ScBAT2 of SEQ ID NO: 37 or SeBAT2 of SEQ ID NO: 43, or a homologue sharing at least 80% sequence identity herewith.

6. The yeast strain according to any one of the preceding claims, wherein the sum of the functional genes in said yeast strain encoding ILV2 and ILV6 is lower than the sum of the functional genes encoding ILV5 and ILV3.

7. The yeast strain according to any one of the preceding claims, wherein the functional gene ratio in said yeast strain of *ILV5* and *ILV3* vs. *ILV2* is at least 1, such as at least 1.5, such as at least 2, such as at least 2.5 or such as at least 3 and/or the functional gene ratio in said yeast strain of *ILV5* and *ILV3* vs. *ILV2* and *ILV6* is at least 1, such as at least 1.2, such as at least 1.4, such as at least 1.6, such as at least 1.8 or such as at least 2.

8. The yeast strain according to any one of the preceding claims, wherein said yeast strain carries a mutation in or a deletion of one or more *ILV2* genes and/or said yeast strain carries a frameshift mutation in one or more *ILV2* genes and/or
said yeast strain carries a mutation resulting in lower or no expression of one or more *ILV2* genes.

9. The yeast strain according to any one of the preceding claims, wherein said yeast strain is capable of producing a fermented test solution upon incubation in a test solution, wherein the test solution is an extract of malt and/or cereal having an apparent extract of at least 10° Plato, wherein said fermented test solution contains at the most 60 ppb diacetyl at the earliest time point when the apparent extract of said test solution has not decreased by more than 0.50° Plato in the preceding 24 hours, wherein said fermentation occurs at a temperature of at the most 18 °C.

10. The yeast strain according to claim 9, wherein said yeast strain is capable of generating at least 4.0 mL/L ethanol per °Plato, when said yeast strain is incubated in said test solution.

11. The yeast strain according to any one of the preceding claims, wherein said yeast strain is capable of growing on media with melibiose as the sole carbon source.

12. The yeast strain according to any one of claims 9 to 11, wherein said fermented test solution contains at the most 60 ppb diacetyl, such as at the most 55 ppb diacetyl, such as at the most 50 ppb diacetyl, such as at the most 45 ppb diacetyl, such as at the most 40 ppb diacetyl.

13. The yeast strain according to any one of claims 9 to 12, wherein said fermented aqueous extract contains a least 25 mg/L propanol, such as at the least 30 mg/L propanol and/or wherein said fermented aqueous extract contains at the most 8 mg/L isobutanol, such as at the most 6 mg/L isobutanol and/or wherein said fermented aqueous extract contains a propanol:isobutanol ratio of at least 2.0, such as at least 2.5. such as at least 3.0, such as at least 4.0, such as at least 5.0, such as at least 5.5.

14. A method of producing a fermented aqueous extract, said method comprising the steps of:
i) providing an aqueous extract of malt and/or cereal;
ii) providing a yeast strain of the species *Saccharomyces pastorianus,* wherein said yeast strain is according to any one of claims 1 to 13; and
iii) fermenting the aqueous extract provided in step i) with said yeast strain of step ii), thereby obtaining a fermented aqueous extract.

15. A method for producing a beverage, said method comprising the steps of:
i. preparing a fermented aqueous extract according to claim 14, and
ii. processing said fermented aqueous extract into a beverage,
optionally wherein the steps of processing comprise one or more of the following:
i. Filtration,
ii. Carbonation,
iii. Maturation, or
iv. Bottling.

16. A beverage prepared by the method according to claim 15, wherein said beverage has a propanol:isobutanol ratio of at least 3.0, such as at least 4.0, such as at least 5.0, such as at least 5.5.

17. The beverage according to claim 16, wherein said beverage is a beer.

18. The beverage according to any one of claims 16 to 17, wherein said beverage contains
at least 25 mg/L propanol, such as at least 30 mg/L propanol and/or
at the most 8 mg/L isobutanol, such as at the most 6 mg/L isobutanol.

## Patentansprüche

1. Hefestamm, der in seinem Genom Folgendes kodiert
a. höchstens zwei funktionelle Gene, die für ILV2 kodieren, wobei jedes Gen, das für ILV2 kodiert, für ScILV2 von SEQ-ID-NR: 32 oder SeILV2 von SEQ-ID-NR: 38 oder ein funktionelles Homolog, das mindestens 80 % Sequenzidentität damit teilt, kodiert; und
b. mindestens fünf funktionelle Gene, wie etwa mindestens sechs funktionelle Gene, die für ILV3 kodieren, wobei jedes Gen, das für ILV3 kodiert, für ScILV3 von SEQ-ID-NR: 35 oder SeILV3 von SEQ-ID-NR: 41 oder ein funktionelles Homolog, das mindestens 80 % der Sequenzidentität damit teilt, kodiert.

2. Hefestamm nach Anspruch 1, wobei der Hefestamm in seinem Genom mindestens vier funktionelle Gene kodiert, die für ILV5 kodieren, wie etwa mindestens fünf funktionelle Gene, die für ILV5 kodieren, wobei jedes Gen, das für ILV5 kodiert, für ScILV5 der SEQ-ID-NR: 34 kodiert oder SeILV5 von SEQ-ID-NR: 40 oder ein funktionelles Homolog, das mindestens 80 % Sequenzidentität damit teilt, kodiert.

3. Hefestamm nach einem der vorhergehenden Ansprüche, wobei die für ILV2, ILV3 und/oder ILV5 kodierenden Gene von ihren nativen Promotoren exprimiert werden, optional wobei die für ILV6, BAT1 und/oder BAT2 kodierenden Gene von ihren nativen Promotoren exprimiert werden.

4. Hefestamm nach einem der vorhergehenden Ansprüche, wobei der Hefestamm keine heterologe DNA umfasst und/oder der Hefestamm keinem gentechnischen Schritt unterzogen wurde.

5. Hefestamm nach einem der vorhergehenden Ansprüche, wobei der Hefestamm höchstens 15 funktionelle Gene aufweist, die für ILV3 kodieren, wie etwa zwischen 5 und 10 funktionelle Gene, die für ILV3 kodieren, wie etwa zwischen 5 und 6 funktionelle Gene, die für ILV3 kodieren, und/oder
wobei der Hefestamm höchstens 15 funktionelle Gene aufweist, die für ILV5 kodieren, wie etwa zwischen 4 und 10 funktionelle Gene, die für ILV5 kodieren, wie etwa zwischen 4 und 5 funktionelle Gene, die für ILV5 kodieren, und/oder wobei der Hefestamm höchstens vier funktionelle Gene aufweist, die für ILV6 kodieren, wobei jedes für ILV6 kodierende Gen für ScILV6 von SEQ-ID-NR: 33 oder SeILV6 von SEQ-ID-NR: 39 oder ein Homolog, das mindestens 80 % Sequenzidentität damit teilt, kodiert, und/oder
wobei der Hefestamm mindestens drei funktionelle Gene aufweist, wie etwa mindestens vier funktionelle Gene, die für BAT1 kodieren, wobei jedes Gen, das für BAT1 kodiert, für ScBAT1 von SEQ-ID-NR: 36 oder SeBAT1 von SEQ-ID-NR: 42 oder ein Homolog, das mindestens 80 % Sequenzidentität damit teilt, kodiert, und/oder
wobei der Hefestamm mindestens vier funktionelle Gene aufweist, wie etwa mindestens fünf funktionelle Gene, die für BAT2 kodieren, wobei jedes Gen, das für BAT2 kodiert, für ScBAT2 von SEQ-ID-NR: 37 oder SeBAT2 von SEQ-ID-NR: 43 oder ein funktionelles Homolog, das mindestens 80 % der Sequenzidentität damit teilt, kodiert.

6. Hefestamm nach einem der vorhergehenden Ansprüche, wobei die Summe der funktionellen Gene in dem Hefestamm, die für ILV2 und ILV6 kodieren, niedriger ist als die Summe der funktionellen Gene, die für ILV5 und ILV3 kodieren.

7. Hefestamm nach einem der vorhergehenden Ansprüche, wobei das funktionelle Genverhältnis in dem Hefestamm von *ILV5* und *ILV3* gegenüber *ILV2* mindestens 1 beträgt, wie etwa mindestens 1,5, wie etwa mindestens 2, wie etwa mindestens 2,5 oder wie etwa mindestens 3 und/oder das funktionelle Genverhältnis in dem Hefestamm von *ILV5* und *ILV3* gegenüber *ILV2* und *ILV6* mindestens 1 beträgt, wie etwa mindestens 1,2, wie etwa mindestens 1,4, wie etwa mindestens 1,6, wie etwa mindestens 1,8 oder wie etwa mindestens 2.

8. Hefestamm nach einem der vorhergehenden Ansprüche, wobei der Hefestamm eine Mutation oder eine Deletion von einem oder mehreren *ILV2*-Genen trägt und/oder der Hefestamm eine Frameshift-Mutation in einem oder mehreren *ILV2*-Genen trägt und/oder
der Hefestamm eine Mutation trägt, die zu einer geringeren oder keiner Expression eines oder mehrerer *ILV2*-Gene führt.

9. Hefestamm nach einem der vorhergehenden Ansprüche, wobei der Hefestamm in der Lage ist, bei Inkubation in einer Testlösung eine fermentierte Testlösung zu produzieren, wobei die Testlösung ein Extrakt aus Malz und/oder Getreide mit einem scheinbaren Extrakt von mindestens 10° Plato ist, wobei die fermentierte Testlösung zum frühesten Zeitpunkt höchstens 60 ppb Diacetyl enthält, wenn der scheinbare Extrakt der Testlösung in den vorangegangenen 24 Stunden nicht um mehr als 0,50° Plato abgenommen hat, wobei die Fermentation bei einer Temperatur von höchstens 18 °C auftritt.

10. Hefestamm nach Anspruch 9, wobei der Hefestamm in der Lage ist, mindestens 4,0 ml/l Ethanol pro °Plato zu erzeugen, wenn der Hefestamm in der Testlösung inkubiert wird.

11. Hefestamm nach einem der vorhergehenden Ansprüche, wobei der Hefestamm in der Lage ist, auf Medien mit Melibiose als einziger Kohlenstoffquelle zu wachsen.

12. Hefestamm nach einem der Ansprüche 9 bis 11, wobei die fermentierte Testlösung höchstens 60 ppb Diacetyl, wie etwa höchstens 55 ppb Diacetyl, wie etwa höchstens 50 ppb Diacetyl, wie etwa höchstens 45 ppb Diacetyl, wie etwa höchstens 40 ppb Diacetyl enthält.

13. Hefestamm nach einem der Ansprüche 9 bis 12, wobei der fermentierte wässrige Extrakt mindestens 25 mg/l Propanol, wie etwa mindestens 30 mg/l Propanol, enthält und/oder wobei der fermentierte wässrige Extrakt höchstens 8 mg/l Isobutanol, wie etwa höchstens 6 mg/l Isobutanol, enthält und/oder wobei der fermentierte wässrige Extrakt ein Propanol:Isobutanol-Verhältnis von mindestens 2,0, wie etwa mindestens 2,5, wie etwa mindestens 3,0, wie etwa mindestens 4,0, wie etwa mindestens 5,0, wie etwa mindestens 5,5 enthält.

14. Verfahren zum Produzieren eines fermentierten wässrigen Extrakts, wobei das Verfahren die folgenden Schritte umfasst:
i) Bereitstellen eines wässrigen Extrakts aus Malz und/oder Getreide;
ii) Bereitstellen eines Hefestamms der Art *Saccharomyces pastorianus,* wobei der Hefestamm einem der Ansprüche 1 bis 13 entspricht; und
iii) Fermentieren des in Schritt i) bereitgestellten wässrigen Extrakts mit dem Hefestamm aus Schritt ii), wodurch ein fermentierter wässriger Extrakt erhalten wird.

15. Verfahren zum Produzieren eines Getränks, wobei das Verfahren die folgenden Schritte umfasst:
i. Herstellen eines fermentierten wässrigen Extrakts nach Anspruch 14 und
ii. Verarbeiten des fermentierten wässrigen Extrakts zu einem Getränk, wobei die Verarbeitungsschritte optional einen oder mehrere der folgenden Schritte umfassen:
i. Filtration,
ii. Karbonisierung,
iii. Reifung oder
iv. Abfüllung.

16. Getränk, hergestellt nach dem Verfahren nach Anspruch 15, wobei das Getränk ein Propanol:Isobutanol-Verhältnis von mindestens 3,0, wie etwa mindestens 4,0, wie etwa mindestens 5,0, wie etwa mindestens 5,5 aufweist.

17. Getränk nach Anspruch 16, wobei das Getränk ein Bier ist.

18. Getränk nach einem der Ansprüche 16 bis 17, wobei das Getränk mindestens 25 mg/l Propanol, wie etwa mindestens 30 mg/l Propanol und/oder
höchstens 8 mg/l Isobutanol, wie etwa höchstens 6 mg/l Isobutanol enthält.

## Revendications

1. Souche de levure codant au sein de son génome
a. au plus deux gènes fonctionnels codant pour l'ILV2, dans laquelle chaque gène codant pour l'ILV2 code pour le ScILV2 de SEQ ID NO : 32 ou le SeILV2 de SEQ ID NO : 38, ou un homologue fonctionnel partageant au moins 80 % d'identité de séquence avec celui-ci ; et
b. au moins cinq gènes fonctionnels, tel qu'au moins six gènes fonctionnels codant pour l'ILV3, dans laquelle chaque gène codant pour l'ILV3 code pour le ScILV3 de SEQ ID NO : 35 ou le SeILV3 de SEQ ID NO : 41, ou un homologue fonctionnel partageant au moins 80 % d'identité de séquence avec celui-ci.

2. Souche de levure selon la revendication 1, dans laquelle ladite souche de levure code au sein de son génome au moins quatre gènes fonctionnels codant pour l'ILV5, tel qu'au moins cinq gènes fonctionnels codant pour l'ILV5, dans laquelle chaque gène codant pour l'ILV5 code pour le ScILV5 de SEQ ID NO : 34 ou le SeILV5 de SEQ ID NO : 40, ou un homologue fonctionnel partageant au moins 80 % d'identité de séquence avec celui-ci.

3. Souche de levure selon l'une quelconque des revendications précédentes, dans laquelle les gènes codant pour l'ILV2, l'ILV3 et/ou l'ILV5 sont exprimés à partir de leurs promoteurs natifs, éventuellement dans laquelle les gènes codant pour l'ILV6, le BAT1 et/ou le BAT2 sont exprimés à partir de leurs promoteurs natifs.

4. Souche de levure selon l'une quelconque des revendications précédentes, dans laquelle ladite souche de levure ne comprend aucun ADN hétérologue et/ou ladite souche de levure n'a pas subi d'étape de génie génétique.

5. Souche de levure selon l'une quelconque des revendications précédentes, dans laquelle ladite souche de levure possède au plus 15 gènes fonctionnels codant pour l'ILV3, tel qu'entre 5 et 10 gènes fonctionnels codant pour l'ILV3, tel qu'entre 5 et 6 gènes fonctionnels codant pour l'ILV3, et/ou
dans laquelle ladite souche de levure possède au plus 15 gènes fonctionnels codant pour l'ILV5, tel qu'entre 4 et 10 gènes fonctionnels codant pour l'ILV5, tel qu'entre 4 et 5 gènes fonctionnels codant pour l'ILV5 et/ou dans laquelle ladite souche de levure possède au plus quatre gènes fonctionnels codant pour l'ILV6, dans laquelle chaque gène codant pour l'ILV6 code pour le ScILV6 de SEQ ID NO : 33 ou le SeILV6 de SEQ ID NO : 39, ou un homologue partageant au moins 80 % d'identité de séquence avec celui-ci, et/ou
dans laquelle ladite souche de levure possède au moins trois gènes fonctionnels, tel qu'au moins quatre gènes fonctionnels codant pour le BAT1, dans laquelle chaque gène codant pour le BAT1 code pour le ScBAT1 de SEQ ID NO : 36 ou le SeBAT1 de SEQ ID NO : 42, ou un homologue partageant au moins 80 % d'identité de séquence avec celui-ci, et/ou
dans laquelle ladite souche de levure possède au moins quatre gènes fonctionnels, tel qu'au moins cinq gènes fonctionnels codant pour le BAT2, dans laquelle chaque gène codant pour le BAT2 code pour le ScBAT2 de SEQ ID NO : 37 ou le SeBAT2 de SEQ ID NO : 43, ou un homologue partageant au moins 80 % d'identité de séquence avec celui-ci.

6. Souche de levure selon l'une quelconque des revendications précédentes, dans laquelle la somme des gènes fonctionnels dans ladite souche de levure codant pour l'ILV2 et l'ILV6 est inférieure à la somme des gènes fonctionnels codant pour l'ILV5 et l'ILV3.

7. Souche de levure selon l'une quelconque des revendications précédentes, dans laquelle le rapport des gènes fonctionnels dans ladite souche de levure de l'*ILV*5 et de l*'ILV3* à l*'ILV2* est d'au moins 1, tel qu'au moins 1,5, tel qu'au moins 2, tel qu'au moins 2,5 ou tel qu'au moins 3 et/ou le rapport des gènes fonctionnels dans ladite souche de levure de l*'ILV5* et de l*'ILV3* à l*'ILV2* et l*'ILV6* est d'au moins 1, tel qu'au moins 1,2, tel qu'au moins 1,4, tel qu'au moins 1,6, tel qu'au moins 1,8 ou tel qu'au moins 2.

8. Souche de levure selon l'une quelconque des revendications précédentes, dans laquelle ladite souche de levure porte une mutation ou une délétion d'un ou plusieurs gènes d*'ILV2* et/ou ladite souche de levure porte une mutation par déphasage dans un ou plusieurs gènes d*'ILV2* et/ou ladite souche de levure porte une mutation entraînant une expression moindre ou nulle d'un ou plusieurs gènes d*'ILV2.*

9. Souche de levure selon l'une quelconque des revendications précédentes, dans laquelle ladite souche de levure est capable de produire une solution test fermentée lors de l'incubation dans une solution test, dans laquelle la solution test est un extrait de malt et/ou de céréales ayant un extrait apparent d'au moins 10° Plato, dans laquelle ladite solution test fermentée contient au plus 60 ppb de diacétyle au moment le plus précoce lorsque l'extrait apparent de ladite solution test n'a pas diminué de plus de 0,50° Plato au cours des 24 heures précédentes, dans laquelle ladite fermentation se produit à une température maximum de 18 °C.

10. Souche de levure selon la revendication 9, dans laquelle ladite souche de levure est capable de générer au moins 4,0 ml/l d'éthanol par °Plato, lorsque ladite souche de levure est incubée dans ladite solution test.

11. Souche de levure selon l'une quelconque des revendications précédentes, dans laquelle ladite souche de levure est capable de croître sur des milieux avec du mélibiose comme seule source de carbone.

12. Souche de levure selon l'une quelconque des revendications 9 à 11, dans laquelle ladite solution test fermentée contient au plus 60 ppb de diacétyle, tel qu'au plus 55 ppb de diacétyle, tel qu'au plus 50 ppb de diacétyle, tel qu'au plus 45 ppb de diacétyle, tel qu'au plus 40 ppb de diacétyle.

13. Souche de levure selon l'une quelconque des revendications 9 à 12, dans laquelle ledit extrait aqueux fermenté contient au moins 25 mg/l de propanol, tel qu'au moins 30 mg/l de propanol et/ou dans laquelle ledit extrait aqueux fermenté contient au plus 8 mg/l d'isobutanol, tel qu'au plus 6 mg/l d'isobutanol et/ou dans laquelle ledit extrait aqueux fermenté contient un rapport propanol:isobutanol d'au moins 2,0, tel qu'au moins 2,5, tel qu'au moins 3,0, tel qu'au moins 4,0, tel qu'au moins 5,0, tel qu'au moins 5,5.

14. Procédé de fabrication d'un extrait aqueux fermenté, ledit procédé comprenant les étapes de :
i) fourniture d'un extrait aqueux de malt et/ou de céréales ;
ii) fourniture d'une souche de levure de l'espèce *Saccharomyces pastorianus,* dans lequel ladite souche de levure est conforme à l'une quelconque des revendications 1 à 13 ; et
iii) fermentation de l'extrait aqueux fourni à l'étape i) avec ladite souche de levure de l'étape ii), obtenant ainsi un extrait aqueux fermenté.

15. Procédé de fabrication d'une boisson, ledit procédé comprenant les étapes de :
i. préparation d'un extrait aqueux fermenté selon la revendication 14, et
ii. traitement dudit extrait aqueux fermenté en une boisson, éventuellement dans lequel les étapes de traitement comprennent une ou plusieurs des étapes suivantes :
i. Filtration,
ii. Carbonatation,
iii. Maturation, ou
iv. Embouteillage.

16. Boisson préparée par le procédé selon la revendication 15, dans laquelle ladite boisson a un rapport propanol : isobutanol d'au moins 3,0, tel qu'au moins 4,0, tel qu'au moins 5,0, tel qu'au moins 5,5.

17. Boisson selon la revendication 16, dans laquelle ladite boisson est une bière.

18. Boisson selon l'une quelconque des revendications 16 et 17, dans laquelle ladite boisson contient
au moins 25 mg/l de propanol, tel qu'au moins 30 mg/l de propanol et/ou
au plus 8 mg/l d'isobutanol, tel qu'au plus 6 mg/l d'isobutanol.
